(19) Europäisches Patentamt
European Patent Office
Office européen des brevets

(11) EP 1 092 716 B1

(12) **FASCICULE DE BREVET EUROPEEN**

(45) Date de publication et mention de la délivrance du brevet:
**16.02.2005 Bulletin 2005/07**

(21) Numéro de dépôt: **00402831.2**

(22) Date de dépôt: **13.10.2000**

(51) Int Cl.[7]: **C07D 409/06**, A61K 31/47, A61K 31/44, C07D 333/64, C07D 409/14, C07D 401/06, A61P 7/04, A61P 7/02

(54) **Dérivés benzothiophéniques, benzofuraniques et indoliques, leur procédé de préparation et les compositions pharmaceutiques qui les contiennent**

Benzothiophenderivate, Benzofuranderivate und Indolderivate, Verfahren zu ihrer Herstellung und sie enthaltende pharmazeutische Zubereitungen

Derivatives of benzothiophenes, benzofurans and indoles, method for their preparation and pharmaceutical compositions containing them

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **15.10.1999 FR 9912899**

(43) Date de publication de la demande:
**18.04.2001 Bulletin 2001/16**

(73) Titulaire: **LES LABORATOIRES SERVIER**
**92200 Neuilly sur Seine (FR)**

(72) Inventeurs:
- **de Nanteuil, Guillaume**
  **92150 Suresnes (FR)**
- **Lila, Christine**
  **91190 Gif Sur Yvette (FR)**
- **Verbeuren, Tony**
  **78540 Vernouillet (FR)**
- **Rupin, Alain**
  **37510 Savonnieres (FR)**

(56) Documents cités:
**WO-A-92/03132**
**WO-A-93/25546**
**WO-A-94/08962**
**WO-A-94/12179**
**WO-A-95/32190**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux dérivés benzothiophèniques, benzofuraniques et indoliques, leur procédé de préparation et les compositions pharmaceutiques qui les contiennent. Ces nouveaux composés sont utiles pour leur activité thérapeutique dans le domaine de la fibrinolyse et de la thrombose, grâce à leur propriété inhibitrice de l'activité du PAI-1.

Le PAI-1 est un inhibiteur puissant des activateurs du plasminogène (activateur tissulaire du plasminogène et urokinase). Il provoque, in-vitro et in-vivo, l'inhibition de la lyse des caillots fibrineux formés par l'action de la thrombine sur le fibrinogène. De nombreuses études épidémiologiques ont montré que chez l'homme, des taux élevés de PAI-1 sont associés à la survenue plus fréquente de maladies thromboemboliques. De plus, dans des modèles expérimentaux de thrombose et de thrombolyse, l'inhibition de l'activité du PAI-1 par des anticorps monoclonaux anti-PAI-1 diminue l'incidence des thromboses ou des réocclusions. L'intérêt thérapeutique de molécules possédant la propriété d'inhiber l'activité du PAI-1 au sein du caillot fibrineux formé ou en formation est donc de permettre sa lyse précoce avant sa complexation avec le facteur XIIIa et ainsi de diminuer l'incidence des accidents thromboemboliques chez les patients possédant des taux élevés de PAI-1. De tels composés présentent un intérêt thérapeutique dans toutes les pathologies dont l'origine est la thrombose (tel que l'infarctus du myocarde, l'angor, la claudication intermittente, les accidents vasculaires cérébraux, la thrombose veineuse profonde, ou l'embolie pulmonaire) ainsi que pour les pathologies dans lesquelles les risques thrombotiques sont augmentés (telles que l'hypertension, l'hypercholestérolémie, le diabète, l'obésité, les anomalies génétiques de la coagulation (facteur V leiden, déficit en protéines C et S) ou les anomalies acquises de la coagulation).

Les composés de la présente invention, outre le fait qu'ils soient nouveaux, se sont révélés être des inhibiteurs du PAI-1 plus puissants que ceux décrits dans la littérature, ce qui les rend donc potentiellement utiles pour le traitement de la thrombose, des pathologies dont l'origine est la thrombose, et des pathologies entraînant une augmentation des risques thrombotiques.

Un certain nombre d'antithrombotiques ont été décrits dans la littérature. C'est le cas, plus particulièrement, des composés décrits dans les brevets WO 97/45424, WO 94/08962, EP 540051 ou GB 2225012.

La demande de brevet WO 95/32190 décrit des pipérazinediones en tant qu'inhibiteurs de PAI.

Plus spécifiquement, la présente invention concerne les composés de formule (I):

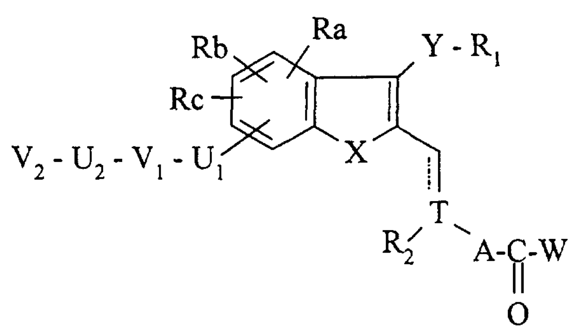

(I)

dans laquelle :

X représente un atome d'oxygène, de soufre, ou un groupement $NR_3$ dans lequel $R_3$ représente un atome d'hydrogène, un groupement alkyle ($C_1$-$C_6$) linéaire ou ramifié, acyle ($C_1$-$C_6$) linéaire ou ramifié, aryle, arylalkyle ($C_1$-$C_6$) linéaire ou ramifié, ou hétéroarylalkyle ($C_1$-$C_6$) linéaire ou ramifié,

Y représente un atome d'oxygène, de soufre, un groupement $NR_3$, le groupement $R_3$ ayant la même définition que précédemment, ou peut représenter une liaison simple quand X représente un groupement $NR'_3$ dans lequel $R'_3$ représente un groupement hétéroarylalkyle ($C_1$-$C_6$) linéaire ou ramifié,

T représente un atome d'azote quand la liaison qui le relie avec l'atome de carbone voisin est simple (——), un atome de carbone ou un groupement CH selon que la liaison qui le relie avec l'atome de carbone voisin est simple (——) ou double ($\overset{----}{——}$),

A représente une liaison simple ou un groupement choisi parmi alkylène ($C_1$-$C_6$) (éventuellement substitué par un ou plusieurs groupements alkyle ($C_1$-$C_6$) linéaire ou ramifié, aryle, arylalkyle ($C_1$-$C_6$) linéaire ou ramifié, cycloalkyle, hétérocycloalkyle, ou hétéroaryle), arylène, cycloalkylène, hétérocycloalkylène,

hétéroarylène, et un groupement $-SO_2-R_4-$ (la partie $SO_2$ étant reliée à T) dans lequel $R_4$ représente un groupement choisi parmi alkylène ($C_1$-$C_6$) linéaire ou ramifié, arylène, arylalkylène ($C_1$-$C_6$) linéaire ou ramifié, cycloalkylène, hétérocycloalkylène, et hétéroarylène,

W représente un groupement choisi parmi hydroxy, alkoxy ($C_1$-$C_6$) linéaire ou ramifié, aryloxy, arylalkoxy ($C_1$-$C_6$) linéaire ou ramifié, cycloalkyloxy, hétérocycloalkyloxy, hétéroaryloxy, amino (lui-même pouvant être substitué par un ou deux groupements, identiques ou différents, indépendamment l'un de l'autre, choisis parmi alkyle ($C_1$-$C_6$) linéaire ou ramifié, aryle, arylalkyle ($C_1$-$C_6$) linéaire ou ramifié, ou cycloalkyle), et hydroxyamino,

$U_1$ représente un atome d'oxygène, de soufre ou une chaîne alkylène ($C_1$-$C_6$) linéaire ou ramifié, dont un ou plusieurs des atomes de carbone peuvent éventuellement être remplacés par un ou plusieurs hétéroatomes choisis parmi oxygène, azote et soufre, ladite chaîne alkylène étant éventuellement substituée par un ou plusieurs groupements, identiques ou différents, choisis parmi atomes d'halogène, groupements hydroxy, alkyle ($C_1$-$C_6$) linéaire ou ramifié, et alkoxy ($C_1$-$C_6$) linéaire ou ramifié,

$V_1$ représente un groupement arylène, hétéroarylène ou hétérocycloalkylène,

$U_2$ représente une liaison simple, un atome d'oxygène, d'azote, de soufre ou une chaîne alkylène ($C_1$-$C_6$) linéaire ou ramifié dont un ou plusieurs atomes de carbone peuvent éventuellement être remplacés par un ou plusieurs groupements, identiques ou différents, choisis parmi atomes d'oxygène, de soufre, d'azote (l'atome d'azote étant substitué par un groupement choisi parmi hydrogène et alkyle ($C_1$-$C_6$) linéaire ou ramifié), et un groupement $SO_2$,

$V_2$ représente un groupement aryle, hétéroaryle ou hétérocycloalkyle,

Ra, Rb, Rc, identiques ou différents, indépendamment l'un de l'autre, représentent chacun un groupement choisi parmi :

- atome d'hydrogène, d'halogène,
- groupement hydroxy, cyano, nitro,
- alkyle ($C_1$-$C_6$) linéaire ou ramifié, alkoxy ($C_1$-$C_6$) linéaire ou ramifié, acyle ($C_1$-$C_6$) linéaire ou ramifié, carboxy, alkoxycarbonyle ($C_1$-$C_6$) linéaire ou ramifié, trihalogénoalkyle ($C_1$-$C_6$) linéaire ou ramifié,
- amino (éventuellement substitué par un ou deux groupements, identiques ou différents, indépendamment l'un de l'autre, alkyle ($C_1$-$C_6$) linéaire ou ramifié, aryle, ou arylalkyle ($C_1$-$C_6$) linéaire ou ramifié),
- aryloxy, arylalkoxy ($C_1$-$C_6$) linéaire ou ramifié, hétéroaryloxy, hétéroarylalkoxy ($C_1$-$C_6$) linéaire ou ramifié,
- et un groupement de formule $-U_1-V_1-U_2-V_2$ dans laquelle $U_1$, $U_2$, $V_1$ et $V_2$ sont tels que définis précédemment,
- ou pris par deux forment ensemble un groupement méthylènedioxy, ou éthylènedioxy (chacun de ces groupements étant éventuellement substitués par un ou deux groupements alkyle ($C_1$-$C_6$) linéaire ou ramifié, aryle, ou arylalkyl ($C_1$-$C_6$) linéaire ou ramifié),

$R_1$ représente :

- un groupement aryle substitué par un à cinq substituants, identiques ou différents, indépendamment l'un de l'autre, choisis parmi halogène, hydroxy, cyano, nitro, carboxy, alkyle ($C_1$-$C_6$) linéaire ou ramifié, alkoxy ($C_1$-$C_6$) linéaire ou ramifié, acyle ($C_1$-$C_6$) linéaire ou ramifié, alkoxycarbonyle ($C_1$-$C_6$) linéaire ou ramifié, trihalogénoalkyle ($C_1$-$C_6$) linéaire ou ramifié (éventuellement substitué par un groupement hydroxy), trihalogénoalkoxy ($C_1$-$C_6$) linéaire ou ramifié, amino (éventuellement substitué par un ou deux groupements alkyle ($C_1$-$C_6$) linéaire ou ramifié, un desdits groupements alkyles étant éventuellement substitué par un groupement choisi parmi amino, alkylamino ($C_1$-$C_6$) linéaire ou ramifié, et dialkylamino ($C_1$-$C_6$) linéaire ou ramifié), aminoalkoxy ($C_1$-$C_6$) linéaire ou ramifié (la partie amino étant éventuellement substituée par un ou deux groupements, identiques ou différents, alkyle ($C_1$-$C_6$) linéaire ou ramifié), alkoxycarbonylalkyle ($C_1$-$C_6$) linéaire ou ramifié, alkyl($C_1$-$C_6$)carbonylamino linéaire ou ramifié, arylakyle ($C_1$-$C_6$) linéaire ou ramifié, aryloxy, arylalkoxy ($C_1$-$C_6$) linéaire ou ramifié, arylamino, arylalkylamino ($C_1$-$C_6$) linéaire ou ramifié, arylsulfanyle, arylalkyl($C_1$-$C_6$)sulfanyle linéaire ou ramifié, hétéroaryle, hétéroarylalkyle ($C_1$-$C_6$) linéaire ou ramifié, hétéroaryloxy, hétéroarylalkoxy ($C_1$-$C_6$) linéaire ou

ramifié, hétéroarylamino, hétéroarylalkyl($C_1$-$C_6$)amino linéaire ou ramifié, hétéroarylsulfanyle, hétéroarylalkyl($C_1$-$C_6$)sulfanyle linéaire ou ramifié,
-un groupement 1,3-dihydro-2*H*-indol-2-one, 3,4-dihydro-2(1*H*)-quinolinone, 1-hydroxy-2(1*H*)-pyridinone,
-ou un groupement hétéroaryle éventuellement substitué,

$R_2$ représente un groupement choisi parmi atome d'hydrogène, groupement alkyle ($C_1$-$C_6$) linéaire ou ramifié, aryle, arylalkyle ($C_1$-$C_6$) linéaire ou ramifié, cycloalkyle, hétérocycloalkyle, hétérocycloalkylalkyle ($C_1$-$C_6$) linéaire ou ramifié, hétéroaryle, et hétéroarylalkyle ($C_1$-$C_6$) linéaire ou ramifié,

étant entendu que par:

- groupement aryle, on comprend un groupement phényle, biphényle, naphtyle, tétrahydronaphtyle, ou dihydronaphtyle, chacun de ces groupements étant éventuellement substitué de façon identique ou différente, par un ou plusieurs groupements choisis parmi atome d'halogène, groupement hydroxy, cyano, nitro, alkyle ($C_1$-$C_6$) linéaire ou ramifié (éventuellement substitué par un ou plusieurs groupements choisis parmi hydroxy, amino, mono ou dialkylamine ($C_1$-$C_6$) linéaire ou ramifié), trihalogénoalkyle ($C_1$-$C_6$) linéaire ou ramifié, alkoxy ($C_1$-$C_6$) linéaire ou ramifié, aryloxy, acyle ($C_1$-$C_6$) linéaire ou ramifié, carboxy, alkoxycarbonyle ($C_1$-$C_6$) linéaire ou ramifié, et amino (substitué éventuellement par un ou deux groupements, identiques ou différents, alkyle ($C_1$-$C_6$) linéaire ou ramifié),

- groupement cycloalkyle, on comprend un groupement mono ou bicyclique, comportant de 3 à 8 atomes de carbone,

- hétérocycloalkyle, on comprend un groupement mono ou bicyclique, saturé ou insaturé, à caractère non aromatique, de 5 à 12 chaînons contenant un, deux ou trois hétéroatomes, identiques ou différents, choisis parmi oxygène, azote et soufre, étant entendu que l'hétérocycloalkyle peut être éventuellement substitué, de façon identique ou différente, par un ou plusieurs groupements choisis parmi atome d'halogène, groupement hydroxy, alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, aryloxy, arylalkoxy ($C_1$-$C_6$) linéaire ou ramifié, amino (substitué éventuellement par un ou deux groupements alkyle ($C_1$-$C_6$) linéaire ou ramifié), acyle ($C_1$-$C_6$) linéaire ou ramifié, alkoxycarbonyle ($C_1$-$C_6$) linéaire ou ramifié, nitro, et oxo,

- hétéroaryle, on comprend un hétérocycloalkyle tel que défini précédemment, mono ou bicyclique, dont au moins un des cycles possède un caractère aromatique, le ou lesdits hétéroatomes pouvant se situer, dans le cas d'un système bicyclique, sur le cycle à caractère aromatique ou sur le cycle partiellement insaturé, étant entendu que l'hétéroaryle peut éventuellement être substitué par un ou plusieurs groupements, identiques ou différents, tels que définis dans la définition des substituants de l'hétérocycloalkyle,

leurs isomères ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Parmi les hétérocycloalkyles, on peut citer à titre non limitatif les hétérocycloalkyles tels que pipéridine, pipérazine ou morpholine.

Parmi les hétéroaryles, on peut citer à titre non limitatif les hétéroaryles tels que pyridine, pyrimidine, quinoline, isoquinoline, 1,3-dihydro-2*H*-pyrrolopyridine-2-one, 3*H*-imidazopyridine, 1*H*-pyrrolopyridine, 1,2,3,4-tétrahydronaphtpyridine ou 2,3-dihydro-1*H*-pyrrolopyridine.

Les composés préférés de l'invention sont ceux pour lesquels X représente un atome de soufre ou un groupement $NR_3$ avec $R_3$ tel que défini dans la formule (I).

Les composés préférés de l'invention sont ceux pour lesquels Y représente un atome d'oxygène.

Les substituants $R_1$ préférés selon l'invention sont les groupements choisis parmi phényle éventuellement substitué par un groupement tel que défini dans la formule (I), quinolyle éventuellement substitué, et pyridinyle éventuellement substitué.

Les substituants $R_2$ préférés selon l'invention sont les groupements choisis parmi aryle et hétéroaryle, chacun de ces groupements étant éventuellement substitué. Selon une variante avantageuse, le substituant $R_2$ préféré est le groupement pyridinyle.

Les substituants $-U_1-V_1-U_2-V_2$ préférés selon l'invention sont les substituants dans lesquels $U_1$ représente une chaîne alkylène ($C_1$ -$C_4$) linéaire dont l'un des atomes de carbone est remplacé par un atome d'oxygène, $V_1$

représente un groupement arylène, $U_2$ représente une liaison simple et $V_2$ représente un groupement aryle éventuellement substitué par un des groupements tels que définis dans la formule (I).

D'une façon particulièrement avantageuse, le substituant $-U_1-V_1-U_2-V_2$ préféré est le groupement [1,1'-biphényl]-4-ylméthoxy.

Selon une variante préférentielle de l'invention, les composés préférés sont ceux pour lesquels un des groupements Ra, Rb, ou Rc représente un groupement de formule $-U_1-V_1-U_2-V_2$ telle que définie dans la formule (I).

Selon une variante avantageuse de l'invention, les composés préférés sont ceux pour lesquels X représente un atome de soufre et Y représente un atome d'oxygène.

Selon une autre variante particulièrement avantageuse de l'invention, les composés préférés sont ceux pour lesquels :

- X représente un atome de soufre,
- Y représente un atome d'oxygène,
- $R_1$ représente un groupement phényle éventuellement substitué ou un groupement pyridinyle éventuellement substitué,
- A représente une liaison simple quand T représente un atome de carbone ou un groupement CH.

Selon une troisième variante avantageuse, les composés préférés de l'invention sont ceux pour lesquels :

- X représente un atome de soufre,
- Y représente un atome d'oxygène,
- $R_1$ représente un groupement phényle éventuellement substitué par un groupement tel que défini dans la formule (I),
- A représente un groupement alkylène (éventuellement substitué par un groupement alkyle ($C_1$-$C_6$) linéaire ou ramifié, aryle, ou arylalkyle ($C_1$-$C_6$) linéaire ou ramifié) ou un groupement arylène quand T représente un atome d'azote.

Selon une quatrième variante avantageuse, les composés préférés de l'invention sont les composés de formule (Ibis) :

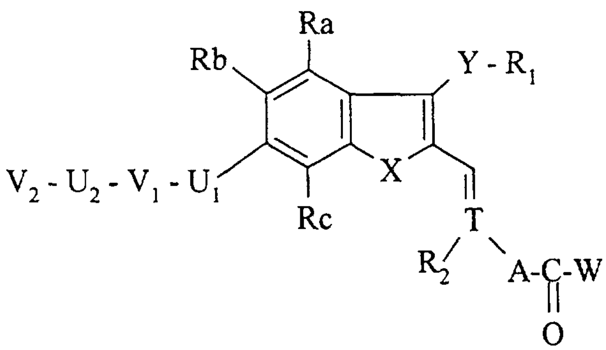

**(I bis)**

dans laquelle :

| | |
|---|---|
| X | représente un atome de soufre, |
| Y | représente un atome d'oxygène, |
| $R_1$ | représente un groupement phényle éventuellement substitué ou un groupement pyridinyle éventuellement substitué, |
| A | représente une liaison simple, |
| T | représente un atome de carbone, |
| Ra et Rc | représentent chacun un atome d'hydrogène, |
| $U_1$ | représente une chaîne alkylènoxy ($C_1$-$C_4$) linéaire, |
| $V_1$ | représente un groupement arylène, |
| $U_2$ | représente une liaison simple, |
| $V_2$ | représente un groupement aryle, |
| Rb | représente un groupement $U_1$-$V_1$-$U_2$-$V_2$ tel que défini précédemment, |
| $R_2$ | représente un groupement hétéroaryle, |

W représente un groupement tel que défini dans la formule (I).

D'une façon très avantageuse, les composés préférés de l'invention sont les composés de formule (Ibis) dans laquelle Rb et $-U_1-V_1-U_2-V_2$ représentent chacun un groupement [1,1'-biphényl]-4-ylméthoxy.

D'une autre façon très avantageuse, les composés préférés de l'invention sont les composés de formule (Ibis) dans laquelle $R_2$ représente un groupement pyridinyle.

D'une troisième façon très avantageuse, les composés préférés de l'invention sont les composés de formule (Ibis) dans laquelle $R_1$ représente :

- un groupement phényle éventuellement substitué par un à trois groupements choisis parmi atome d'halogène, groupement alkyle ($C_1$-$C_6$) linéaire ou ramifié, hétéroaryle, hétéroarylalkoxy ($C_1$-$C_6$) linéaire ou ramifié, alkoxy ($C_1$-$C_6$) linéaire ou ramifié, amino, et aminoalkoxy ($C_1$-$C_6$) linéaire ou ramifié, la partie amino pouvant être substituée (dans chacun de ces deux groupements) par un ou deux groupements, identiques ou différents, alkyles ($C_1$-$C_6$) linéaire ou ramifié,
- ou un groupement hétéroaryle choisi parmi pyridinyle et quinolinyle éventuellement substituée par un atome d'halogène ou un groupement alkyle ($C_1$-$C_6$) linéaire ou ramifié.

Les composés préférés selon l'invention sont :

- l'acide (E)-3-[5,6-bis([1,1'-biphényl]-4-ylméthoxy)-3-(4-chlorophénoxy)benzo[*b*]thiophène-2-yl]-2-(4-pyridinyl)-2-propènoïque,
- le (*E*)-3-[5,6-bis-([1,1'-biphényl]-4-ylméthoxy)-3-(4-chlorophénoxy)benzo[*b*]thiophène-2-yl]-2-(4-pyridinyl)-2-propènoate d'éthyle,
- l'acide (*E*)-3-[5,6-bis-([1,1'-biphényl]-4-ylméthoxy)-3-(3-pyridinyloxy)benzo[*b*]thiophène-2-yl]-2-(4-pyridinyl)-2-propènoïque,
- l'acide 3-(*E*)-{5,6-bis-([1,1'-biphényl]-4-ylméthoxy)-3-[6-(méthyl)pyridinyl-3-oxy]benzo[*b*] thiophène-2,yl}-2-(4-pyridinyl)-2-propènoïque,
- l'acide 3-(*E*)-[5,6-bis-([1,1'-biphényl]-4-ylméthoxy)-3-(6-quinolinyloxy)benzo[*b*]thiophène-2-yl]-2-(4-pyridinyl)-2-propènoïque,
- l'acide (*E*)-3-[5,6-bis-([1,1'-biphényl]-2-ylméthoxy)-3-(4-chlorophénoxy)benzo[*b*]thiophène-2-yl]-2-(4-pyridinyl)-2-propènoïque,
- l'acide (E)-3-[5,6-bis([1,1'-biphényl]-3-ylméthoxy)-3-(4-chlorophénoxy)benzo[*b*]thiophène-2-yl]-2-(4-pyridinyl)-2-propénoïque,
- l'acide (*E*)-3-[5,6-bis([1,1'-biphényl]-4-ylméthoxy)-3-(4-chloro-3-fluorophénoxy)benzo[*b*] thiophène-2-yl]-2-(4-pyridinyl)-2-propénoïque,
- l'acide (*E*)-3-[5,6-bis([1,1'-biphényl]-4-ylméthoxy)-3-(4-chloro-3,5-diméthylphénoxy)benzo [*b*]thiophène-2-yl]-2-(4-pyridinyl)-2-propénoïque,
- l'acide (*E*)-3-[5,6-bis([1,1'-biphényl]-4-ylméthoxy)-3-(4-chloro-3-méthylphénoxy)benzo[*b*] thiophène-2-yl]-2-(4-pyridinyl)-2-propénoïque,
- l'acide(*E*)-3-{5,6-bis([1,1'-biphényl]-4-ylméthoxy)-3-[4-(4-pyridinyloxy)phénoxy]benzo[*b*]thiophène-2-yl}-2-(4-pyridinyl)-2-propénoïque,
- l'acide (*E*)-3-{5,6-bis([1,1'-biphényl]-4-ylméthoxy)-3-[4-(1*H*-imidazol-1-yl)phénoxy]benzo [*b*]thiophène-2-yl}-2-(4-pyridinyl)-2-propénoïque,
- l'acide (*E*)-3-[5,6-bis([1,1'-biphényl]-4-ylméthoxy)-3-phénoxybenzo[*b*]thiophène-2-yl]-2-(4-pyridinyl)-2-propénoïque,
- l'acide (*E*)-3-[5,6-bis([1,1'-biphényl]-4-ylméthoxy)-3-(3-fluorophénoxy)benzo[*b*]thiophène-2-yl]-2-(4-pyridinyl)-2-propénoïque,
- l'acide (*E*)-3-[5,6-bis([1,1'-biphényl]-4-ylméthoxy)-3-(3,4-difluorophénoxy)benzo[*b*] thiophène-2-yl]-2-(4-pyridinyl)-2-propénoïque,
- l'acide (*E*)-3-{5,6-bis([1,1'-biphényl]-4-ylméthoxy)-3-[(6-chloro-3-pyridinyl)oxy]benzo[*b*] thiophène-2-yl}-2-(4-pyridinyl)-2-propénoïque.

Les isomères, ainsi que les sels d'addition à un acide ou à une base pharmaceutiquement acceptable, des composés préférés font partie intégrante de l'invention.

L'invention s'étend également au procédé de préparation des composés de formule (I), caractérisé en ce qu'on utilise comme produit de départ un composé de formule (II):

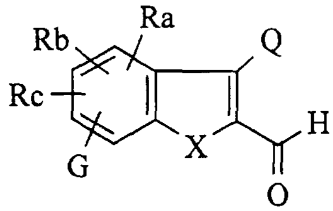

**(II)**

dans laquelle Ra, Rb, Rc et X ont la même signification que dans la formule (I), G représente un groupement hydroxy protégé par un groupement protecteur classiquement utilisé en synthèse organique et Q représente un atome d'halogène ou un groupement hydroxy et de façon préférée, Q représente un atome d'halogène quand X représente un atome de soufre ou un groupement $NR_3$ avec $R_3$ tel que défini dans la formule (I) et Q représente un groupement hydroxy quand X représente un atome d'oxygène,
composé de formule (II) que l'on fait réagir, en condition basique,

- *soit quand Q représente un atome d'halogène :*

  ✧ avec un composé de formule (III),

$$H - Y_1 - R_1 \qquad \textbf{(III)}$$

dans laquelle $R_1$ a la même signification que dans la formule (I) et $Y_1$ représente un atome d'oxygène, de soufre, ou un groupement $NR_3$ avec $R_3$ ayant la même signification que dans la formule (I),
pour conduire aux composés de formule (IV/a) :

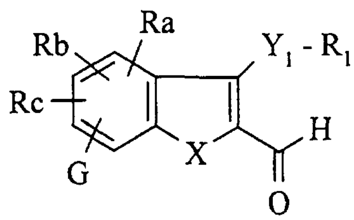

**(IV/a)**

dans laquelle Ra, Rb, Rc, G, $R_1$, X et $Y_1$ sont tels que définis précédemment,

  ✧ ou avec un composé de formule (V) :

$$(HO)_2B - R_1 \qquad \textbf{(V)}$$

dans laquelle $R_1$ a la même signification que dans la formule (I),
pour conduire aux composés de formule (IV/b) :

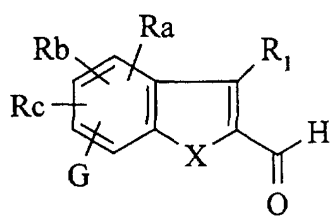

**(IV/b)**

dans laquelle Ra, Rb, Rc, G et $R_1$ sont tels que définis précédemment et $X_1$ représente un groupement $NR_3$ dans lequel $R_3$ représente un groupement hétéroarylalkyle ($C_1$-$C_6$) linéaire ou ramifié,

- *soit quand Q représente un groupement hydroxy,* avec un composé de formule (VI),

Hal - $R_1$ **(VI)**

dans laquelle Hal représente un atome d'halogène et $R_1$ est tel que défini précédemment, pour conduire aux composés de formule (IV/c) :

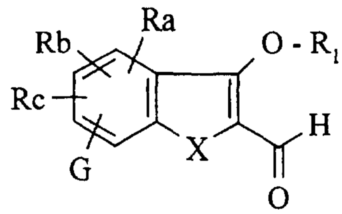

**(IV/c)**

dans laquelle Ra, Rb, Rc, G, X et $R_1$ sont tels que définis précédemment,
l'ensemble des composés de formule (IV/a), (IV/b) et (IV/c) forment les composés de formule (IV) :

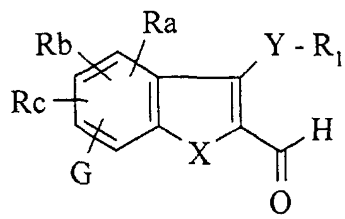

**(IV)**

dans laquelle Ra, Rb, Rc, G, $R_1$, X et Y ont la même définition que dans la formule (I),
composés de formule (IV) :

- ♦ que l'on condense, en présence d'anhydride acétique, avec un composé de formule (VII),

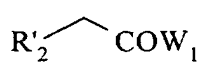

**(VII)**

dans laquelle $R'_2$ a la même signification que $R_2$ dans la formule (I), excepté que $R'_2$ ne peut pas représenter un atome d'hydrogène, et $W_1$ représente un groupement choisi parmi alkoxy ($C_1$-$C_6$) linéaire ou ramifié, aryloxy, arylalkoxy ($C_1$-$C_6$) linéaire ou ramifié, cycloalkyloxy, hétérocycloalkoxy, hétéroaryloxy, et un groupement amino (lui-même étant éventuellement substitué par un ou deux groupements, identiques ou différents, indépendamment l'un de l'autre, choisis parmi alkyle ($C_1$-$C_6$) linéaire ou ramifié, aryle, arylalkyle ($C_1$-$C_6$) linéaire ou ramifié, et cycloalkyle),
pour conduire aux composés de formule (VIII):

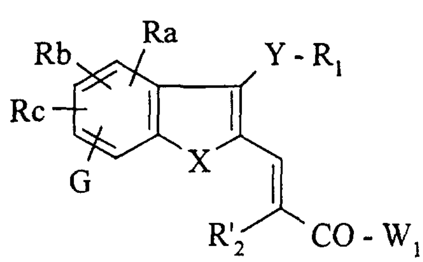

**(VIII)**

dans laquelle Ra, Rb, Rc, G, $R_1$, $R'_2$, X, Y et $W_1$ ont la même définition que précédemment,
composés de formule (VIII) dont on déprotège la fonction hydroxy selon des conditions classique de la synthèse organique, puis que l'on fait réagir en milieu basique avec un composé de formule (IX) :

$$V_2 - U_2 - V_1 - U_1 - Hal \qquad \textbf{(IX)}$$

dans laquelle $U_1$, $V_1$, $U_2$ et $V_2$ sont tels que définis dans la formule (I) et Hal représente un atome d'halogène, pour conduire aux composés de formule (I/a), cas particulier des composés de formule (I) :

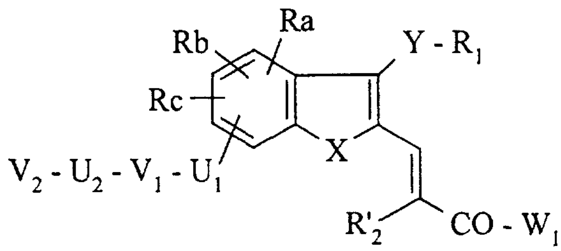

**(I/a)**

dans laquelle Ra, Rb, Rc, $U_1$, $V_1$, $U_2$, $V_2$, $R_1$, $R'_2$, X, Y et $W_1$ ont la même définition que précédemment, composés de formule (I/a) que l'on soumet, si on le désire :

✱ soit à des conditions d'hydrogénation catalytique, en présence de Palladium, pour conduire aux composés de formule (I/b), cas particulier des composés de formule (I):

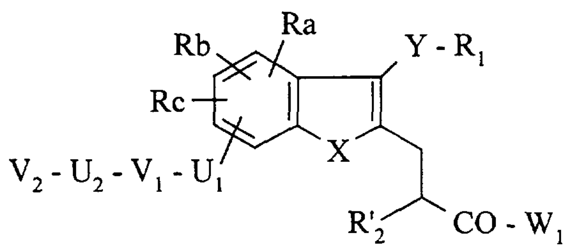

**(I/b)**

dans laquelle Ra, Rb, Rc, $U_1$, $V_1$, $U_2$, $V_2$, $R_1$, $R'_2$, X, Y et $W_1$ sont tels que définis précédemment,

✱ soit à des conditions d'hydrolyse, en milieu basique, pour conduire aux composés de formule (I/c), cas particulier des composés de formule (I) :

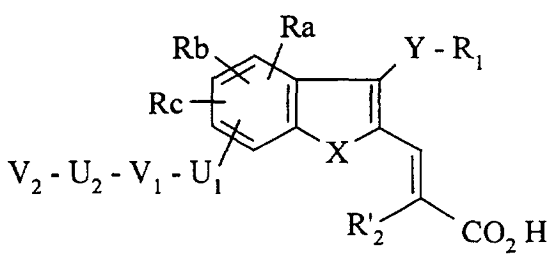

**(I/c)**

dans laquelle Ra, Rb, Rc, $U_1$, $V_1$, $U_2$, $V_2$, $R_1$, $R'_2$, X et Y ont la même définition que précédemment, composés de formule (I/c), dont on réduit, si on le souhaite, la double liaison par hydrogénation catalytique, pour conduire aux composés de formule (I/d), cas particulier des composés de formule (I):

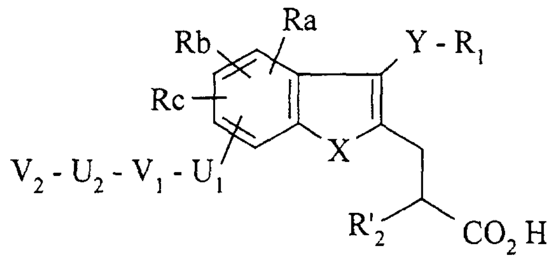

**(I/d)**

dans laquelle Ra, Rb, Rc, $U_1$, $V_1$, $U_2$, $V_2$, $R_1$, $R'_2$, X et Y sont tels que définis précédemment,

- ou que l'on soumet, à l'action d'un ylure de phosphore de formule (X),

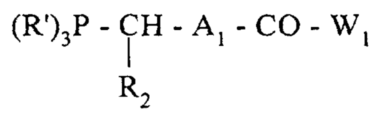

**(X)**

dans laquelle R' représente un groupement alkyle ($C_1$-$C_6$) linéaire ou ramifié, ou un groupement phényle, $R_2$ a la même définition que dans la formule (I), $W_1$ a la même définition que précédemment et $A_1$ représente une liaison simple, un groupement alkylène (éventuellement substitué par un ou plusieurs groupements choisis parmi alkyle ($C_1$-$C_6$) linéaire ou ramifié, aryle, arylalkyle ($C_1$-$C_6$) linéaire ou ramifié, cycloalkyle, hétérocycloalkyle, ou hétéroaryle), un groupement arylène, cycloalkylène, hétérocycloalkylène, ou hétéroarylène, pour conduire aux composés de formule (XI) :

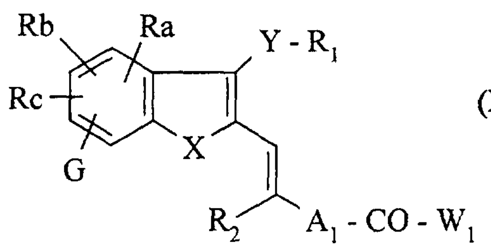

**(XI)**

dans laquelle Ra, Rb, Rc, G, $R_1$, $R_2$, X, Y, $A_1$ et $W_1$ ont la même définition que précédemment, composés de formule (XI) dont on déprotège la fonction hydroxy selon des conditions classiques de la synthèse organique, puis que l'on fait réagir en milieu basique avec un composé de formule (IX) :

$$V_2 - U_2 - V_1 - U_1 \text{ Hal} \qquad \textbf{(IX)}$$

dans laquelle $U_1$, $V_1$, $U_2$ et $V_2$ sont tels que définis dans la formule (I) et Hal représente un atome d'halogène, pour conduire aux composés de formule (I/e), cas particulier des composés de formule (I):

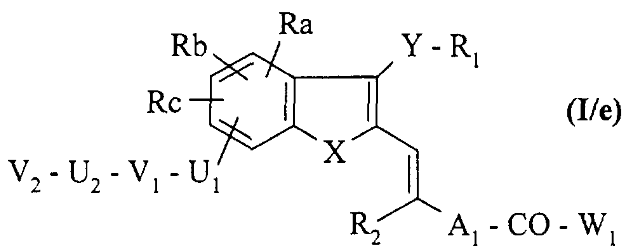

**(I/e)**

dans laquelle Ra, Rb, Rc, $U_1$, $V_1$, $U_2$, $V_2$, $R_1$, $R_2$, $A_1$, X, Y et $W_1$ ont la même définition que précédemment,

composés de formule (I/e) que l'on soumet, si on le désire :

✱ soit à des conditions d'hydrolyse, en condition basique, pour conduire aux composés de formule (I/f), cas particulier des composés de formule (I):

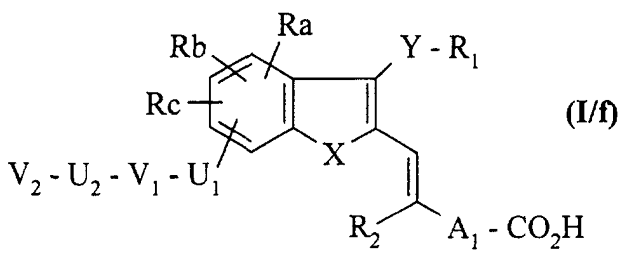

**(I/f)**

dans laquelle Ra, Rb, Rc, $U_1$, $V_1$, $U_2$, $V_2$, $R_1$, $R_2$, X, Y et $A_1$ sont tels que définis précédemment,

✱ soit à des conditions d'hydrogénation catalytique, pour conduire aux composés de formule (I/g), cas particulier des composés de formule (I) :

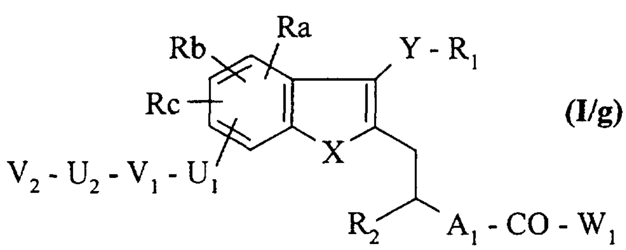

**(I/g)**

dans laquelle Ra, Rb, Rc, $U_1$, $V_1$, $U_2$, $V_2$, $R_1$, $R_2$, X, Y, $A_1$ et $W_1$ sont tels que définis précédemment, composés de formule (I/g) que l'on peut traiter dans des conditions d'hydrolyse basique, pour conduire aux composés de formule (I/h), cas particulier des composés de formule (I):

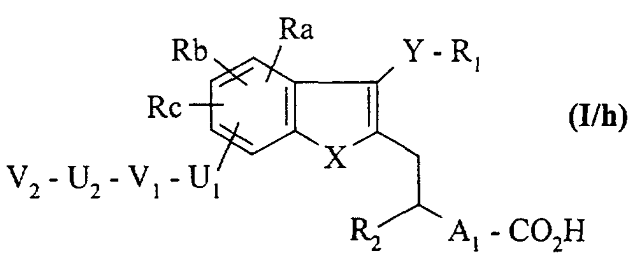

**(I/h)**

dans laquelle Ra, Rb, Rc, $U_1$, $V_1$, $U_2$, $V_2$, $R_1$, $R_2$, X, Y et $A_1$ sont tels que définis précédemment,

- ou dont on déprotège la fonction hydroxy selon des conditions classiques de la synthèse organique, puis que l'on fait réagir en milieu basique avec un composé de formule (IX) :

$$V_2 - U_2 - V_1 - U_1 - Hal \qquad \textbf{(IX)}$$

dans laquelle $U_1$, $V_1$, $U_2$ et $V_2$ sont tels que définis dans la formule (I) et Hal représente un atome d'halogène, pour conduire aux composés de formule (XII) :

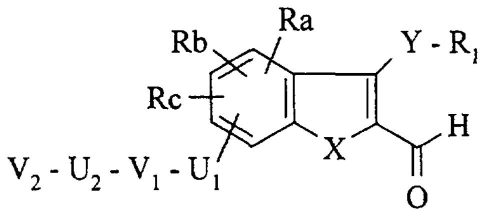

**(XII)**

dans laquelle Ra, Rb, Rc, $U_1$, $V_1$, $U_2$, $V_2$, $R_1$, X et Y ont la même définition que précédemment, composés de formule (XII), dont on réduit la fonction aldéhyde en alcool primaire, pour conduire aux composés de formule (XIII) :

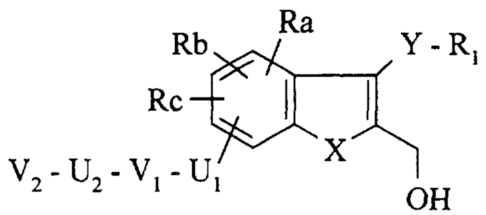

**(XIII)**

dans laquelle Ra, Rb, Rc, $U_1$, $V_1$, $U_2$, $V_2$, $R_1$, X et Y ont la même définition que dans la formule (I), composés de formule (XIII) dont on substitue l'hydroxy terminal par un halogène, selon des conditions classiques, pour conduire aux composés de formule (XIV):

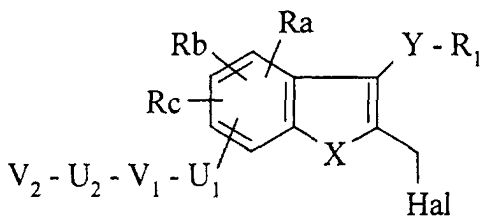

**(XIV)**

dans laquelle Ra, Rb, Rc, $U_1$, $V_1$, $U_2$, $V_2$, $R_1$, X et Y sont tels que définis précédemment, et Hal représente un atome de chlore ou de brome,
composés de formule (XIV):

✱ dont on substitue l'atome d'halogène, en condition basique, par un dérivé aminé de formule (XV):

$R_2$- NH - $A_1$- CO - $W_1$ **(XV)**

dans laquelle $R_2$ a la même définition que dans la formule (I) et, $A_1$, $W_1$ ont la même signification que précédemment,
pour conduire aux composés de formule (I/i), cas particulier des composés de formule (I):

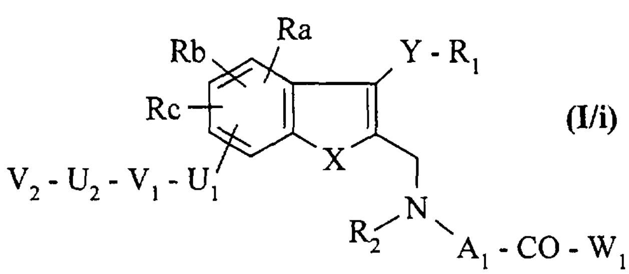

**(I/i)**

dans laquelle Ra, Rb, Rc, $U_1$, $V_1$, $U_2$, $V_2$, $R_1$, $R_2$, X, Y, $A_1$ et $W_1$ sont tels que définis précédemment, composés de formule (I/i), dont on hydrolyse en condition basique le groupement carbonyle terminal, pour conduire aux composés de formule (I/j), cas particulier des composés de formule (I):

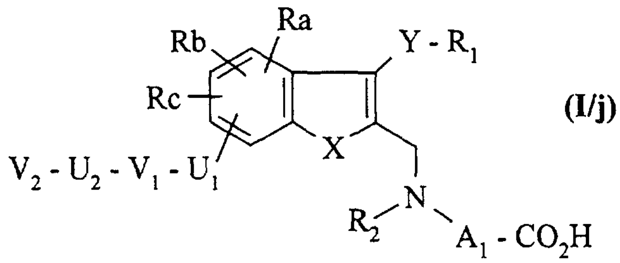

**(I/j)**

dans laquelle Ra, Rb, Rc, $U_1$, $V_1$, $U_2$, $V_2$, $R_1$, $R_2$, X, Y et $A_1$ sont tels que définis précédemment,

✱ ou que l'on traite par de l'azidure de sodium dans un premier temps, et dont on réduit l'azide obtenue en amine primaire dans des conditions d'hydrogénation catalytique, pour conduire aux composés de formule (XVI) :

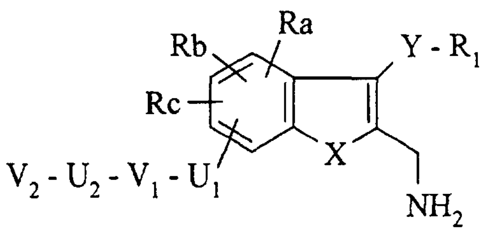

**(XVI)**

dans laquelle Ra, Rb, Rc, $U_1$, $V_1$, $U_2$, $V_2$, $R_1$, X et Y ont la même définition que dans la formule (I), composés de formule (XVI) que l'on condense, en condition basique, sur un dérivé chlorosulphonyle de formule (XVII) :

$$Cl - SO_2 - R_4 - CO - W_1 \qquad \textbf{(XVII)}$$

dans laquelle $R_4$ a la même définition que dans la formule (I), et $W_1$ est tel que défini précédemment, pour conduire aux composés de formule (I/k), cas particulier des composés de formule (I),

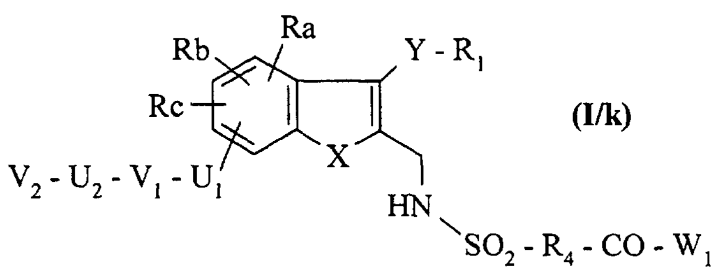

**(I/k)**

dans laquelle Ra, Rb, Rc, $U_1$, $V_1$, $U_2$, $V_2$, $R_1$, $R_4$, X, Y et $W_1$ sont tels que définis précédemment, composés de formule (I/k) :

- que l'on soumet, si on le souhaite, à des conditions d'hydrolyse en condition basique, pour conduire aux composés de formule (I/l), cas particulier des composés de formule (I):

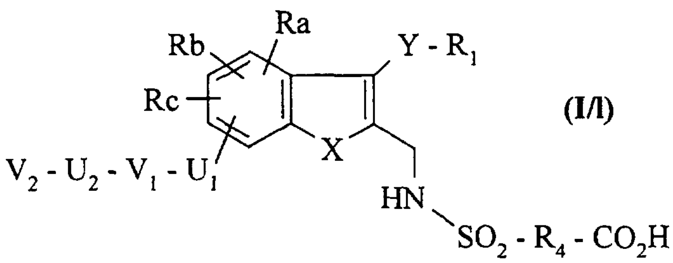

(I/l)

dans laquelle Ra, Rb, Rc, $U_1$, $V_1$, $U_2$, $V_2$, $R_1$, $R_4$, X et Y sont tels que définis précédemment,

- ou que l'on condense, en milieu basique, avec un composé de formule (XVIII) :

$$Hal - R'_2 \quad \textbf{(XVIII)}$$

dans laquelle Hal représente un atome d'halogène tel que l'iode, et $R'_2$ a la même définition que précédemment, pour conduire aux composés de formule (I/m), cas particulier des composés de formule (I):

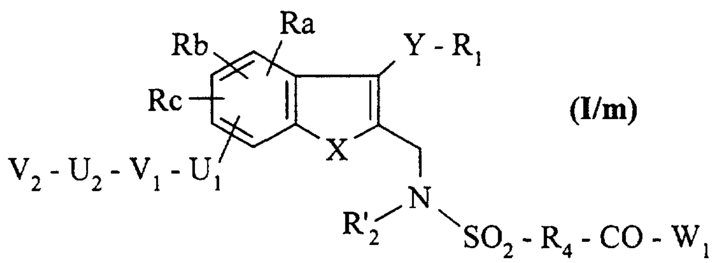

(I/m)

dans laquelle Ra, Rb, Rc, $U_1$, $V_1$, $U_2$, $V_2$, $R_1$, $R'_2$, $R_4$, X, Y et $W_1$ sont tels que définis précédemment, composés de formule (I/m) que l'on traite par des conditions d'hydrolyse en milieu basique, pour conduire aux composés de formule (I/n), cas particulier des composés de formule (I) :

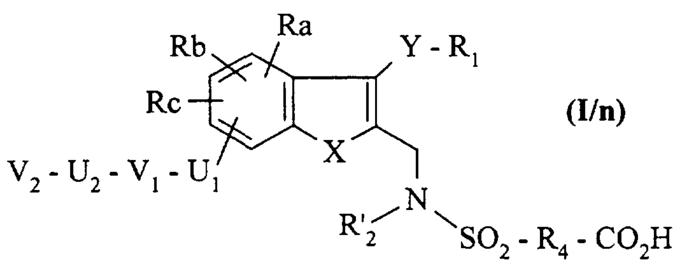

(I/n)

dans laquelle Ra, Rb, Rc, $U_1$, $V_1$, $U_2$, $V_2$, $R_1$, $R'_2$, $R_4$, X et Y sont tels que définis précédemment,

l'ensemble des composés de formule (I/c), (I/d), (I/f), (I/h), (I/j), (I/l) et (I/n) formant les composés de formule (I') :

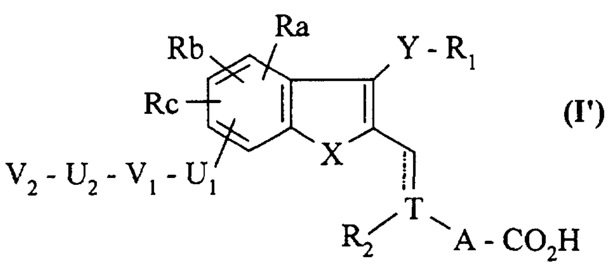

(I')

dans laquelle Ra, Rb, Rc, $U_1$, $V_1$, $U_2$, $V_2$, $R_1$, $R_2$, X, Y, Z et A sont tels que définis dans la formule (I), composés de formule (I') que l'on met en réaction avec une hydroxylamine-O-substituée, pour conduire, après déprotection de la fonction hydroxylamine, aux composés de formule (I/o), cas particulier des composés de formule (I) :

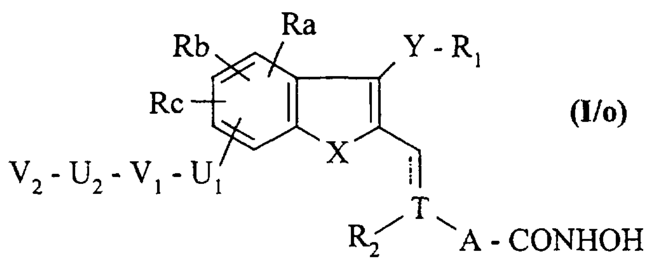

**(I/o)**

dans laquelle Ra, Rb, Rc, $U_1$, $V_1$, $U_2$, $V_2$, $R_1$, $R_2$, X, Y, Z et A sont tels que définis précédemment, les composés (I/a) à (I/o) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

Les composés de formule (II) sont obtenus selon des méthodes classiques de synthèse organique. Notamment les composés de formule (II) dans laquelle X représente un atome d'oxygène et Q un groupement hydroxy, sont obtenus à partir de composés de formule (II/A):

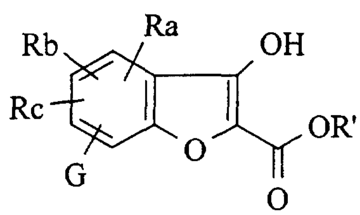

**(II/A)**

dont le schéma de synthèse est décrit dans *J. Med. Chem.,* 1992, 35, 958-965, et dans laquelle Ra, Rb, Rc, G ont les mêmes définitions que précédemment et R' représente un groupement alkyle ($C_1$-$C_6$) linéaire ou ramifié, et dont on protège la fonction hydroxy, en condition basique, par un groupement trialkylsilyle, puis dont on réduit la fonction ester en fonction alcool primaire par action de $LiAlH_4$ par exemple, cette dernière étant ensuite oxydée en fonction aldéhyde, puis dont on déprotège la fonction alcool sous l'action de $nBu_4NF$, permettant d'obtenir les composés particuliers de formule (II) dans laquelle X représente un atome d'oxygène et Q représente un groupement hydroxy.

Les composés particuliers de formule (II) dans laquelle X représente un groupement $NR_3$ sont obtenus à partir de composés de formule (II/B) :

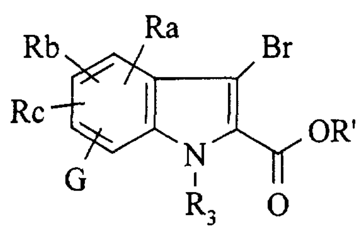

**(II/B)**

dont le schéma de synthèse est décrit dans *Heterocycles,* 1992, 34 (12), 2349-62 et *Synthesis,* 1984, 862-865, et dans laquelle Ra, Rb, Rc, G, $R_3$ ont les mêmes définitions que précédemment et R' représente un groupement alkyle ($C_1$-$C_6$) linéaire ou ramifié, et dont la fonction ester est réduite en fonction alcool primaire, celle-ci étant ensuite oxydée sous l'action du dioxyde de Manganèse en fonction aldéhyde, pour conduire aux composés de formule (II) dans laquelle X représente un groupement $NR_3$ et Q un atome d'halogène.

Les composés particuliers de formule (II) dans laquelle X représente un atome de soufre sont obtenus à partir

de composés de formule (II/C) :

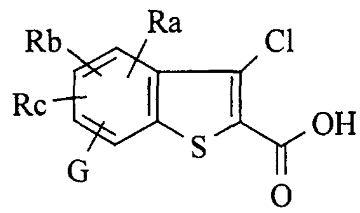

**(II/C)**

dont le schéma de synthèse est décrit dans *J. Heterocyclic. Chem.,* 1971, 8, 711-714, et dans laquelle Ra, Rb, Rc, et G ont les mêmes définitions que précédemment, et dont la fonction acide carboxylique est d'abord réduite en alcool primaire puis oxydée en aldéhyde pour conduire aux composés de formule (II) dans laquelle X représente un atome de soufre et Q un atome d'halogène.

Les composés de formule (III), (V), (VI), (VII), (IX), (X), (XV), (XVII) et (XVIII) sont soit des composés commerciaux, soit obtenus selon les méthodes classiques de synthèse organique.

La présente invention a également pour objet les compositions pharmaceutiques renfermant comme principe actif au moins un composé de formule (I), ses isomères optiques ou un de ses sels d'addition à une base ou un acide pharmaceutiquement acceptable, seul ou en combinaison avec un ou plusieurs excipients ou véhicules inertes non toxiques, pharmaceutiquement acceptable.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les sachets, les gélules, les tablettes, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection et la prise de traitements éventuels associés et s'échelonne de 0,1 mg à 1 g en une ou plusieurs prises par jour.

Les exemples suivants illustrent l'invention mais ne la limitent en aucune façon.

Les produits de départ utilisés sont des produits connus ou préparés selon des modes opératoires connus.

Les différents stades de synthèse conduisent à des intermédiaires de synthèse, utiles pour la préparation des composés de l'invention.

Les structures des composés décrits dans les exemples et les stades de synthèse ont été déterminées selon les techniques spectrophotométriques usuelles (infrarouge, RMN, spectromètre de masse, ...).

**EXEMPLE 1 : (E)-3-[5,6-bis-([1,1'-biphényl]4-ylméthoxy)-3-(4-chlorophénoxy) benzo[*b*]thiophène-2-yl]-2-(4-pyridinyl)-2-propènoate d'éthyle**

**Stade A : Chlorure de 3-chloro-5,6-(méthylènedioxy)-benzo [*b*]thiophène-2-carbonyl**

A une suspension de 0,26 mol d'acide 3,4-(méthylènedioxy)cinnamique dans 365 ml de chlorobenzène, sont additionnés successivement à température ambiante 0,026 mol de pyridine, et goutte à goutte 1,33 mol de $SOCl_2$. Le milieu réactionnel est ensuite porté au reflux pendant 2 jours. Après retour à température ambiante, un précipité se forme. Après filtration, rinçage à l'hexane et séchage, on obtient 51,2 g du produit attendu.

**Stade B : Acide 3-chloro-5,6-(méthylènedioxy)-benzo[*b*]thiophène-2-carboxylique**

A une solution de 70 mmol du composé du stade A dans 250 ml de dioxanne, est ajouté 40 ml d'eau. Après 20 heures de reflux, puis retour à température ambiante, un précipité se forme. Après filtration, rinçage à l'eau jusqu'à neutralité, le précipité est séché sur $P_2O_5$ sous pression réduite permettant d'isoler le produit attendu.

**Stade C : (3-Chloro-5,6-(méthylènedioxy)-benzo[*b*]thiophène-2-yl)méthanol**

A une solution de 0,15 mol de $LiAlH_4$ dans 450 ml de tétrahydrofurane, sous atmosphère inerte, est additionné, à 5°C, 0,14 mol du composé obtenu dans le stade B. Après 2 heures de réaction à température ambiante, la réaction est hydrolysée par addition de 85 ml d'isopropanol et 31 ml d'une solution saturée en chlorure de sodium. Après 12 heures d'agitation à température ambiante, le mélange réactionnel est filtré sur célite. La phase organique est alors

concentrée sous pression réduite, reprise par du dichlorométhane, lavée à l'eau puis par une solution saturée en NaCl. Après séchage de la phase organique sur sulfate de calcium, la solution est concentrée sous pression réduite permettant d'obtenir le produit attendu.

**Stade D : 3-Chloro-5,6-(méthylènedioxy)-benzo[*b*]thiophène-2-carbaldéhyde**

A une suspension de 0,12 mol du composé obtenu dans le stade C dans 925 ml de dioxane anhydre, est additionné à température ambiante sous atmosphère inerte 3,5 équivalents de $MnO_2$. Après 3,5 heures de réaction au reflux, le milieu réactionnel est filtré à chaud sur célite, rincé au dioxane, puis le filtrat est concentré sous pression réduite. Le résidu obtenu est repris par 100 ml d'acétate d'éthyle et la solution obtenue est portée au reflux puis ramenée à température ambiante. Un précipité se forme, celui-ci est filtré, rincé à l'acétate d'éthyle, puis à l'eau et au pentane, et enfin séché sous vide permettant d'isoler le produit attendu.

**Stade E : 3-(4-Chlorophénoxy)-5,6-(méthylènedioxy)benzo[*b*]thiophène-2-carbaldéhyde**

A une solution de 0,10 mol de 4-chlorophénol dans 250 ml de diméthylformamide, sont additionnés à température ambiante et sous atmosphère inerte 1 équivalent d'hydrure de sodium, puis 0,094 mol du produit obtenu dans le stade D. Après 12 heures de réaction à 80°C, le milieu réactionnel est concentré sous pression réduite. Le résidu est alors dilué dans l'acétate d'éthyle, lavé à l'eau puis par une solution aqueuse de NaOH puis de NaCl, séché sur sulfate de calcium, filtré et concentré sous pression réduite. Une chromatographie sur gel de silice (Dichlorométhane/ Acétate d'éthyle : 98/2) permet d'isoler le produit attendu.

**Stade F : (E)-3-[3-(4-Chlorophénoxy)-5,6-(méthylènedioxy)benzo[*b*]thiophène-2-yl]-2-(4-pyridinyl)-2-propènoate d'éthyle**

Une solution contenant 85 mmol du produit obtenu dans le stade E, 127 mmol de 4-pyridylacétate d'éthyle et 75 ml d'anhydride acétique est portée à 100°C pendant 18 heures. Après retour à température ambiante, la réaction est hydrolysée par une solution saturée en $NaHCO_3$, extraite à l'acétate d'éthyle. Les phases organiques sont ensuite lavées à l'eau, puis par une solution de Nacl, séchées sur sulfate de calcium, filtrées et concentrées sous pression réduite. Une chromatographie sur gel de silice (dichlorométhane/éthanol : 98/2) permet d'isoler le produit attendu.

**Stade G : (E)-3-[3-(4-chlorophénoxy)-5,6-(dihydroxy)-benzo[*b*]thiophène-2-yl]-2-(4-pyridinyl)-2-propénoate d'éthyle**

A une solution de 0,025 mol du produit obtenu dans le stade F dans 170 ml de dichlorométhane anhydre est additionné goutte à goutte à 5°C, sous argon, 0,1 mol de $BBr_3$ en solution 1M dans du dichlorométhane. Après 2 heures d'agitation, 125 ml d'alcool sont additionnés goutte à goutte au milieu réactionnel puis celui-ci est concentré sous pression réduite. Le résidu est repris à l'acétate d'éthyle et agité pendant 30 minutes. Le précipité formé est filtré, rincé à l'acétate d'éthyle puis séché sous vide, permettant d'isoler le produit attendu.

**Stade H : (E)-3-[5,6-bis([1,1'-biphényl]-4-ylméthoxy)-3-(4-chlorophénoxy) benzo[*b*]thiophène-2-yl]-2-(4-pyridinyl)-2-propénoate d'éthyle**

A une solution de 2,1 mmol du produit obtenu dans le stade G, 4,6 mmol de 4-(chlorométhyl)-1,1'-biphényle dans 30 ml de diméthylformamide anhydre sont ajoutés 7,5 mmol de $K_2CO_3$. Après 12 heures à 80°C, le milieu réactionnel est ramené à température ambiante et 30 ml d'eau sont ajoutés entraînant la formation d'un précipité. Celui-ci est filtré, rincé à l'eau puis séché sous vide. Une chromatographie sur gel de silice (dichlorométhane/acétate d'éthyle : 95/5) permet d'isoler le produit attendu.
*Point de fusion : 212°C*

| *Microanalyse élémentaire:* | | | | | |
|---|---|---|---|---|---|
| | *%C* | *%H* | *%S* | *%Cl* | *%N* |
| *calculé* | *75,03* | *4,79* | *4,01* | *4,43* | *1,75* |
| *trouvé* | *75,34* | *4,97* | *3,62* | *4,69* | *1,81* |

**EXEMPLE 2 :Acide (E)-3-[5,6-bis-([1,1'-biphényl]4-ylméthoxy)-3-(4-chlorophénoxy) benzo[*b*]thiophène-2-yl]-2-(4- pyridinyl)-2-propènoïque**

Une solution contenant 1,5 mmol du produit de l'exemple 1, 3 ml d'une solution aqueuse 1N de soude et 30 ml d'éthanol est portée au reflux pendant 12 heures. Après retour à température ambiante, la réaction est concentrée sous pression réduite, puis le résidu est dilué dans de l'eau, puis repris à l'éther éthylique. La phase organique est alors acidifiée par addition de 6 ml d'une solution d'HCl 1N. Un précipité se forme, celui-ci étant filtré, rincé à l'eau, puis séché sous pression réduite, permettant d'obtenir le composé attendu.

| *Microanalyse élémentaire :* | | | | | |
|---|---|---|---|---|---|
| | *%C* | *%H* | *%Cl* | *%N* | *%S* |
| *calculé* | *74,65* | *4,44* | *4,59* | *1,81* | *4,15* |
| *trouvé* | *74,69* | *4,46* | *4,50* | *1,90* | *4,04* |

**EXEMPLE 3 : (E)-3-[5,6-bis-([1,1'-biphényl]-4-ylméthoxy)-3-(4-chlorophénoxy) benzo[*b*]thiophène-2-yl]-2-(4-pyridinyl)-2-propènoate de sodium**

A une suspension de 1 g du produit de l'exemple 2 dans 2,5 ml de soude 1N est ajoutée de l'eau jusqu'à dilution totale. Une lyophilisation permet d'isoler le produit attendu.

**EXEMPLE 4 : Acide (E)-3-[5,6-bis-([1,1'-biphényl]-4-ylméthoxy)-3-(4-chlorophénoxy)-benzo[*b*]thiophène-2-yl]-2-phényl-2-propènoïque**

On procède comme dans l'exemple 1 des stades A à H en utilisant au stade F, comme réactif, l'acide phényl éthanoïque.

**EXEMPLE 5 : (E)-3-[5,6-bis-([1,1'-biphényl]-4-ylméthoxy)-3-(4-chlorophénoxy)-benzo[*b*]thiophène-2-yl]-propènoate d'éthyle**

A une suspension de 0,02 mol de bromure de (carboéthoxyméthyl)triphénylphosphonium dans 90 ml de tétrahydrofurane, sous atmosphère inerte, est additionnée goutte à goutte, à 0°C, 20 ml d'une solution de tertio-butylate de potassium 1M dans le tétrahydrofurane. Après addition complète et retour à température ambiante, on ajoute 0,01 mol du composé obtenu dans le stade E de l'exemple 1 dilué dans 30 ml de tétrahydrofurane. Après 12 heures, le milieu réactionnel est hydrolysé par addition de 100 ml d'une solution HCl 1N, puis extrait à l'acétate d'éthyle ; les phases organiques rassemblées sont lavées à l'eau, puis par une solution saturée en NaCl, séchées sur sulfate de sodium, filtrées et concentrées sous pression réduite. Une chromatographie sur gel de silice (Pentane/Acétate d'éthyle : 90/10) permet d'isoler le produit attendu.

**EXEMPLE 6 : 2-({[5,6-bis-([1,1'-biphényl]-4-ylméthoxy)-3-(4-chlorophénoxy) benzo[*b*]thiophène-2-yl] méthyl} anilino)acétate d'éthyle**

**Stade 1 : [5,6-(Méthylènedioxy)-3-(4-chlorophénoxy)-benzo[*b*]thiophène-2-yl]méthanol**

A une solution de 24 mmol du composé obtenu dans le stade E de l'exemple 1 dans 100 ml de méthanol est additionné à température ambiante 26 mmol de $NaBH_4$. Après 2 heures de réaction, un équivalent de $NaBH_4$ est ajouté au milieu réactionnel. Après 12 heures de réaction, la solution est concentrée, puis diluée à l'acétate d'éthyle, lavée par une solution HCl 1N, puis par de l'eau, puis par une solution saturée en NaCl, puis séchée sur sulfate de calcium, filtrée et concentrée sous pression réduite. Une chromatographie sur gel de silice (Dichlorométhane/Acétate d'éthyle : 95/5) permet d'isoler le produit attendu.

**Stade 2 : [2-Chlorométhyl-3-(4-chlorophénoxy)-5,6-(méthylènedioxy)]-benzo[*b*]thiophène**

A 4 mmol du composé du stade 1 dilué dans 10 ml de dichlorométhane est additionné, goutte à goutte et à 0°C, 0,63 ml de $SOCl_2$. Après retour à température ambiante, puis chauffage au reflux du dichlorométhane pendant 6 heures, le milieu réactionnel est concentré sous pression réduite, permettant d'obtenir le produit attendu.

**Stade 3 : 2-({[3-(4-Chlorophénoxy)-5,6-(méthylènedioxy)benzo[*b*]thiophène-2-yl] méthyl}anilino) acétate d'éthyle**

Une solution contenant 6,5 mmol du composé obtenu dans le stade 2, dans 16 ml de diméthylformamide, 1,5 équivalents de N-phénylglycine éthyl ester et 1,5 équivalents de $K_2CO_3$ est portée à 80°C pendant 18 heures. Après évaporation du solvant, le résidu est dilué à l'acétate d'éthyle et la phase organique est lavée à l'eau, puis par une solution saturée en NaCl, séchée sur sulfate de calcium, filtrée et évaporée sous pression réduite. Une chromatographie sur gel de silice (Toluène/Acétate d'éthyle : 98/2) permet d'isoler 2,56 g du produit attendu sous forme d'huile.

**Stade 4 : 2-({[5,6-bis-([1,1'-biphényl]-4-ylméthoxy)-3-(4-chlorophénoxy)benzo [*b*]thiophène-2-yl]méthyl}anilino)acétate d'éthyle**

On procède comme dans l'exemple 1 des stades G à H en utilisant comme substrat le produit obtenu dans le stade 3 précédent.

**EXEMPLE 7 : 2-({[5,6-bis-([1,1'-biphényl]-4-ylméthoxy)-3-(4-chlorophénoxy) benzo[*b*]thiophène-2-yl]méthyl} amino)benzoate d'éthyle**

On procède comme dans l'exemple 6, en utilisant au stade 3, comme réactif, l'anthranilate d'éthyle.

**EXEMPLE 8 : 2-[({[5,6-bis-([1,1'-biphényl]-4-ylméthoxy)-3-(4-chlorophénoxy) benzo[*b*]thiophène-2-yl]méthyl} amino)sulfonyl]benzoate de méthyle**

**Stade 1 : [2-Azidométhyl-3-(4-chlorophénoxy)-5,6-(méthylènedioxy)]-benzo[*b*]thiophène**

Une solution contenant 41 mmol du composé obtenu dans le stade 2 de l'exemple 6 et 78 mmol d'azidure de sodium dans 80 ml de diméthylformamide est agitée à température ambiante pendant 48 heures. Le milieu réactionnel est ensuite concentré sous pression réduite. Le résidu est dilué à l'acétate d'éthyle, lavé à l'eau puis par une solution saturée en NaCl. La phase organique est ensuite séchée sur sulfate de calcium, filtrée et évaporée, permettant d'obtenir le produit attendu.

**Stade 2 : [2-Aminométhyl-3-(4-chlorophénoxy)-5,6-(méthylènedioxy)]-benzo[*b*]thiophène**

Une solution contenant 41 mmol du composé obtenu dans le stade 1 précédent et 1 g de Pd/C dans 6,5 ml de chloroforme et 300 ml de méthanol anhydre est placée sous atmosphère d'hydrogène à température ambiante. Après 12 heures, la réaction est filtrée puis concentrée sous pression réduite permettant d'obtenir le produit attendu.

**Stade 3 : 2-[({[-3-(4-Chlorophénoxy)-5,6-(méthylènedioxy)-benzo[*b*]thiophène-2-yl] méthyl}amino)sulfonyl] benzoate de méthyle**

Une solution contenant 6,5 mmol du composé obtenu dans le stade 2 précédent, 6,5 mmol de 2-(chlorosulfonyl)-benzoate de méthyle, 15,7 mmol de N-méthyl-morpholine, dans 50 ml de dichlorométhane est agitée à température ambiante. Après 12 heures, la réaction est lavée à l'eau, puis par une solution saturée en NaCl, séchée sur sulfate de sodium et concentrée sous pression réduite. Une chromatographie sur gel de silice (Dichlorométhane/Acétate d'éthyle : 98/2) permet d'isoler le produit attendu.

**Stade 4 : 2-[({[5,6-bis-([1,1'-biphényl]-4-ylméthoxy)-3-(4-chlorophénoxy)benzo [*b*]thiophène-2-yl]méthyl}amino)sulfonyl]benzoate de méthyle**

On procède comme dans l'exemple 1 des stades G à H en utilisant comme substrat le produit obtenu dans le stade 3 précédent.

**EXEMPLE 9 : Acide 2-[({[5,6-bis-([1,1'-biphényl]-4-ylméthoxy)-3-(4-chlorophénoxy) benzo[*b*]thiophène-2-yl] méthyl}amino)sulfonyl]benzoïque**

On procède comme dans l'exemple 2, en utilisant comme substrat, le composé obtenu dans le stade 4 de l'exemple 8.

**EXEMPLE 10 : Acide (E)-3-[5,6-bis-([1,1'-biphényl]-4-ylméthoxy)-3-(3-pyridinyloxy)-benzo[*b*]thiophène-2-yl]-2-(4-pyridinyl)-2-propènoïque**

On procède comme dans l'exemple 1, en utilisant comme réactif au stade E, la 3-hydroxypyridine, puis on suit le protocole décrit dans l'exemple 2.
*Point de fusion : 237°C*

| *Microanalyse élémentaire :* | | | | |
|---|---|---|---|---|
| | *%C* | *%H* | *%N* | *%S* |
| *calculé* | *76,40* | *4,64* | *3,79* | *4,34* |
| *trouvé* | *76,69* | *4,63* | *3,83* | *4,28* |

**EXEMPLE 11 : Acide (E)-3-[5,6-bis-([1,1'-biphényl]-4-ylméthoxy)-3-(4-chlorophénoxy)-1*H*-2-indolyl]-2-(4-pyridinyl)-2-propènoïque**

On procède comme dans l'exemple 1, du stade E au stade H, en utilisant comme substrat au stade E le 3-bromo-5,6-diméthoxy-1*H*-2-indolecarbaldéhyde, puis on suit le protocole décrit dans l'exemple 2.

**EXEMPLE 12 : Acide (E)-3-[5,6-bis-([1,1'-biphényl]-4-ylméthoxy)-3-[(4-méthoxyphényl)sulfanyl] benzo[*b*]thiophène-2-yl]-2-(4- pyridinyl)-2-propènoïque**

On procède comme dans l'exemple 1, en utilisant comme réactif au stade E, le 4-méthoxybenzènethiol, puis on suit le protocole décrit dans l'exemple 2.

**EXEMPLE 13 : Acide 3-(E)-{3-[4-(2-(diméthylamino)éthoxy)-phénoxy]-5,6-bis-([1,1'-biphényl]-4-ylméthoxy)-benzo[*b*]thiophène-2-yl}-2-phényl-2-propènoïque**

On procède comme dans l'exemple 1 en utilisant comme réactif au stade E, le 2-(diméthylamino)éthoxyphénol, et au stade F l'acide phényl éthanoïque puis on suit le protocole décrit dans l'exemple 2.

**EXEMPLE 14 : Acide (E)-3-[5,6-bis-([1,1'-biphényl]-4-ylméthoxy)-3-(3,4-dichlorophénoxy) benzo[*b*]thiophène-2-yl],2-(4-pyridinyl)-2-propènoïque**

On procède comme dans l'exemple 1, en utilisant comme réactif au stade E le 3,4-dichlorophénol, puis on suit le protocole décrit dans l'exemple 2.

**EXEMPLE 15 : Acide 3-(E)-{5,6-bis-([1,1'-biphényl]-4-ylméthoxy)-3-[6-(méthyl)pyridinyl-3-oxy]-benzo[*b*]thiophène-2-yl}-2-(4-pyridinyl)-2-propènoïque**

On procède comme dans l'exemple 1 en utilisant comme réactif au stade E, la 2-méthyl-5-hydroxy-pyridine, puis on suit le protocole décrit dans l'exemple 2.

| *Microanalyse élémentaire :* | | | | |
|---|---|---|---|---|
| | *%C* | *%H* | *%N* | *%S* |
| *calculé* | *76,58* | *4,82* | *3,72* | *4,26* |
| *trouvé* | *76,84* | *4,72* | *3,77* | *4,15* |

**EXEMPLE 16 : Acide 3-(E)-[5,6-bis-([1,1'-biphényl]-4-ylméthoxy) -3-(6-quinolinyloxy) benzo[*b*]thiophène-2-yl]-2-(4-pyridinyl)-2-propènoïque**

On procède comme dans l'exemple 1 en utilisant comme réactif au stade E la 6-hydroxyquinoléine, puis on suit le protocole décrit dans l'exemple 2.

| *Microanalyse élémentaire :* | | | | |
|---|---|---|---|---|
| | *% C* | *%H* | *%N* | *%S* |
| *calculé* | *77,65* | *4,60* | *3,55* | *4,06* |
| *trouvé* | *78,00* | *4,75* | *3,59* | *3,71* |

**EXEMPLE 17 : Acide (E)-3-[5,6-bis-([1,1'-biphényl]-2-ylméthoxy)-3-(4-chlorophénoxy) benzo[*b*]thiophène-2-yl]-2-(4-pyridinyl)-2-propènoïque**

On procède comme dans l'exemple 1 en utilisant comme réactif au stade H le 2-(bromométhyl)-1,1'-biphényle, puis on suit le protocole décrit dans l'exemple 2.

| *Microanalyse élémentaire :* | | | | | |
|---|---|---|---|---|---|
| | *%C* | *%H* | *%N* | *%S* | *%Cl* |
| *calculé* | *74,65* | *4,44* | *1,81* | *4,15* | *4,59* |
| *trouvé* | *74,97* | *4,55* | *1,88* | *4,06* | *4,57* |

**EXEMPLE 18 : Acide (E)-3-[5,6-bis-(4-phénoxyphénoxy)-3-(4-chlorophénoxy) benzo[*b*]thiophène-2-yl]-2-(4-pyridinyl)-2-propènoïque**

On procède comme dans l'exemple 1, en utilisant comme réactif au stade H le 1-bromo-4-phénoxybenzène, puis on suit le protocole décrit dans l'exemple 2.

**EXEMPLE 19 : Acide (E)-3-{3-(4-chlorophénoxy)-5,6-bis-[4-(4-pyridinylméthyl)phénoxy] benzo[*b*]thiophène-2-yl}-2-(4-pyridinyl)-2-propènoïque**

On procède comme dans l'exemple 1 en utilisant comme réactif au stade H la 4-(4-chlorobenzyl)pyridine, puis on suit le protocole décrit dans l'exemple 2.

**EXEMPLE 20 : Acide (*E*)-3-[5,6-bis([1,1'-biphényl]-3-ylméthoxy)-3-(4-chlorophénoxy)-benzo[*b*]thiophène-2-yl]-2-(4-pyridinyl)-2-propénoïque**

On procède comme dans l'exemple 1 en utilisant comme réactif au stade H le 3-(bromométhyl)-1,1'-biphényle, puis on suit la procédure décrit dans l'exemple 2.
*Point de fusion : 205-210°C*

**EXEMPLE 21: Acide (*E*)-3-[5,6-bis([1,1'-biphényl]-4-ylméthoxy)-3-(4-chloro-3-fluorophénoxy)-benzo[*b*]thiophène-2-yl]-2-(4-pyridinyl)-2-propénoïque**

On procède comme dans l'exemple 1 en utilisant comme réactif au stade E le 4-chloro-3-fluorophénol, puis on suit le protocole décrit dans l'exemple 2.

| *Microanalyse élémentaire :* | | | | |
|---|---|---|---|---|
| | *%C* | *%H* | *%N* | *%S* |
| *calculé* | *72,95* | *4,21* | *1,77* | *4,06* |
| *trouvé* | *72,85* | *4,27* | *1,79* | *3,65* |

**EXEMPLE 22 : Acide (*E*)-3-[5,6-bis([1,1'-biphényl]-4-ylméthoxy)-3-(4-chloro-3,5-diméthylphénoxy)-benzo[*b*]thiophène-2-yl]-2-(4-pyridinyl)-2-propénoïque**

On procède comme dans l'exemple 1 en utilisant comme réactif au stade E le 4-chloro-3,5-diméthyl-phénol, puis on suit le protocole décrit dans l'exemple 2.

| *Microanalyse élémentaire :* | | | | | |
|---|---|---|---|---|---|
| | *%C* | *%H* | *%N* | *%S* | *%Cl* |
| *calculé* | *75,03* | *4,79* | *1,75* | *4,01* | *4,43* |
| *trouvé* | *74,45* | *4,91* | *1,80* | *3,51* | *4,19* |

**EXEMPLE 23 : Acide (*E*)-3-[5,6-bis([1,1'-biphényl]-4-ylméthoxy)-3-(4-chloro-3-méthylphénoxy)-benzo [*b*]thiophène-2-yl]-2-(4-pyridinyl)-2-propénoïque**

On procède comme dans l'exemple 1 en utilisant comme réactif au stade E le 4-chloro-3-méthylphénol, puis on suit le protocole décrit dans l'exemple 2.

| *Microanalyse élémentaire :* | | | | | |
|---|---|---|---|---|---|
| | *%C* | *%H* | *%N* | *%S* | *%Cl* |
| *calculé* | *74,85* | *4,61* | *1,78* | *4,08* | *4,51* |
| *trouvé* | *74,64* | *4,77* | *1,83* | *4,00* | *4,39* |

**EXEMPLE 24 : Acide (*E*)-3-{5,6-bis([1,1'-biphényl]-4-ylméthoxy)-3-[4-(4-pyridinyloxy) phénoxy]-benzo[*b*]thiophène-2-yl}-2-(4-pyridinyl)-2-propénoïque**

On procède comme dans l'exemple 1 en utilisant comme réactif au stade E le 4-pyridinyloxyphénol, puis on suit le protocole décrit dans l'exemple 2.
*Point de fusion : 265°C*

| *Microanalyse élémentaire :* | | | | |
|---|---|---|---|---|
| | *%C* | *%H* | *%N* | *%S* |
| *calculé* | *76,61* | *4,61* | *3,37* | *3,86* |
| *trouvé* | *76,66* | *4,72* | *3,40* | *3,46* |

**EXEMPLE 25 : Acide (*E*)-3-{5,6-bis([1,1'-biphényl]-4-ylméthoxy)-3-[4-(1*H*-imidazol-1-yl) phénoxy]-benzo[*b*]thiophène-2-yl}-2-(4-pyridinyl)-2-propénoïque**

On procède comme dans l'exemple 1 en utilisant comme réactif au stade E le 4-(1*H*-imidazol-1-yl)-phénol, puis on suit le protocole décrit dans l'exemple 2.

| *Microanalyse élémentaire :* | | | | |
|---|---|---|---|---|
| | *%C* | *%H* | *%N* | *%S* |
| *calculé* | *76,20* | *4,64* | *5,23* | *3,99* |
| *trouvé* | *75,97* | *4,68* | *5,17* | *3,92* |

**EXEMPLE 26 : Acide (*E*)-3-[5,6-bis([1,1'-biphényl]-4-ylméthoxy)-3-phénoxy-benzo[*b*] thiophène-2-yl]-2-(4-pyridinyl)-2-propénoïque**

On procède comme dans l'exemple 1 en utilisant comme réactif au stade E le phénol, puis on suit le protocole décrit dans l'exemple 2.

| *Microanalyse élémentaire :* | | | | |
|---|---|---|---|---|
| | *%C* | *%H* | *%N* | *%S* |
| *calculé* | *78,13* | *4,78* | *1,90* | *4,35* |
| *trouvé* | *78,30* | *4,88* | *1,94* | *4,16* |

**EXEMPLE 27 : Acide (*E*)-3-[5,6-bis([1,1'-biphényl]-4-ylméthoxy)-3-(3-fluorophénoxy)-benzo[b]thiophène-2-yl]-2-(4-pyridinyl)-2-propénoique**

On procède comme dans l'exemple 1 en utilisant comme réactif au stade E le 3-fluoro-phénol, puis on suit le protocole décrit dans l'exemple 2.

| *Microanalyse élémentaire :* | | | | |
|---|---|---|---|---|
| | *%C* | *%H* | *%N* | *%S* |
| *calculé* | *76,27* | *4,53* | *1,85* | *4,24* |
| *trouvé* | *76,37* | *4,56* | *1,90* | *4,05* |

**EXEMPLE 28 : Acide (*E*)-3-[5,6-bis([1,1'-biphényl]-4-ylméthoxy)-3-(3,4-difluorophénoxy)benzo[*b*]thiophène-2-yl]-2-(4-pyridinyl)-2-propénoïque**

On procède comme dans l'exemple 1 en utilisant comme réactif au stade E le 3,4-difluorophénol, puis on suit le protocole décrit dans l'exemple 2.

| *Microanalyse élémentaire :* | | | | |
|---|---|---|---|---|
| | *%C* | *%H* | *%N* | *%S* |
| *calculé* | *74,50* | *4,30* | *1,81* | *4,14* |
| *trouvé* | *74,48* | *4,24* | *1,88* | *3,98* |

**EXEMPLE 29 : Acide (*E*)-3-{5,6-bis([1,1'-biphényl]-4-ylméthoxy)-3-[(6-chloro-3-pyridinyl)oxy]-benzo[*b*]thiophène-2-yl}-2-(4-pyridinyl)-2-propénoïque**

On procède comme dans l'exemple 1 en utilisant comme réactif au stade E la 2-chloro-5-hydroxy-pyridine, puis on suit le protocole décrit dans l'exemple 2.

| *Microanalyse élémentaire :* | | | | | |
|---|---|---|---|---|---|
| | *%C* | *%H* | *%N* | *%S* | *%Cl* |
| *calculé* | *70,98* | *4,06* | *3,52* | *4,03* | *4,46* |
| *trouvé* | *71,27* | *4,44* | *3,53* | *3,51* | *4,30* |

**EXEMPLE 30 : Acide (*E*)-3-{3-(4-chlorophénoxy)-5,6-bis[(4'-méthoxy[1,1'-biphényl]-4-yl)méthoxy]-benzo[*b*]thiophèn-2-yl}-2-(4-pyridinyl)-2-propénoïque**

On procède comme dans l'exemple 1 en utilisant comme réactif au stade H le 4-(bromométhyl)-4'-méthoxy-1,1'-biphényl, puis on suit la procédure décrit dans l'exemple 2.
*Point de fusion : 235°C*

| *Microanalyse élémentaire :* | | | | | |
|---|---|---|---|---|---|
| | *%C* | *%H* | *%N* | *%S* | *%Cl* |
| *calculé* | *72,15* | *4,60* | *1,68* | *3,85* | *4,26* |
| *trouvé* | *71,97* | *4,55* | *1,82* | *4,03* | *4,33* |

**EXEMPLE 31 : Acide (*E*)-3-[5,6-bis(2-[1 ,1'-biphényl]-4-yléthoxy)-3-(4-chlorophénoxy)benzo[*b*]thiophèn-2-yl]-2-(4-pyridinyl)-2-propénoïque**

On procède comme dans l'exemple 1 en utilisant comme réactif au stade H le 4-(2-bromoéthyl)-1,1'-biphényl, puis on suit la procédure décrit dans l'exemple 2.

**EXEMPLE 32 : Acide (*E*)-3-[5,6-bis[(4-benzylbenzyl)oxy]-3-(4-chlorophénoxy)-benzo[*b*] thiophèn-2-yl]-2-(4-pyridinyl)-2-propénoïque**

On procède comme dans l'exemple 1 en utilisant comme réactif au stade H le 1-benzyl-4-(bromométhyl) benzène, puis on suit la procédure décrit dans l'exemple 2.

**EXEMPLE 33 : Acide (*E*)-3-{3-(4-chlorophénoxy)-5,6-bis[(4-phénoxybenzyl)oxy]-benzo [*b*]thiophèn-2-yl}-2-(4-pyridinyl)-2-propénoïque**

On procède comme dans l'exemple 1 en utilisant comme réactif au stade H le 1-(bromométhyl)-4-phénoxybenzène, puis on suit la procédure décrit dans l'exemple 2.

**EXEMPLE 34 : Acide (*E*)-3-(3-(4-chlorophénoxy)-5,6-bis{[4-(phénylsulfanyl)benzyl]oxy}benzo[*b*]thiophèn-2-yl)-2-(4-pyridinyl)-2-propénoïque**

On procède comme dans l'exemple 1 en utilisant comme réactif au stade H le 1-(bromométhyl)-4-(phénylsulfanyl)benzène, puis on suit la procédure décrit dans l'exemple 2.

**EXEMPLE 35 : Acide (*E*)-3-(3-(4-chlorophénoxy)-5,6-bis{[4-(phénylsulfonyl)benzyl]oxy}benzo[*b*]thiophèn-2-yl)-2-(4-pyridinyl)-2-propénoïque**

On procède comme dans l'exemple 1 en utilisant comme réactif au stade H le 1-(bromométhyl)-4-(phénylsulfonyl)benzène, puis on suit la procédure décrit dans l'exemple 2.

**EXEMPLE 36 : Acide (*E*)-3-[3-(4-chlorophénoxy)-5,6-bis({4-[(4-phénoxyphényl)sulfonyl] benzyl}oxy)-benzo[*b*] thiophèn-2-yl]-2-(4-pyridinyl)-2-propénoïque**

On procède comme dans l'exemple 1 en utilisant comme réactif au stade H le 1-(bromométhyl)-4-[(4-phénoxyphényl)sulfonyl]benzène , puis on suit la procédure décrit dans l'exemple 2.

**EXEMPLE 37 : Acide (*E*)-3-[6-(benzyloxy)-5-([1,1'-biphényl]-4-ylméthoxy)-3-(4-chlorophénoxy)-benzo[*b*]thiophèn-2-yl]-2-(4-pyridinyl)-2-propénoïque**

**Stade 1 : Acide (*E*)-3-[6-(benzyloxy)-3-(4-chlorophénoxy)-5-hydroxy-benzo[b]thiophèn-2-yl]-2-(4-pyridinyl)-2-propénoïque**

On procède comme dans l'exemple 1 des stades A à H, en utilisant comme réactif au stade H le chlorométhyl-phényle.

**Stade 2 : Acide (*E*)-3-[6-(benzyloxy)-5-([1,1'-biphényl]-4-ylméthoxy)-3-(4-chlorophénoxy)-benzo[*b*]thiophèn-2-yl]-2-(4-pyridinyl)-2-propénoïque**

On procède comme dans le stade H de l'exemple 1, puis on suit la procédure décrite dans l'exemple 2.

| *Microanalyse élémentaire :* | | | | | |
|---|---|---|---|---|---|
| | %C | *%H* | *%N* | %S | *% Cl* |
| *calculé* | *72,46* | *4,34* | *2,01* | *4, 61* | *5, 09* |
| *trouvé* | 73,39 | *4,38* | *2,14* | *4,34* | *5,08* |

**EXEMPLE 38 : Acide (*E*)-3-[6-([1,1'-biphényl]-4-ylméthoxy)-3-(4-chlorophénoxy)-5-phénoxy-benzo[*b*]thiophèn-2-yl]-2-(4-pyridinyl)-2-propénoïque**

On procède comme dans l'exemple 1 en utilisant comme réactif au stade H le 4-chlorométhyl-(1,1'-biphényl) puis le chlorométhyl-phényle.

**EXEMPLE 39 : Acide 3-[6-([1,1'-biphényl]-4-ylméthoxy)-5-([1,1'-biphényl]-4-yloxy)-3-(4-chlorophénoxy)-benzo[*b*]thiophèn-2-yl]-2-phénylpropanoïque**

On procède comme dans l'exemple 1 des stades A à F, en utilisant comme réactif au stade F l'acide phényl éthanoïque à la place du 4-pyridylacétate d'éthyle, puis des stades G à H décrits dans l'exemple 1. Le produit ainsi obtenu est ensuite traité par un courant d'hydrogène en présence de Pd/C à 10 % dans le méthanol pendant 24 heures. Une filtration sur célite en fin de réaction, suivie d'une chromatographie sur gel de silice permet d'isoler le produit attendu.

**EXEMPLE 40 : Acide 2-({[6-([1,1'-biphényl]-4-ylméthoxy)-5-([1,1'-biphényl]-4-yloxy)-3-(4-chlorophénoxy)-benzo[*b*]thiophèn-2-yl]méthyl}amino)benzoïque**

On procède comme dans l'exemple 2 en utilisant comme produit de départ le composé obtenu dans l'exemple 7.

**ETUDE PHARMACOLOGIQUE DES COMPOSES DE L'INVENTION**

**EXEMPLE 41 : Inhibition de l'activité du PAI-1**

L'inhibition de l'activité du PAI-1 a été réalisée in-vitro dans des puits de microplaque dans lesquels la formation puis la lyse d'un caillot de fibrine est suivie en continu en turbidimétrie grâce à un spectrophotomètre. Pour ce faire, en utilisant comme diluant un tampon phosphate 50 mM pH 7,4 contenant 0,05% de sérum albumine bovine, 50 µl de l'inhibiteur est mis en présence avec 50 µl d'une solution de PAI-1 actif recombinant humain à 2 nM pendant 5 minutes à température ambiante. 50 µl d'une solution d'activateur tissulaire du plasminogène à 0,42 nM, 50 µl d'une solution de plasminogène humain à 800 nM et 50 µl d'une solution de fibrinogène à 2 g/l sont ensuite additionnés et la fibrinoformation est déclenchée par l'ajout de 50 µl de thrombine humaine purifiée à 14 nM. En l'absence de produit, l'inhibition de la lyse deux heures après le début de la fibrinoformation, est mesurée par l'absorbance du caillot et représente 100 % de l'activité PAI-1. En l'absence de produit et de PAI-1, la lyse est mesurée par l'absorbance du caillot lysé et représente 0 % de l'activité PAI-1. La concentration du produit inhibant 50 % de l'activité du PAI-1 est recherchée en mesurant l'absorbance du caillot deux heures après la fibrinoformation en présence de PAI-1 et de concentration croissante du produit. A titre d'exemple, les composés des exemples 2, 10 et 26 montrent respectivement un $IC_{50}$ de 0,13 µM, 0,53 µM et 0,06 µM. Ce résultat démontre l'activité fibrinolytique très supérieure des composés de l'invention, le produit de référence, le XR 5082 (*Thrombosis and Haemostasis* **1996**, 75(5), 808-815), présentant un $IC_{50}$ de 190 µM :

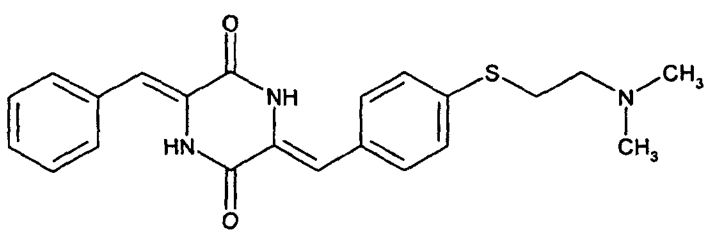

XR 5082

**EXEMPLE 42: Composition pharmaceutique**

Formule de préparation pour 1000 comprimés dosés à 10 mg :

| Formule de préparation pour 1000 comprimés dosés à 10 mg : | |
|---|---|
| Composé de l'exemple 2 | 10 g |
| Hydroxypropylcellulose | 2 g |
| Polyvinylpyrrolidone | 2 g |
| Amidon de blé | 10 g |

(suite)

| Formule de préparation pour 1000 comprimés dosés à 10 mg : | |
|---|---|
| Lactose | 100 g |
| Stéarate de magnésium | 3 g |

## Revendications

**1.** Composés de formule (I) :

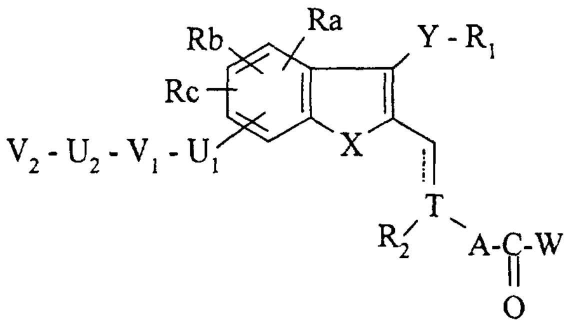

(I)

dans laquelle :

X représente un atome d'oxygène, de soufre, ou un groupement $NR_3$ dans lequel $R_3$ représente un atome d'hydrogène, un groupement alkyle ($C_1$-$C_6$) linéaire ou ramifié, acyle ($C_1$-$C_6$) linéaire ou ramifié, aryle, arylalkyle ($C_1$-$C_6$) linéaire ou ramifié, ou hétéroarylalkyle ($C_1$-$C_6$) linéaire ou ramifié,

Y représente un atome d'oxygène, de soufre, un groupement $NR_3$, le groupement $R_3$ ayant la même définition que précédemment, ou peut représenter une liaison simple quand X représente un groupement $NR'_3$ dans lequel $R'_3$ représente un groupement hétéroarylalkyle ($C_1$-$C_6$) linéaire ou ramifié,

T représente un atome d'azote quand la liaison qui le relie avec l'atome de carbone voisin est simple (—), un atome de carbone ou un groupement CH selon que la liaison qui le relie avec l'atome de carbone voisin est simple (——) ou double (═══),

A représente une liaison simple ou un groupement choisi parmi alkylène ($C_1$-$C_6$) (éventuellement substitué par un ou plusieurs groupements alkyle ($C_1$-$C_6$) linéaire ou ramifié, aryle, arylalkyle ($C_1$-$C_6$) linéaire ou ramifié, cycloalkyle, hétérocycloalkyle, ou hétéroaryle), arylène, cycloalkylène, hétérocycloalkylène, hétéroarylène, et un groupement $-SO_2-R_4-$ (la partie $SO_2$ étant reliée à T) dans lequel $R_4$ représente un groupement choisi parmi alkylène ($C_1$-$C_6$) linéaire ou ramifié, arylène, arylalkylène ($C_1$-$C_6$) linéaire ou ramifié, cycloalkylène, hétérocycloalkylène, et hétéroarylène,

W représente un groupement choisi parmi hydroxy, alkoxy ($C_1$-$C_6$) linéaire ou ramifié, aryloxy, arylalkoxy ($C_1$-$C_6$) linéaire ou ramifié, cycloalkyloxy, hétérocycloalkyloxy, hétéroaryloxy, amino (lui-même pouvant être substitué par un ou deux groupements, identiques ou différents, indépendamment l'un de l'autre, choisis parmi alkyle ($C_1$-$C_6$) linéaire ou ramifié, aryle, arylalkyle ($C_1$-$C_6$) linéaire ou ramifié, ou cycloalkyle), et hydroxyamino,

$U_1$ représente un atome d'oxygène, de soufre ou une chaîne alkylène ($C_1$-$C_6$) linéaire ou ramifié, dont un ou plusieurs des atomes de carbone peuvent éventuellement être remplacés par un ou plusieurs hétéroatomes choisis parmi oxygène, azote et soufre, ladite chaîne alkylène étant éventuellement substituée par un ou plusieurs groupements, identiques ou différents, choisis parmi atomes d'halogène, groupements hydroxy, alkyle ($C_1$-$C_6$) linéaire ou ramifié, et alkoxy ($C_1$-$C_6$) linéaire ou ramifié,

$V_1$ représente un groupement arylène, hétéroarylène ou hétérocycloalkylène,

$U_2$ représente une liaison simple, un atome d'oxygène, d'azote, de soufre, ou une chaîne alkylène ($C_1$-$C_6$) linéaire ou ramifiée dont un ou plusieurs atomes de carbone peuvent éventuellement être remplacé par un ou plusieurs groupements, identiques ou différents, choisis parmi atomes d'oxygène, de soufre, d'azote, (l'atome d'azote étant substitué par un groupement choisi parmi hydrogène et alkyle ($C_1$-$C_6$) linéaire ou ramifié), et un groupement $SO_2$,

$V_2$ représente un groupement aryle, hétéroaryle ou hétérocycloalkyle,

Ra, Rb, Rc, identiques ou différents, indépendamment l'un de l'autre, représentent chacun un groupement choisi parmi :

- atome d'hydrogène, d'halogène,
- groupement hydroxy, cyano, nitro,
- alkyle ($C_1$-$C_6$) linéaire ou ramifié, alkoxy ($C_1$-$C_6$) linéaire ou ramifié, acyle ($C_1$-$C_6$) linéaire ou ramifié, carboxy, alkoxycarbonyle ($C_1$-$C_6$) linéaire ou ramifié, trihalogénoalkyle ($C_1$-$C_6$) linéaire ou ramifié,
- amino (éventuellement substitué par un ou deux groupements, identiques ou différents, indépendamment l'un de l'autre, alkyle ($C_1$-$C_6$) linéaire ou ramifié, aryle, ou arylalkyle ($C_1$-$C_6$) linéaire ou ramifié),
- aryloxy, arylalkoxy ($C_1$-$C_6$) linéaire ou ramifié, hétéroaryloxy, hétéroarylalkoxy ($C_1$-$C_6$) linéaire ou ramifié,
- et un groupement de formule $-U_1-V_1-U_2-V_2$ dans laquelle $U_1$, $U_2$, $V_1$ et $V_2$ sont tels que définis précédemment,
- ou pris par deux forment ensemble un groupement méthylènedioxy, ou éthylènedioxy (chacun de ces groupements étant éventuellement substitués par un ou deux groupements alkyle ($C_1$-$C_6$) linéaire ou ramifié, aryle, ou arylalkyl ($C_1$-$C_6$) linéaire ou ramifié),

$R_1$ représente :

- un groupement aryle substitué par un à cinq substituants, identiques ou différents, indépendamment l'un de l'autre, choisis parmi halogène, hydroxy, cyano, nitro, carboxy, alkyle ($C_1$-$C_6$) linéaire ou ramifié, alkoxy ($C_1$-$C_6$) linéaire ou ramifié, acyle ($C_1$-$C_6$) linéaire ou ramifié, alkoxycarbonyle ($C_1$-$C_6$) linéaire ou ramifié, trihalogénoalkyle ($C_1$-$C_6$) linéaire ou ramifié (éventuellement substitué par un groupement hydroxy), trihalogénoalkoxy ($C_1$-$C_6$) linéaire ou ramifié, amino (éventuellement substitué par un ou deux groupements alkyle ($C_1$-$C_6$) linéaire ou ramifié, un desdits groupements alkyles étant éventuellement substitué par un groupement choisi parmi amino, alkylamino ($C_1$-$C_6$) linéaire ou ramifié, et dialkylamino ($C_1$-$C_6$) linéaire ou ramifié), aminoalkoxy ($C_1$-$C_6$) linéaire ou ramifié (la partie amino étant éventuellement substituée par un ou deux groupements, identiques ou différents, alkyle ($C_1$-$C_6$) linéaire ou ramifié), alkoxycarbonylalkyle ($C_1$-$C_6$) linéaire ou ramifié, alkyl($C_1$-$C_6$)carbonylamino linéaire ou ramifié, arylakyle ($C_1$-$C_6$) linéaire ou ramifié, aryloxy, arylalkoxy ($C_1$-$C_6$) linéaire ou ramifié, arylamino, arylalkylamino ($C_1$-$C_6$) linéaire ou ramifié, arylsulfanyle, arylalkyl($C_1$-$C_6$)sulfanyle linéaire ou ramifié, hétéroaryle, hétéroarylalkyle ($C_1$-$C_6$) linéaire ou ramifié, hétéroaryloxy, hétéroarylalkoxy ($C_1$-$C_6$) linéaire ou ramifié, hétéroarylamino, hétéroarylalkyl($C_1$-$C_6$)amino linéaire ou ramifié, hétéroarylsulfanyle, hétéroarylalkyl($C_1$-$C_6$)sulfanyle linéaire ou ramifié,
- un groupement 1,3-dihydro-2*H*-indol-2-one, 3,4-dihydro-2(1*H*)-quinolinone, 1-hydroxy-2(1*H*)-pyridinone,
- ou un groupement hétéroaryle éventuellement substitué,

$R_2$ représente un groupement choisi parmi atome d'hydrogène, groupement alkyle ($C_1$-$C_6$) linéaire ou ramifié, aryle, arylalkyle ($C_1$-$C_6$) linéaire ou ramifié, cycloalkyle, hétérocycloalkyle, hétérocycloalkylalkyle ($C_1$-$C_6$) linéaire ou ramifié, hétéroaryle, et hétéroarylalkyle ($C_1$-$C_6$) linéaire ou ramifié,

étant entendu que par :

- groupement aryle, on comprend un groupement phényle, biphényle, naphtyle, tétrahydronaphtyle, ou dihydronaphtyle, chacun de ces groupements étant éventuellement substitué de façon identique ou différente, par un ou plusieurs groupements choisis parmi atome d'halogène, groupement hydroxy, cyano, nitro, alkyle ($C_1$-$C_6$) linéaire ou ramifié (éventuellement substitué par un ou plusieurs groupements choisis parmi hydroxy, amino, mono ou dialkylamino ($C_1$-$C_6$) linéaire ou ramifié), trihalogénoalkyle ($C_1$-$C_6$) linéaire ou ramifié, alkoxy ($C_1$-$C_6$) linéaire ou ramifié, aryloxy, acyle ($C_1$-$C_6$) linéaire ou ramifié, carboxy, alkoxycarbonyle ($C_1$-$C_6$) linéaire ou ramifié, et amino (substitué éventuellement par un ou deux groupements, identiques ou différents, alkyle ($C_1$-$C_6$) linéaire ou ramifié),

- groupement cycloalkyle, on comprend un groupement mono ou bicyclique, comportant de 3 à 8 atomes de carbone,

- hétérocycloalkyle, on comprend un groupement mono ou bicyclique, saturé ou insaturé, à caractère non aromatique, de 5 à 12 chaînons contenant un, deux ou trois hétéroatomes, identiques ou différents, choisis parmi oxygène, azote et soufre, étant entendu que l'hétérocycloalkyle peut être éventuellement substitué, de façon identique ou différente, par un ou plusieurs groupements choisis parmi atome d'halogène, groupement hydroxy, alkyle ($C_1$-$C_6$) linéaire ou ramifié, trihalogénoalkyle ($C_1$-$C_6$) linéaire ou ramifié, alkoxy ($C_1$-$C_6$) linéaire ou ramifié, aryloxy, arylalkoxy ($C_1$-$C_6$) linéaire ou ramifié, amino (substitué éventuellement par un ou deux groupements alkyle ($C_1$-$C_6$) linéaire ou ramifié), acyle ($C_1$-$C_6$) linéaire ou ramifié, alkoxycarbonyle ($C_1$-$C_6$) linéaire ou ramifié, nitro, et oxo,

- hétéroaryle, on comprend un hétérocycloalkyle tel que défini précédemment, mono ou bicyclique, dont au moins un des cycles possède un caractère aromatique, le ou lesdits hétéroatomes pouvant se situer, dans le cas d'un système bicyclique, sur le cycle à caractère aromatique ou sur le cycle partiellement insaturé, étant entendu que l'hétéroaryle peut éventuellement être substitué par un ou plusieurs groupements identiques ou différents, tels que définis dans la définition des substituants de l'hétérocycloalkyle,

leurs isomères ainsi que leurs sels d'addition à un acide ou à une base pharmaceutiquement acceptable.

2. Composés de formule (I) selon la revendication 1 **caractérisés en ce que** X représente un atome de soufre ou un groupement $NR_3$ avec $R_3$ tel que défini dans la formule (I), leurs isomères ainsi que leurs sels d'addition à un acide ou à une base pharmaceutiquement acceptable.

3. Composés de formule (I) selon la revendication 1 **caractérisés en ce que** Y représente un atome d'oxygène, leurs isomères ainsi que leurs sels d'addition à un acide ou à une base pharmaceutiquement acceptable.

4. Composés de formule (I) selon la revendication 1 **caractérisés en ce que** $R_1$ représente un groupement choisi parmi phényle éventuellement substitué par un groupement tel que défini dans la formule (I), quinolyle éventuellement substitué, et pyridinyle éventuellement substitué, leurs isomères ainsi que leurs sels d'addition à un acide ou à une base pharmaceutiquement acceptable.

5. Composés de formule (I) selon la revendication 1 **caractérisés en ce que** $R_2$ représente un groupement choisi parmi aryle et hétéroaryle, chacun de ces groupements étant éventuellement substitué, leurs isomères ainsi que leurs sels d'addition à un acide ou à une base pharmaceutiquement acceptable.

6. Composés de formule (I) selon l'une quelconque des revendications 1 et 5 **caractérisés en ce que** $R_2$ représente un groupement pyridinyle, leurs isomères ainsi que leurs sels d'addition à un acide ou à une base pharmaceutiquement acceptable.

7. Composés de formule (I) selon la revendication 1 **caractérisés en ce que** $U_1$ représente une chaîne alkylène ($C_1$-$C_4$) linéaire dont l'un des atomes de carbone est remplacé par un atome d'oxygène, $V_1$ représente un groupement arylène, $U_2$ représente une liaison simple et $V_2$ représente un groupement aryle éventuellement substitué par un des groupements tels que définis dans la formule (I), leurs isomères ainsi que leurs sels d'addition à un acide ou à une base pharmaceutiquement acceptable.

8. Composés de formule (I) selon l'une quelconque des revendications 1 et 7 **caractérisés en ce que** $-U_1-V_1-U_2-V_2$ représente un groupement [1,1'-biphényl]-4-ylméthoxy, leurs isomères ainsi que leurs sels d'addition à un acide ou à une base pharmaceutiquement acceptable.

**9.** Composés de formule (I) selon la revendication 1 **caractérisés en ce que** au moins un des groupements Ra, Rb ou Rc représente un groupement de formule $-U_1-V_1-U_2-V_2$ telle que définie dans la formule (I), leurs isomères ainsi que leurs sels d'addition à un acide ou à une base pharmaceutiquement acceptable.

**10.** Composés de formule (I) selon l'une quelconque des revendications 1 à 3 **caractérisés en ce que** X représente un atome de soufre et Y représente un atome d'oxygène, leurs isomères ainsi que leurs sels d'addition à un acide ou à une base pharmaceutiquement acceptable.

**11.** Composés de formule (I) selon la revendication 1 **caractérisés en ce que** X représente un atome de soufre, Y représente un atome d'oxygène, $R_1$ représente un groupement phényle éventuellement substitué ou un groupement pyridinyle éventuellement substitué, et A représente une liaison simple quand T représente un atome de carbone ou un groupement CH, leurs isomères ainsi que leurs sels d'addition à un acide ou à une base pharmaceutiquement acceptable.

**12.** Composés de formule (I) selon la revendication 1 **caractérisés en ce que** X représente un atome de soufre, Y représente un atome d'oxygène, $R_1$ représente un groupement phényle éventuellement substitué par un groupement tel que défini dans la formule (I), et A représente un groupement alkylène (éventuellement substitué par un groupement alkyle ($C_1$-$C_6$) linéaire ou ramifié, aryle, ou arylalkyle ($C_1$-$C_6$) linéaire ou ramifié) ou un groupement arylène quand T représente un atome d'azote, leurs isomères ainsi que leurs sels d'addition à un acide ou à une base pharmaceutiquement acceptable.

**13.** Composés de formule (I) selon la revendication 1 **caractérisés en ce qu'**ils représentent des composés de formule (Ibis) :

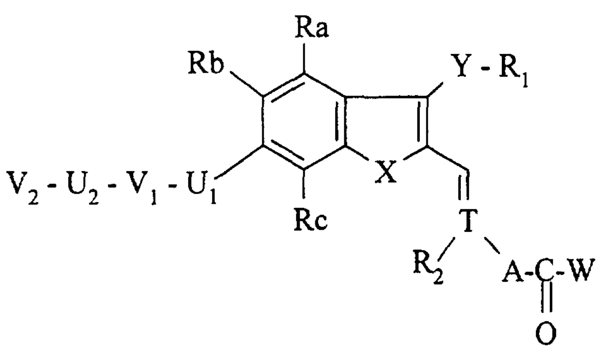

**(I bis)**

dans laquelle :

| | |
|---|---|
| X | représente un atome de soufre, |
| Y | représente un atome d'oxygène, |
| $R_1$ | représente un groupement phényle éventuellement substitué ou un groupement hétéroaryle choisi parmi pyridinyle éventuellement substitué et quinolinyle éventuellement substitué, |
| A | représente une liaison simple, |
| T | représente un atome de carbone, |
| Ra et Rc | représentent chacun un atome d'hydrogène, |
| $U_1$ | représente une chaîne alkylènoxy ($C_1$-$C_4$) linéaire, |
| $V_1$ | représente un groupement arylène, |
| $U_2$ | représente une liaison simple, |
| $V_2$ | représente un groupement aryle, |
| Rb | représente un groupement $U_1$ $-V_1-U_2-V_2$ tel que défini précédemment, |
| $R_2$ | représente un groupement hétéroaryle, |
| W | représente un groupement tel que défini dans la formule (I), leurs isomères ainsi que leurs sels d'addition à un acide ou à une base pharmaceutiquement acceptable. |

**14.** Composés de formule (I) selon la revendication 13 **caractérisés en ce qu'**ils représentent des composés de formule (Ibis) dans laquelle Rb et $-U_1-V_1-U_2-V_2$ représentent chacun un groupement [1,1'-biphényl]-4-ylméthoxy, leurs isomères ainsi que leurs sels d'addition à un acide ou à une base pharmaceutiquement acceptable.

**15.** Composés de formule (I) selon la revendication 13 **caractérisés en ce qu'**ils représentent des composés de formule (Ibis) dans laquelle $R_2$ représente un groupement pyridinyle, leurs isomères ainsi que leurs sels d'addition à un acide ou à une base pharmaceutiquement acceptable.

**16.** Composés de formule (I) selon la revendication 13 **caractérisés en ce qu'**ils représentent des composés de formule (Ibis) dans laquelle $R_1$ représente :

- un groupement phényle éventuellement substitué par un à trois groupements choisis parmi atome d'halogène, groupement alkyle ($C_1$-$C_6$) linéaire ou ramifié, hétéroaryle, hétéroarylalkoxy ($C_1$-$C_6$) linéaire ou ramifié, alkoxy ($C_1$-$C_6$) linéaire ou ramifié, amino, et aminoalkoxy ($C_1$-$C_6$) linéaire ou ramifié, la partie amino pouvant être substituée par un ou deux groupements, identiques ou différents, alkyles ($C_1$-$C_6$) linéaire ou ramifié,
- ou un groupement hétéroaryle choisi parmi pyridinyle et quinolinyle éventuellement substituée par un atome d'halogène ou un groupement alkyle ($C_1$-$C_6$) linéaire ou ramifié,

leurs isomères ainsi que leurs sels d'addition à un acide ou à une base pharmaceutiquement acceptable.

**17.** Composé de formule (I) selon la revendication 1 qui est l'acide (E)-3-[5,6-bis([1,1'-biphényl]-4-ylméthoxy)-3-(4-chlorophénoxy)benzo[*b*]thiophène-2-yl]-2-(4-pyridinyl)-2-propènoïque, ses isomères ainsi que ses sels d'addition à un acide ou à une base pharmaceutiquement acceptable.

**18.** Composés de formule (I) selon la revendication 1 qui sont :

- le (*E*)-3-[5,6-bis-([1,1'-biphényl]-4-ylméthoxy)-3-(4-chlorophénoxy)-benzo[*b*]thiophène-2-yl]-2-(4-pyridinyl)-2-propènoate d'éthyle,
- l'acide (*E*)-3-[5,6-bis-([1,1'-biphényl]-4-ylméthoxy)-3-(3-pyridinyloxy)-benzo[*b*]thiophène-2-yl]-2-(4-pyridinyl)-2-propènoïque,
- l'acide 3-(*E*)-{5,6-bis-([1,1'-biphényl]-4-ylméthoxy)-3-[6-(méthyl)pyridinyl-3-oxy]-benzo[*b*] thiophène-2-yl},2,(4,pyridinyl)-2-propènoïque,
- l'acide 3-(*E*)-[5,6-bis-([1,1'-biphényl]-4-ylméthoxy)-3-(6-quinolinyloxy)benzo[*b*]thiophène-2-yl]-2-(4-pyridinyl)-2-propènoïque,
- l'acide (*E*)-3-[5,6-bis-([1,1'-biphényl]-2,ylméthoxy)-3,(4,chlorophénoxy)benzo[*b*]thiophène, 2-yl]-2-(4-pyridinyl)-2-propènoïque,
- l'acide (*E*)-3-[5,6-bis([1,1'-biphényl]-3-ylméthoxy)-3-(4-chlorophénoxy)-benzo[*b*]thiophène-2-yl]-2-(4-pyridinyl)-2-propénoïque,
- l'acide (*E*)-3-[5,6-bis([1,1'-biphényl]-4-ylméthoxy)-3-(4-chloro-3-fluorophénoxy)-benzo[*b*] thiophène-2-yl]-2-(4-pyridinyl)-2-propénoïque,
- l'acide (*E*)-3-[5,6,bis([1,1',biphényl]-4-ylméthoxy)-3-(4-chloro-3,5-diméthylphénoxy)-benzo [*b*]thiophène-2-yl]-2-(4-pyridinyl)-2-propénoïque,
- l'acide (*E*)-3-[5,6-bis([1,1'-biphényl]-4-ylméthoxy)-3-(4-chloro-3-méthylphénoxy)-benzo[*b*] thiophène-2-yl]-2-(4-pyridinyl)-2-propénoïque,
- l'acide (*E*)-3-{5,6-bis([1,1'-biphényl]-4-ylméthoxy)-3-[4-(4-pyridinyloxy)phénoxy]-benzo[*b*] thiophène-2-yl}-2-(4-pyridinyl)-2-propénoïque,
- l'acide (*E*)-3-{5,6-bis([1,1',biphényl]-4-ylméthoxy)-3-[4-(1*H*-imidazol-1-yl)phénoxy]-benzo [*b*]thiophène-2-yl}-2-(4-pyridinyl)-2-propénoïque,
- l'acide (*E*)-3-[5,6-bis([1,1'-biphényl]-4-ylméthoxy)-3-phénoxy-benzo[*b*]thiophène-2-yl]-2-(4-pyridinyl)-2-propénoïque,
- l'acide (*E*)-3-[5,6-bis([1,1'-biphényl]-4-ylméthoxy)-3-(3-fluorophénoxy)-benzo[*b*]thiophène-2-yl]-2-(4-pyridinyl)-2-propénoïque,
- l'acide (*E*)-3-[5,6-bis([1,1'-biphényl]-4-ylméthoxy)-3-(3,4-difluorophénoxy)-benzo[*b*] thiophène-2-yl]-2-(4-pyridinyl)-2-propénoïque,
- l'acide (*E*)-3-{5,6-bis([1,1'-biphényl]-4-ylméthoxy)-3-[(6-chloro-3-pyridinyl)oxy]-benzo[*b*] thiophène-2-yl}-2-(4-pyridinyl)-2-propénoïque,

leurs isomères ainsi que leurs sels d'addition à un acide ou à une base pharmaceutiquement acceptable.

**19.** Procédé de préparation des composés de formule (I) selon la revendication 1, **caractérisé en ce qu'**on utilise comme produit de départ un composé de formule (II):

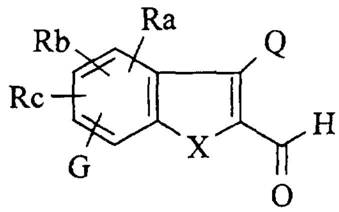

**(II)**

dans laquelle Ra, Rb, Rc et X ont la même signification que dans la formule (I), G représente un groupement hydroxy protégé par un groupement protecteur classiquement utilisé en synthèse organique et Q représente un atome d'halogène ou un groupement hydroxy et de façon préférée, Q représente un atome d'halogène quand X représente un atome de soufre ou un groupement $NR_3$ avec $R_3$ tel que défini dans la formule (I) et Q représente un groupement hydroxy quand X représente un atome d'oxygène,
composé de formule (II) que l'on fait réagir, en condition basique,

- *soit quand Q représente un atome d'halogène :*

  ✧ avec un composé de formule (III),

$$H\text{-}Y_1\text{-}R_1 \qquad \textbf{(III)}$$

dans laquelle $R_1$ a la même signification que dans la formule (I) et $Y_1$ représente un atome d'oxygène, de soufre, ou un groupement $NR_3$ avec $R_3$ ayant la même signification que dans la formule (I),
pour conduire aux composés de formule (IV/a) :

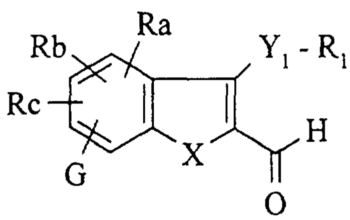

**(IV/a)**

dans laquelle Ra, Rb, Rc, G, $R_1$, X et $Y_1$ sont tels que définis précédemment,

  ✧ ou avec un composé de formule (V) :

$$(HO)_2B - R_1 \qquad \textbf{(V)}$$

dans laquelle $R_1$ a la même signification que dans la formule (I),
pour conduire aux composés de formule (IV/b) :

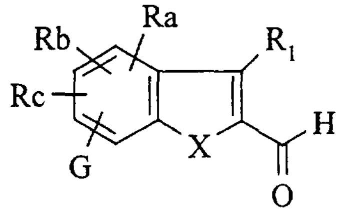

**(IV/b)**

dans laquelle Ra, Rb, Rc, G et $R_1$ sont tels que définis précédemment et $X_1$ représente un groupement $NR_3$ dans lequel $R_3$ représente un groupement hétéroarylalkyle ($C_1$-$C_6$) linéaire ou ramifié,

- *soit quand Q représente un groupement hydroxy*,avec un composé de formule (VI),

Hal - $R_1$ **(VI)**

dans laquelle Hal représente un atome d'halogène et $R_1$ est tel que défini précédemment, pour conduire aux composés de formule (IV/c) :

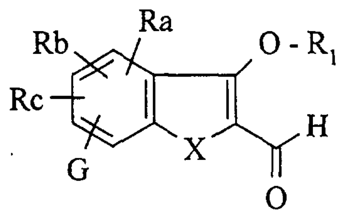

**(IV/c)**

dans laquelle Ra, Rb, Rc, G, X et $R_1$ sont tels que définis précédemment,
l'ensemble des composés de formule (IV/a), (IV/b) et (IV/c) forment les composés de formule (IV) :

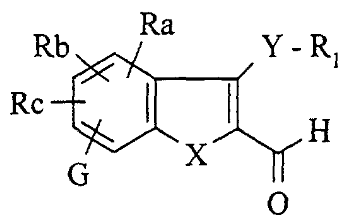

**(IV)**

dans laquelle Ra, Rb, Rc, G, $R_1$, X et Y ont la même définition que dans la formule (I),
composés de formule (IV) :

- ♦ que l'on condense, en présence d'anhydride acétique, avec un composé de formule (VII),

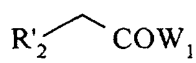

**(VII)**

dans laquelle $R'_2$ a la même signification que $R_2$ dans la formule (I), excepté que $R'_2$ ne peut pas représenter un atome hydrogène, et $W_1$ représente un groupement choisi parmi alkoxy ($C_1$-$C_6$) linéaire ou ramifié, aryloxy, arylalkoxy ($C_1$-$C_6$) linéaire ou ramifié, cycloalkyloxy, hétérocycloalkoxy, hétéroaryloxy et un groupement amino (lui-même étant éventuellement substitué par un ou deux groupements, identiques ou différents, indépendamment l'un de l'autre, choisis parmi alkyle ($C_1$-$C_6$) linéaire ou ramifié, aryle, arylalkyle ($C_1$-$C_6$) linéaire ou ramifié, et cycloalkyle),
pour conduire aux composés de formule (VIII) :

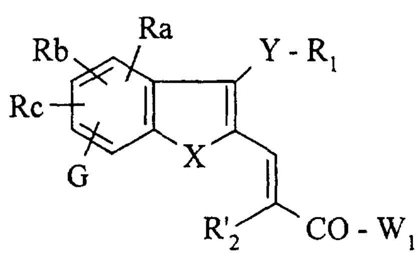

**(VIII)**

dans laquelle Ra, Rb, Rc, G, $R_1$, $R'_2$, X, Y et $W_1$ ont la même définition que précédemment,
composés de formule (VIII) dont on déprotège la fonction hydroxy selon des conditions classique de la synthèse organique, puis que l'on fait réagir en milieu basique avec un composé de formule (IX) :

$$V_2 - U_2 - V_1 - U_1 - Hal \qquad \textbf{(IX)}$$

dans laquelle $U_1$, $V_1$, $U_2$ et $V_2$ sont tels que définis dans la formule (I) et Hal représente un atome d'halogène,
pour conduire aux composés de formule (I/a), cas particulier des composés de formule (I):

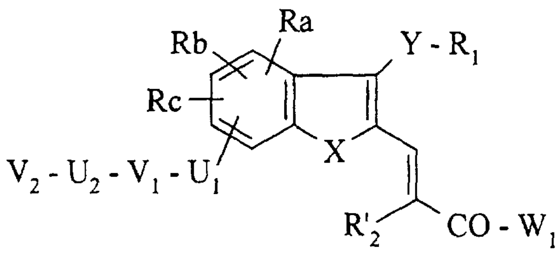

**(I/a)**

dans laquelle Ra, Rb, Rc, $U_1$, $V_1$, $U_2$, $V_2$, $R_1$, $R'_2$, X, Y et $W_1$ ont la même définition que précédemment, composés de formule (I/a) que l'on soumet, si on le désire :

✱ soit à des conditions d'hydrogénation catalytique, en présence de Palladium, pour conduire aux composés de formule (I/b), cas particulier des composés de formule (I) :

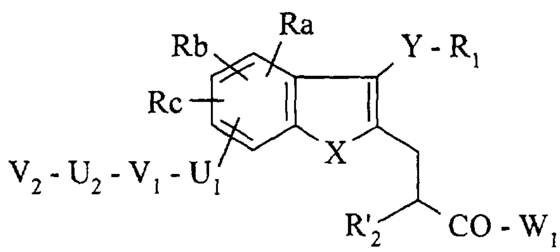

**(I/b)**

dans laquelle Ra, Rb, Rc, $U_1$, $V_1$, $U_2$, $V_2$, $R_1$, $R'_2$, X, Y et $W_1$ sont tels que définis précédemment,

✱ soit à des conditions d'hydrolyse, en milieu basique, pour conduire aux composés de formule (I/c), cas particulier des composés de formule (I):

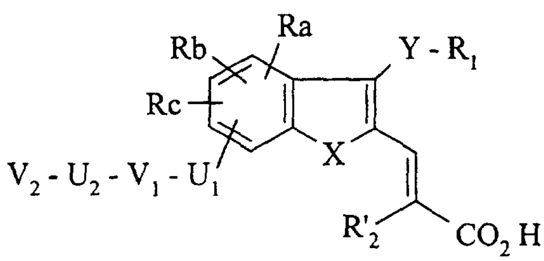

**(I/c)**

dans laquelle Ra, Rb, Rc, $U_1$, $V_1$, $U_2$, $V_2$, $R_1$, $R'_2$, X et Y ont la même définition que précédemment, composés de formule (I/c), dont on réduit, si on le souhaite, la double liaison par hydrogénation catalytique, pour conduire aux composés de formule (I/d), cas particulier des composés de formule (I):

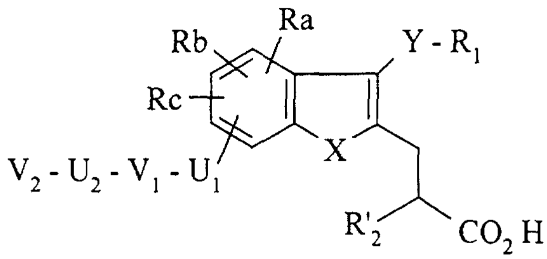

**(I/d)**

dans laquelle Ra, Rb, Rc, $U_1$, $V_1$, $U_2$, $V_2$, $R_1$, $R'_2$, X et Y sont tels que définis précédemment,

- ou que l'on soumet, à l'action d'un ylure de phosphore de formule (X),

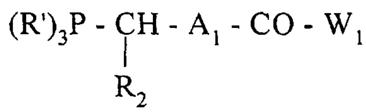

**(X)**

dans laquelle R' représente un groupement alkyle ($C_1$-$C_6$) linéaire ou ramifié, ou un groupement phényle, $R_2$ a la même définition que dans la formule (I), $W_1$ a la même définition que précédemment et $A_1$ représente une liaison simple, un groupement alkylène (éventuellement substitué par un ou plusieurs groupements choisis parmi alkyle ($C_1$-$C_6$) linéaire ou ramifié, aryle, arylalkyle ($C_1$-$C_6$) linéaire ou ramifié, cycloalkyle, hétérocycloalkyle, et hétéroaryle), un groupement arylène, cycloalkylène, hétérocycloalkylène, ou hétéroarylène,
pour conduire aux composés de formule (XI) :

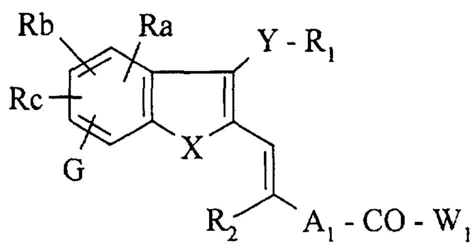

**(XI)**

dans laquelle Ra, Rb, Rc, G, $R_1$, $R_2$, X, Y, $A_1$ et $W_1$ ont la même définition que précédemment,
composés de formule (XI) dont on déprotège la fonction hydroxy selon des conditions classiques de la synthèse organique, puis que l'on fait réagir en milieu basique avec un composé de formule (IX) :

$$V_2 - U_2 - V_1 - U_1 - Hal \qquad \textbf{(IX)}$$

dans laquelle $U_1$, $V_1$, $U_2$ et $V_2$ sont tels que définis dans la formule (I) et Hal représente un atome d'halogène,
pour conduire aux composés de formule (I/e), cas particulier des composés de formule (I):

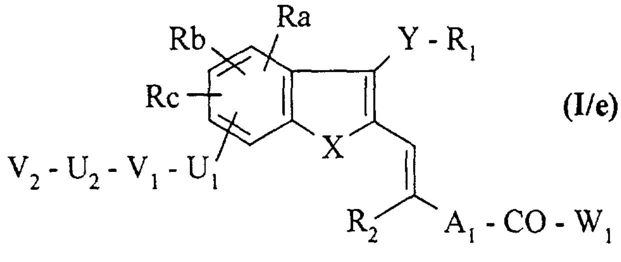

**(I/e)**

dans laquelle Ra, Rb, Rc, $U_1$, $V_1$, $U_2$, $V_2$, $R_1$, $R_2$, $A_1$, X, Y et $W_1$ ont la même définition que précédemment, composés de formule (I/e) que l'on soumet, si on le désire :

✱ soit à des conditions d'hydrolyse, en condition basique, pour conduire aux composés de formule (I/f), cas particulier des composés de formule (I):

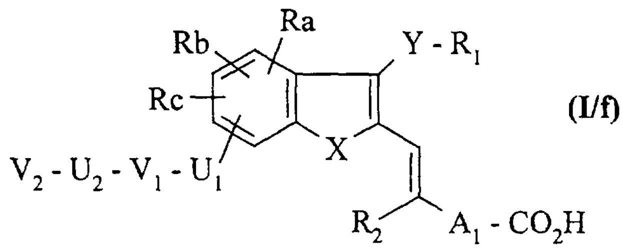

**(I/f)**

dans laquelle Ra, Rb, Rc, $U_1$, $V_1$, $U_2$, $V_2$, $R_1$, $R_2$, X, Y et $A_1$ sont tels que définis précédemment,

✱ soit à des conditions d'hydrogénation catalytique, pour conduire aux composés de formule (I/g), cas particulier des composés de formule (I):

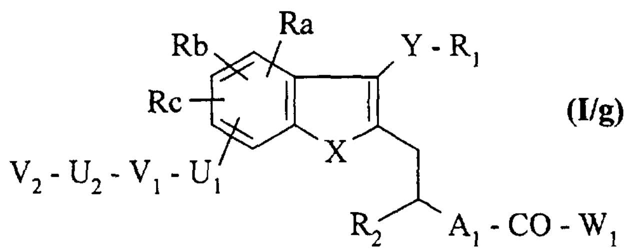

**(I/g)**

dans laquelle Ra, Rb, Rc, $U_1$, $V_1$, $U_2$, $V_2$, $R_1$, $R_2$, X, Y, $A_1$ et $W_1$ sont tels que définis précédemment, composés de formule (I/g) que l'on peut traiter dans des conditions d'hydrolyse basique, pour conduire aux composés de formule (I/h), cas particulier des composés de formule (I) :

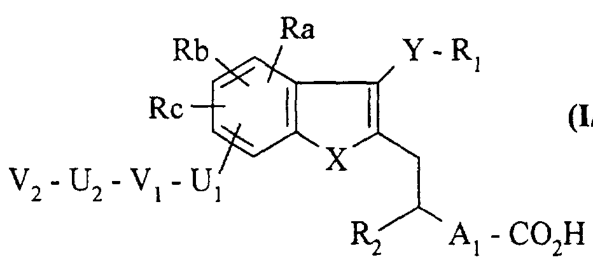

**(I/h)**

dans laquelle Ra, Rb, Rc, $U_1$, $V_1$, $U_2$, $V_2$, $R_1$, $R_2$, X, Y et $A_1$ sont tels que définis précédemment,

♦ ou dont on déprotège la fonction hydroxy selon des conditions classiques de la synthèse organique, puis que l'on fait réagir en milieu basique avec un composé de formule (IX) :

$$V_2 - U_2 - V_1 - U_1 - Hal \qquad \textbf{(IX)}$$

dans laquelle $U_1$, $V_1$, $U_2$ et $V_2$ sont tels que définis dans la formule (I) et Hal représente un atome d'halogène,
pour conduire aux composés de formule (XII) :

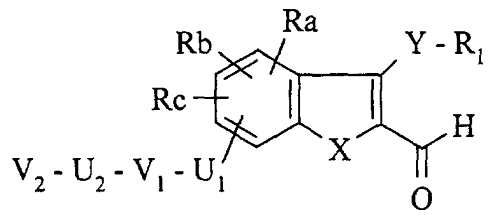

(XII)

dans laquelle Ra, Rb, Rc, $U_1$, $V_1$, $U_2$, $V_2$, $R_1$, X et Y ont la même définition que précédemment, composés de formule (XII), dont on réduit la fonction aldéhyde en alcool primaire, pour conduire aux composés de formule (XIII) :

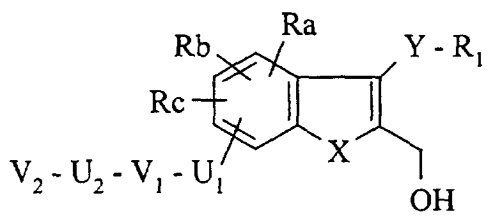

(XIII)

dans laquelle Ra, Rb, Rc, $U_1$, $V_1$, $U_2$, $V_2$, $R_1$, X et Y ont la même définition que dans la formule (I), composés de formule (XIII) dont on substitue l'hydroxy terminal par un halogène, selon des conditions classiques, pour conduire aux composés de formule (XIV) :

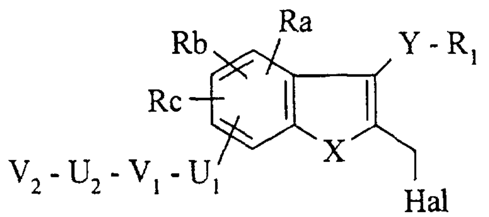

(XIV)

dans laquelle Ra, Rb, Rc, $U_1$, $V_1$, $U_2$, $V_2$, $R_1$, X et Y sont tels que définis précédemment, et Hal représente un atome de chlore ou de brome,
composés de formule (XIV):

✱ dont on substitue l'atome d'halogène, en condition basique, par un dérivé aminé de formule (XV) :

$$R_2 - NH - A_1 - CO - W_1 \qquad \textbf{(XV)}$$

dans laquelle $R_2$ a la même définition que dans la formule (I) et, $A_1$, $W_1$ ont la même signification que précédemment,
pour conduire aux composés de formule (I/i), cas particulier des composés de formule (I):

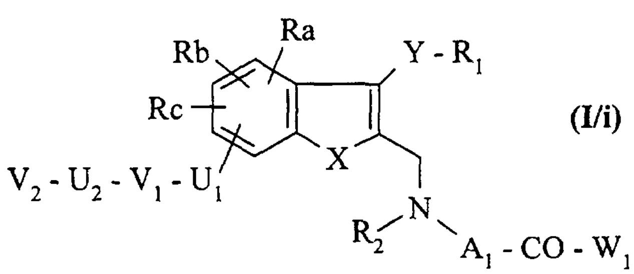

(I/i)

dans laquelle Ra, Rb, Rc, $U_1$, $V_1$, $U_2$, $V_2$, $R_1$, $R_2$, X, Y, $A_1$ et $W_1$ sont tels que définis précédemment,

composés de formule (I/i), dont on hydrolyse en condition basique le groupement carbonyle terminal, pour conduire aux composés de formule (I/j), cas particulier des composés de formule (I) :

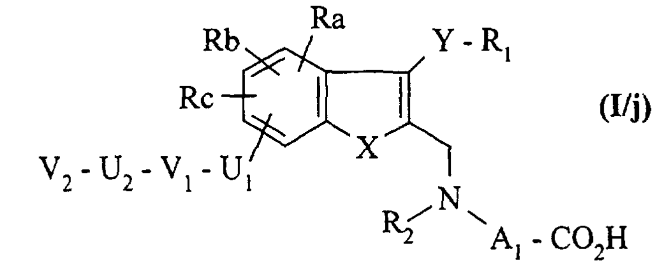

**(I/j)**

dans laquelle Ra, Rb, Rc, $U_1$, $V_1$, $U_2$, $V_2$, $R_1$, $R_2$, X, Y et $A_1$ sont tels que définis précédemment,

✱ ou que l'on traite par de l'azidure de sodium dans un premier temps, et dont on réduit l'azide obtenue en amine primaire dans des conditions d'hydrogénation catalytique, pour conduire aux composés de formule (XVI) :

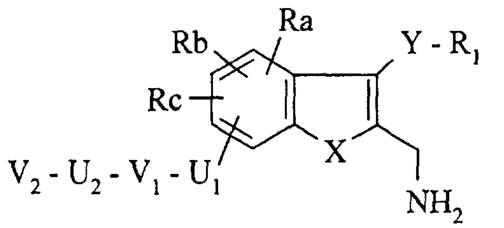

**(XVI)**

dans laquelle Ra, Rb, Rc, $U_1$, $V_1$, $U_2$, $V_2$, $R_1$, X et Y ont la même définition que dans la formule (I), composés de formule (XVI) que l'on condense, en condition basique, sur un dérivé chlorosulphonyle de formule (XVII) :

$$Cl - SO_2 - R_4 - CO - W_1 \qquad \textbf{(XVII)}$$

dans laquelle $R_4$ a la même définition que dans la formule (I), et $W_1$ est tel que défini précédemment, pour conduire aux composés de formule (I/k), cas particulier des composés de formule (I),

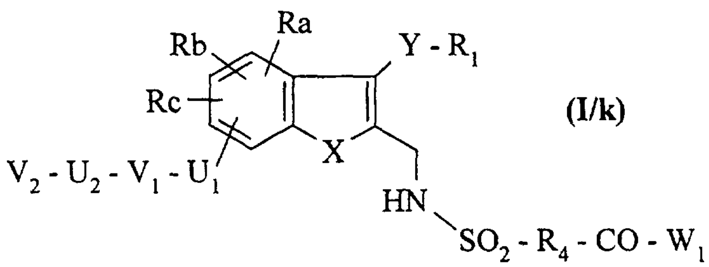

**(I/k)**

dans laquelle Ra, Rb, Rc, $U_1$, $V_1$, $U_2$, $V_2$, $R_1$, $R_4$, X, Y et $W_1$ sont tels que définis précédemment, composés de formule (I/k) :

- que l'on soumet, si on le souhaite, à des conditions d'hydrolyse en condition basique, pour conduire aux composés de formule (I/l), cas particulier des composés de formule (I) :

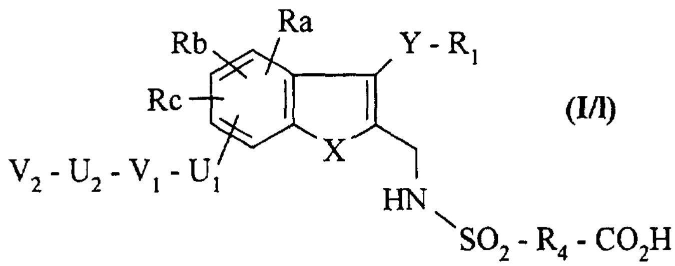

**(I/l)**

dans laquelle Ra, Rb, Rc, $U_1$, $V_1$, $U_2$, $V_2$, $R_1$, $R_4$, X et Y sont tels que définis précédemment,

- ou que l'on condense, en milieu basique, avec un composé de formule (XVIII) :

Hal - $R'_2$ **(XVIII)**

dans laquelle Hal représente un atome d'halogène tel que l'iode, et $R'_2$ a la même définition que précédemment,
pour conduire aux composés de formule (I/m), cas particulier des composés de formule (I):

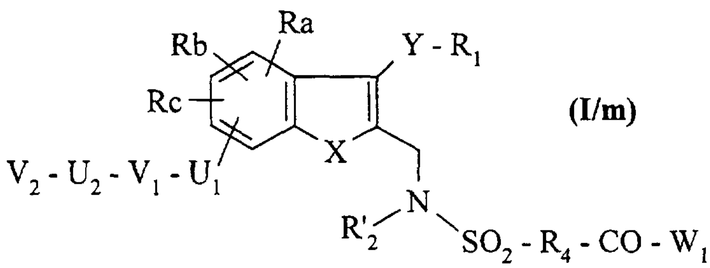

**(I/m)**

dans laquelle Ra, Rb, Rc, $U_1$, $V_1$, $U_2$, $V_2$, $R_1$, $R'_2$, $R_4$, X, Y et $W_1$ sont tels que définis précédemment,
composés de formule (I/m) que l'on traite par des conditions d'hydrolyse en milieu basique, pour conduire aux composés de formule (I/n), cas particulier des composés de formule (I) :

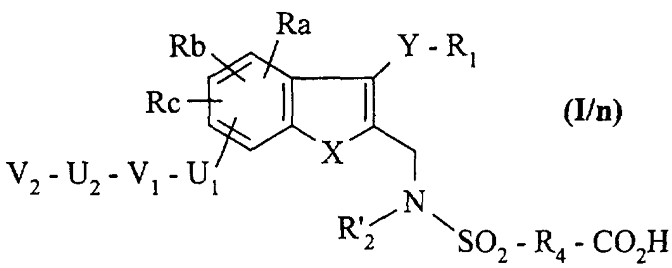

**(I/n)**

dans laquelle Ra, Rb, Rc, $U_1$, $V_1$, $U_2$, $V_2$, $R_1$, $R'_2$, $R_4$, X et Y sont tels que définis précédemment,

l'ensemble des composés de formule (I/c), (I/d), (I/f), (I/h), (I/j), (I/l) et (I/n) formant les composés de formule (I') :

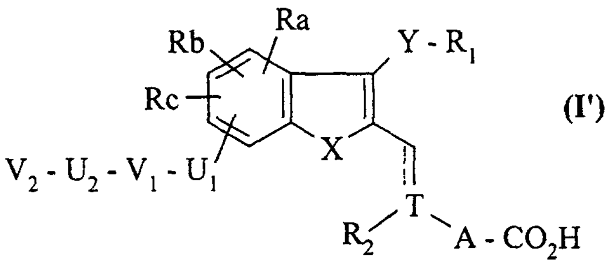

(I')

dans laquelle Ra, Rb, Rc, $U_1$, $V_1$, $U_2$, $V_2$, $R_1$, $R_2$, X, Y, Z et A sont tels que définis dans la formule (I), composés de formule (I') que l'on met en réaction avec une hydroxylamine-O-substituée, pour conduire, après déprotection de la fonction hydroxylamine, aux composés de formule (I/o), cas particulier des composés de formule (I):

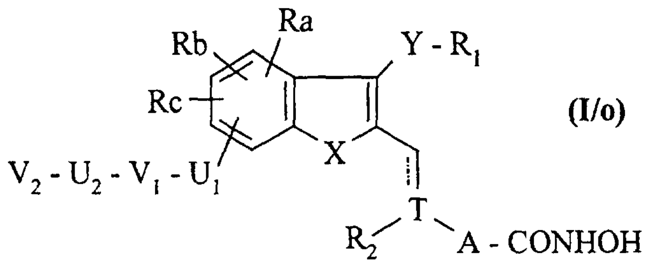

(I/o)

dans laquelle Ra, Rb, Rc, $U_1$, $V_1$, $U_2$, $V_2$, $R_1$, $R_2$, X, Y, Z et A sont tels que définis précédemment, les composés (I/a) à (I/o) formant l'ensemble des composés de l'invention, que l'on purifie, le cas échéant, selon une technique classique de purification, qui peuvent, si on le désire, être séparés en leurs différents isomères selon une technique classique de séparation, et que l'on transforme, le cas échéant, en leurs sels d'addition à un acide ou à une base pharmaceutiquement acceptable.

**20.** Compositions pharmaceutiques contenant comme principe actif au moins un composé de formule (I) selon l'une quelconque des revendications 1 à 18, seul ou en combinaison avec un ou plusieurs excipients ou véhicules inertes, non toxiques, pharmaceutiquement acceptables.

**21.** Compositions pharmaceutiques selon la revendication 20 contenant au moins un principe actif selon l'une quelconque des revendications 1 à 18, utiles en tant qu'inhibiteur du PAI-1.

**22.** Compositions pharmaceutiques selon la revendication 20 contenant au moins un principe actif selon l'une quelconque des revendications 1 à 18, utiles dans le traitement de la thrombose, des pathologies dont l'origine est la thrombose et des pathologies entraînant une augmentation des risques thrombotiques.

## Patentansprüche

**1.** Verbindungen der Formel (I):

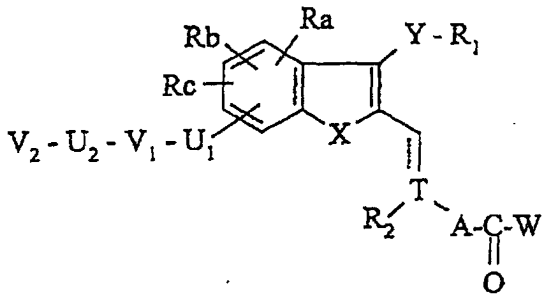

(I)

in der:

X ein Sauerstoffatom, Schwefelatom oder eine Gruppe $NR_3$ bedeutet, in der $R_3$ ein Wasserstoffatom, eine geradkettige oder verzweigte ($C_1$-$C_6$)-Alkylgruppe, geradkettige oder verzweigte ($C_1$-$C_6$-)-Acylgruppe, Arylgruppe, geradkettige oder verzweigte Aryl-($C_1$-$C_6$)-alkylgruppe oder geradkettige oder verzweigte Heteroaryl-($C_1$-$C_6$)-alkylgruppe darstellt,

Y ein Sauerstoffatom, Schwefelatom, eine Gruppe $NR_3$ bedeutet, worin $R_3$ die oben angegebenen Bedeutungen besitzt oder eine Einfachbindung darstellen kann, wenn X eine Gruppe $NR'_3$ bedeutet, in der $R'_3$ eine geradkettige oder verzweigte Heteroaryl-($C_1$-$C_6$)-alkylgruppe darstellt,

T ein Stickstoffatom bedeutet, wenn die es mit dem benachbarten Kohlenstoffatom verbindende Bindung einfach (—) ist, ein Kohlenstoffatom oder eine Gruppe CH bedeutet, wenn die es mit dem benachbarten Kohlenstoffatom verbindende Bindung einfach (——) oder doppelt (═══) ist,

A eine Einfachbindung bedeutet oder eine Gruppe ausgewählt aus ($C_1$-$C_6$)-Alkylen (gegebenenfalls substituiert durch eine oder mehrere geradkettige oder verzweigte ($C_1$-$C_6$)-Alkylgruppen, Arylgruppen, geradkettige oder verzweigte Aryl-($C_1$-$C_6$)-alkylgruppen, Cycloalkylgruppen, Heterocycloalkylgruppen oder Heteroarylgruppen), Arylen, Cycloalkylen, Heterocycloalkylen, Heteroarylen und einer Gruppe -$SO_2$-$R_4$- (wobei der $SO_2$-Rest an T gebunden ist), in der $R_4$ eine Gruppe ausgewählt aus geradkettigem oder verzweigtem ($C_1$-$C_6$)-Alkylen, Arylen, geradkettigem oder verzweigtem Aryl-($C_1$-$C_6$)-alkylen, Cycloalkylen, Heterocycloalkylen und Heteroarylen darstellt,

W eine Gruppe bedeutet ausgewählt aus Hydroxy, geradkettigem oder verzweigtem ($C_1$-$C_6$)-Alkoxy, Aryloxy, geradkettigem oder verzweigtem Aryl-($C_1$-$C_6$)-alkoxy, Cycloalkyloxy, Heterocycloalkyloxy, Heteroaryloxy, Amino (welches seinerseits durch ein oder zwei gleichartige oder verschiedenartige Gruppen substituiert sein kann, die unabhängig voneinander ausgewählt sind aus geradkettigem oder verzweigtem ($C_1$-$C_6$)-Alkyl, Aryl, geradkettigem oder verzweigtem Aryl-($C_1$-$C_6$)-alkyl und Cycloalkyl) und Hydroxyamino,

$U_1$ ein Sauerstoffatom, Schwefelatom oder eine geradkettige oder verzweigte ($C_1$-$C_6$)-Alkylenkette bedeutet, bei der ein oder mehrere Kohlenstoffatome gegebenenfalls durch ein oder mehrere Heteroatome ausgewählt aus Sauerstoff, Stickstoff und Schwefel ersetzt sein können, welche Alkylenkette gegebenenfalls durch ein oder mehrere gleichartige oder verschiedenartige Gruppen ausgewählt aus Halogenatomen, Hydroxygruppen, geradkettigen oder verzweigten ($C_1$-$C_6$)-Alkylgruppen und geradkettigen oder verzweigten ($C_1$-$C_6$)-Alkoxygruppen substituiert ist,

$V_1$ eine Arylen-, Heteroarylen- oder Heterocycloalkylen-Gruppe bedeutet,

$U_2$ eine Einfachbindung, ein Sauerstoffatom, Stickstoffatom, Schwefelatom oder eine geradkettige oder verzweigte ($C_1$-$C_6$)-Alkylenkette bedeutet, bei der ein oder mehrere Kohlenstoffatome gegebenenfalls durch ein oder mehrere gleichartige oder verschiedenartige Gruppen ausgewählt aus Sauerstoff-, Schwefel- und Stickstoffatomen (wobei das Stickstoffatom durch eine Gruppe

ausgewählt aus Wasserstoff und geradkettigem oder verzweigtem ($C_1$-$C_6$)-Alkyl substituiert ist) und eine Gruppe $SO_2$ ersetzt sind,

$V_2$ eine Aryl-, Heteroaryl- oder Heterocycloalkyl-Gruppe bedeutet,

Ra, Rb und Rc, die gleichartig oder verschieden sind, unabhängig voneinander jeweils eine Gruppe bedeuten ausgewählt aus:

- Wasserstoff- und Halogen-Atomen,
- Hydroxy-, Cyano- und Nitro-Gruppen,
- geradkettigem oder verzweigtem ($C_1$-$C_6$)-Alkyl, geradkettigem oder verzweigtem ($C_1$-$C_6$) -Alkoxy, geradkettigem oder verzweigtem ($C_1$-$C_6$)-Acyl, Carboxy, geradkettigem oder verzweigtem ($C_1$-$C_6$)-Alkoxycarbonyl und geradkettigem oder verzweigtem ($C_1$-$C_6$)-Trihalogenalkyl,
- Amino (gegebenenfalls substituiert durch ein oder zwei gleichartige oder verschiedenartige Gruppen, die unabhängig voneinander ausgewählt sind aus geradkettigem oder verzweigtem ($C_1$-$C_6$)-Alkyl, Aryl oder geradkettigem oder verzweigtem Aryl-($C_1$-$C_6$)-alkyl),
- Aryloxy, geradkettigem oder verzweigtem Aryl-($C_1$-$C_6$-)-alkoxy, Heteroaryloxy, und geradkettigem oder verzweigtem Heteroaryl-($C_1$-$C_6$)-alkoxy,
- und einer Gruppe der Formel -$U_1$-$V_1$-$U_2$-$V_2$ in der $U_1$, $U_2$, $V_1$ und $V_2$ die oben angegebenen Bedeutungen besitzen,
- oder jeweils zu zweit gemeinsam eine Methylendioxy- oder Ethylendioxy-Gruppe bilden (wobei jede dieser Gruppen gegebenenfalls durch ein oder zwei geradkettige oder verzweigte ($C_1$-$C_6$-)-Alkylgruppen, Arylgruppen oder geradkettige oder verzweigte Aryl-($C_1$-$C_6$)-alkylgruppen substituiert ist),

$R_1$

- eine Arylgruppe, die durch einen bis fünf gleichartige oder verschiedenartige Substituenten substituiert ist, die unabhängig voneinander ausgewählt sind aus Halogen, Hydroxy, Cyano, Nitro, Carboxy, geradkettigem oder verzweigtem ($C_1$-$C_6$)-Alkyl, geradkettigem oder verzweigtem ($C_1$-$C_6$)-Alkoxy, geradkettigem oder verzweigtem ($C_1$-$C_6$)-Acyl, geradkettigem oder verzweigtem ($C_1$-$C_6$)-Alkoxycarbonyl, geradkettigem oder verzweigtem ($C_1$-$C_6$)-Trihalogenalkyl (welches gegebenenfalls durch eine Hydroxygruppe substituiert ist), geradkettigem oder verzweigtem ($C_1$-$C_6$)-Trihalogenalkoxy, Amino (welches gegebenenfalls durch eine oder zwei geradkettige oder verzweigte ($C_1$-$C_6$)-Alkylgruppen substituiert ist, wobei diese Alkylgruppen gegebenenfalls durch eine Gruppe ausgewählt aus Amino, geradkettigem oder verzweigtem ($C_1$-$C_6$)Alkylamino und geradkettigem oder verzweigtem Di-($C_1$-$C_6$)-alkylamino substituiert sind), geradkettigem oder verzweigtem ($C_1$-$C_6$)-Aminoalkoxy (wobei der Aminorest gegebenenfalls durch ein oder zwei gleichartige oder verschiedenartige, geradkettige oder verzweigte ($C_1$-$C_6$)-Alkylgruppen substituiert ist), geradkettigem oder verzweigtem Alkoxycarbonyl-($C_1$-$C_6$)-alkyl, geradkettigem oder verzweigtem ($C_1$-$C_6$)-Alkylcarbonylamino, geradkettigem oder verzweigtem Aryl-($C_1$-$C_6$)-alkyl, Aryloxy, geradkettigem oder verzweigtem Aryl-($C_1$-$C_6$)-alkoxy, Arylamino, geradkettigem oder verzweigtem Aryl-($C_1$-$C_6$)-alkylamino, Arylsulfanyl, geradkettigem oder verzweigtem Aryl-($C_1$-$C_6$)-alkylsulfanyl, Heteroaryl, geradkettigem oder verzweigtem Heteroaryl-($C_1$-$C_6$)-alkyl, Heteroaryloxy, geradkettigem oder verzweigtem Heteroaryl-($C_1$-$C_6$)-alkoxy, Heteroarylamino, geradkettigem oder verzweigtem Heteroaryl-($C_1$-$C_6$)-alkylamino, Heteroarylsulfanyl und geradkettigem oder verzweigtem Heteroaryl-($C_1$-$C_6$)-alkylsulfanyl,
- eine 1,3-Dihydro-2*H*-indol-2-on-, 3,4-Dihydro-2(1*H*)-chinolinon- oder 1-Hydroxy-2(1*H*)-pyridinon-Gruppe.
- oder eine gegebenenfalls substituierte Heteroaryl-Gruppe bedeutet,

$R_2$ eine Gruppe bedeutet, ausgewählt aus dem Wasserstoffatom, geradkettigen oder verzweigten ($C_1$-$C_6$)-Alkyl-, Aryl-, geradkettigen oder verzweigten Aryl-($C_1$-$C_6$)-alkyl-, Cycloalkyl-, Heterocycloalkyl-, geradkettigen oder verzweigten Heterocycloalkyl-($C_1$-$C_6$)-alkyl-, Heteroaryl- und geradkettigen oder verzweigten Heteroaryl-($C_1$-$C_6$)-alkyl-Gruppen,
mit der Maßgabe, daß:

- man unter einer Arylgruppe eine Phenyl-, Biphenyl-, Naphthyl-, Tetrahydronaphthyl- oder

Dihydronaphthyl-Gruppe versteht, wobei.jede dieser Gruppen gegebenenfalls in gleichartiger oder unterschiedlicher Weise substituiert ist durch eine oder mehrere Gruppen ausgewählt aus einen Halogenatom, Hydroxy, Cyano, Nitro, geradkettigen oder verzweigtem ($C_1$-$C_6$)-Alkyl (das gegebenenfalls durch eine oder mehrere Gruppen ausgewählt aus Hydroxy, Amino, geradkettigem oder verzweigtem Mono- oder Di-($C_1$-$C_6$)-alkylamino substituiert sind), geradkettigem oder verzweigtem ($C_1$-$C_6$)-Trihalogenalkyl, geradkettigem oder verzweigtem ($C_1$-$C_6$)-Alkoxy, Aryloxy, geradkettigem oder verzweigtem ($C_1$-$C_6$)-Acyl, Carboxy, geradkettigem oder verzweigtem ($C_1$-$C_6$)-Alkoxycarbonyl und Amino (welches gegebenenfalls durch ein oder zwei gleichartige oder verschiedenartige geradkettige oder verzweigte ($C_1$-$C_6$)-Alkylgruppen substituiert ist), substituiert ist,

- man unter einer Cycloalkyl-Gruppe eine mono- oder bicyclische Gruppe versteht, die 3 bis 8 Kohlenstoffatome umfaßt,

- man unter Hetercycloalkyl eine mono- oder bicyclische, gesättigte oder ungesättigte Gruppe mit nicht-aromatischem Charakter mit 5 bis 12 Kettengliedern, die eines, zwei oder drei gleichartige oder verschiedenartige Heteroatome ausgewählt aus Sauerstoff, Stickstoff und Schwefel, enthält, versteht, mit der Maßgabe, daß die Heterocycloalkyl-Gruppe gegebenenfalls in gleichartiger oder unterschiedlicher Weise durch ein oder mehrere Gruppen substituiert sein kann, ausgewählt aus Halogenatomen, Hydroxy-, geradkettigen oder verzweigtem ($C_1$-$C_6$)-Alkyl-, geradkettigen oder verzweigten ($C_1$-$C_6$)-Trihalogenalkyl-, geradkettigen oder verzweigten ($C_1$-$C_6$)-Alkoxy-, Aryloxy-, geradkettigen oder verzweigten ($C_1$-$C_6$)-Arylalkoxy-, Amino- (gegebenenfalls substituiert durch ein oder zwei geradkettige oder verzweigte ($C_1$-$C_6$)-Alkylgruppen), geradkettigen oder verzweigten ($C_1$-$C_6$)-Acyl-, geradkettigen oder verzweigten ($C_1$-$C_6$)-Alkoxycarbonyl-, Nitro- und Oxo-Gruppen,

- man unter Heteroaryl eine mono- oder bicyclische Heterocycloalkyl-Gruppe versteht, wie sie oben definiert worden ist, wobei mindestens einer der Ringe einen aromatischen Charakter aufweist und wobei das oder die Heteroatome im Fall eines bicyclischen Systems in dem Ring mit aromatischem Charakter oder dem teilweise ungesättigten Ring vorliegen, mit der Maßgabe, daß die Heteroarylgruppe gegebenenfalls durch eine oder mehrere gleichartige oder verschiedenartige Gruppen, wie sie oben bei der Definition der Sustituenten der Heterocycloalkyl-Gruppe definiert worden sind, substituiert sind,

- deren Isomere sowie deren Additionssalze mit einer pharmazeutisch annehmbaren Säure oder Base.

**2.** Verbindungen der Formel (I) nach Anspruch 1, **dadurch gekennzeichnet, daß** X ein Schwefelatom oder eine Gruppe $NR_3$ bedeutet, worin $R_3$ die bezüglich der Formel (I) angegebenen Bedeutungen besitzt, deren Isomere sowie deren Additionssalze mit einer pharmazeutisch annehmbaren Säure oder Base.

**3.** Verbindungen der Formel (I) nach Anspruch 1, **dadurch gekennzeichnet, daß** Y ein Sauerstoffatom bedeutet, deren Isomere sowie deren Additionssalze mit einer pharmazeutisch annehmbaren Säure oder Base.

**4.** Verbindungen der Formel (I) nach Anspruch 1, **dadurch gekennzeichnet, daß** $R_1$ eine Gruppe bedeutet ausgewählt aus Phenyl, gegebenenfalls substituiert durch eine Gruppe, wie sie bezüglich der Formel (I) definiert worden ist, gegebenenfalls substituiertem Chinolyl und gegebenenfalls substituiertem Pyridinyl, deren Isomere sowie deren Additionssalze mit einer pharmazeutisch annehmbaren Säure oder Base.

**5.** Verbindungen der Formel (I) nach Anspruch 1, **dadurch gekennzeichnet, daß** $R_2$ eine Gruppe bedeutet ausgewählt aus Aryl und Heteroaryl, wobei jede dieser Gruppen gegebenenfalls substituiert ist, deren Isomere sowie deren Additionssalze mit einer pharmazeutisch annehmbaren Säure oder Base.

**6.** Verbindungen der Formel (I) nach einem der Ansprüche 1 und 5, **dadurch gekennzeichnet, daß** $R_2$ eine Pyridinylgruppe bedeutet, deren Isomere sowie deren Additionssalze mit einer pharmazeutisch annehmbaren Säure oder Base.

**7.** Verbindungen der Formel (I) nach Anspruch 1, **dadurch gekennzeichnet, daß** $U_1$ eine geradkettige ($C_1$-$C_4$)-Al-

kylenkette bedeutet, bei der eines der Kohlenstoffatome durch ein Sauerstoffatom ersetzt ist, $V_1$ eine Arylengruppe bedeutet, $U_2$ eine Einfachbindung bedeutet und $V_2$ eine Arylgruppe bedeutet, die gegebenenfalls durch eine Gruppe substituiert ist, wie sie bezüglich der Formel (I) definiert worden ist, deren Isomere sowie deren Additionssalze mit einer pharmazeutisch annehmbaren Säure oder Base.

**8.** Verbindungen der Formel (I) nach einem der Ansprüche 1 und 7, **dadurch gekennzeichnet, daß** $-U_1-V_1-U_2-V_2$ eine [1,1'-Biphenyl]-4-ylmethoxy-Gruppe bedeutet, deren Isomere sowie deren Additionssalze mit einer pharmazeutisch annehmbaren Säure oder Base.

**9.** Verbindungen der Formel (I) nach Anspruch 1, **dadurch gekennzeichnet, daß** mindestens eine der Gruppen Ra, Rb oder Rc eine Gruppe der Formel $-U_1-V_1-U_2-V_2$ bedeutet, wie sie bezüglich der Formel (I) definiert worden ist, deren Isomere sowie deren Additionssalze mit einer pharmazeutisch annehmbaren Säure oder Base.

**10.** Verbindungen der Formel (I) nach einem der Ansprüche 1 bis 3, **dadurch gekennzeichnet, daß** X ein Schwefelatom und Y ein Sauerstoffatom bedeuten, deren Isomere sowie deren Additionssalze mit einer pharmazeutisch annehmbaren Säure oder Base.

**11.** Verbindungen der Formel (I) nach Anspruch 1, **dadurch gekennzeichnet, daß** X ein Schwefelatom, Y ein Sauerstoffatom, $R_1$ eine,gegebenenfalls substituierte Phenylgruppe oder eine gegebenenfalls substituierte Pyridinylgruppe und A eine Einfachbindung, wenn T ein Kohlenstoffatom darstellt, oder eine Gruppe CH bedeuten, deren Isomere sowie deren Additionssalze mit einer pharmazeutisch annehmbaren Säure oder Base.

**12.** Verbindungen der Formel (I) nach Anspruch 1, **dadurch gekennzeichnet, daß** X ein Schwefelatom, Y ein Sauerstoffatom, $R_1$ eine gegebenenfalls durch eine Gruppe, wie sie bezüglich der Formel (I) definiert worden ist, substituierte Phenylgruppe und A eine (gegebenenfalls durch eine geradkettige oder verzweigte ($C_1$-$C_6$)-Alkylgruppe, Arylgruppe oder geradkettige oder verzweigte Aryl-($C_1$-$C_6$)-alkylgruppe substituierte) Alkylengruppe oder eine Arylengruppe, wenn T ein Stickstoffatom darstellt, bedeuten, deren Isomere sowie deren Additionssalze mit einer pharmazeutisch annehmbaren Säure oder Base.

**13.** Verbindungen der Formel (I) nach Anspruch 1, **dadurch gekennzeichnet, daß** sie die Verbindungen der Formel (Ibis) sind:

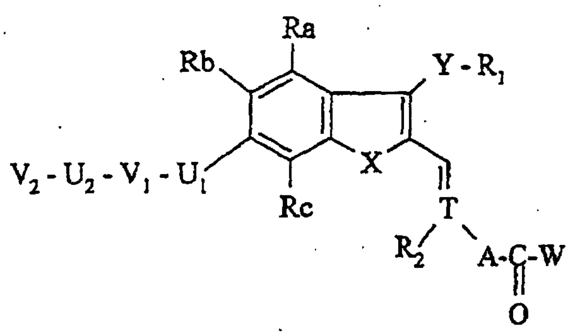

(I bis)

in der:

X ein Schwefelatom,
Y ein Sauerstoffatom,
$R_1$ eine gegebenenfalls substituierte Phenylgruppe oder eine Heteroarylgruppe ausgewählt aus gegebenenfalls substituiertem Pyridinyl und gegebenenfalls substituiertem Chinolinyl,
A eine Einfachbindung,
T ein Kohlenstoffatom.
Ra und Rc jeweils ein Wasserstoffatom,
$U_1$ eine geradkettige (C1-C4)-Alkylenoxykette,
$V_1$ eine Arylgruppe,
$U_2$ eine Einfachbindung,
$V_2$ eine Arylgruppe,

Rb eine Gruppe $U_1$-$V_1$-$U_2$-$V_2$, wie sie oben definiert worden ist,
$R_2$ eine Heteroarylgruppe, und
W eine Gruppe, wie sie bezüglich der Formel (I) definiert ist, bedeuten, deren Isomere sowie deren Additionssalze mit einer pharmazeutisch annehmbaren Säure oder Base.

**14.** Verbindungen der Formel (I) nach Anspruch 13, **dadurch gekennzeichnet, daß** sie Verbindungen der Formel (Ibis) sind, in der Rb und -$U_1$-$V_1$-$U_2$-$V_2$ jeweils eine [1,1'-Biphenyl]-4-ylmethozcy-Gruppe bedeuten, deren Isomere sowie deren Additionssalze mit einer pharmazeutisch annehmbaren Säure oder Base.

**15.** Verbindungen der Formel (I) nach Anspruch 13, **dadurch gekennzeichnet, daß** sie Verbindungen der Formel (Ibis) sind, in der $R_2$ eine Pyridinylgruppe darstellt, deren Isomere sowie deren Additionssalze mit einer pharmazeutisch annehmbaren Säure oder Base.

**16.** Verbindungen der Formel (I) nach Anspruch 13, **dadurch gekennzeichnet, daß** sie Verbindungen der Formel (Ibis) sind, in der $R_1$:

- eine Phenylgruppe, die gegebenenfalls durch ein bis drei Gruppen substituiert ist, ausgewählt aus Halogenatomen, geradkettigen oder verzweigten ($C_1$-$C_6$)-Alkylgruppen, Heteroarylgruppen, geradkettigen oder verzweigten Heteroaryl-($C_1$-$C_6$)-alkoxygruppen, geradkettigen oder verzweigten ($C_1$-$C_6$)-Alkoxygruppen und geradkettigen oder verzweigten ($C_1$-$C_6$)-Aminoalkoxygruppen, wobei der Aminorest durch ein oder zwei gleichartige oder verschiedene, geradkettige oder verzweigte ($C_1$-$C_6$)-Alkylgruppen substituiert sein kann,
- oder eine Heteroarylgruppe ausgewählt aus Pyridinyl und Chinolinyl, die gegebenenfalls durch ein Halogenatom oder eine geradkettige oder verzweigte ($C_1$-$C_6$)-Alkylgruppe substituiert sind, bedeutet,

deren Isomere sowie deren Additionssalze mit einer pharmazeutisch annehmbaren Säure oder Base.

**17.** Verbindung der Formel (I) nach Anspruch 1, nämlich (E)-3-[5,6-Bis ([1,1'-biphenyl]-4-ylmethoxy)-3-(4-chlorphenoxy)-benzo-[*b*]thiophen-2-yl]-2-(4-pyridinyl)-2-propensäure, deren Isomere sowie deren Additionssalze mit einer pharmazeutisch annehmbaren Säure oder Base.

**18.** Verbindungen der Formel (I) nach Anspruch 1, nämlich:

- (*E*)-3-[5,6-Bis-([1,1'-biphenyl]-4-ylmethoxy)-3-(4-chlorphenoxy)-benzo[*b*]-thiophen-2-yl]-2-(4-pyridinyl)-2-propensäurethylester,
- (*E*)-3-[5,6-Bis-([1,1'-biphenyl]-4-ylmethoxy)-3-(3-pyridinyloxy)-benzo[*b*]-thiophen-2-yl]-2-(4-pyridinyl)-2-propensäure,
- 3-{*E*}-{5,6-Bis-([1,1'-biphenyl]-4-ylmethoxy)-3-[6-(methyl)-pyridinyl-3-oxy]-benzo[*b*]thiophen-2-yl}-2-(4-pyridinyl)-2-propensäure,
- 3-(*E*)-[5,6-Bis-([1,1'-biphenyl]-4-ylmethoxy)-3-(6-chinolinyloxy)-benzo[*b*]thiophen-2-yl]-2-(4-pyridinyl)-2-propensäure,
- (*E*)-3-[5,6-Bis-([1,1'-biphenyl]-2-ylmethoxy)-3-(4-chlorphenoxy)-benzo[*b*]-thiophen-2-yl]-2-(4-pyridinyl)-2-propensäure,
- (*E*)-3-[5,6-Bis-([1,1'-biphenyl]-3-ylmethoxy)-3-(4-chlorphenoxy)-benzo[*b*]-thiophen-2-yl]-2-(4-pyridinyl)-2-propensäure,
- (*E*)-3-[5,6-Bis-([1,1'-biphenyl]-4-ylmethoxy)-3-(4-chlor-3-fluorphenoxy)-benzo[*b*]thiophen-2-yl]-2-(4-pyridinyl)-2-propensäure,
- (*E*)-3-[5,6-Bis([1,1'-biphenyl]-4-ylmethoxy)-3-(4-chlor-3,5-dimethylphenoxy)benzo[*b*]thiophen-2-yl]-2-(4-pyridinyl)-2-propensäure.
- (*E*)-3-[5,6-Bis([1,1'-biphenyl]-4-ylmethoxy)-3-(4-chlor-3- methylphenoxy)-benzo[*b*]thiophen-2-yl]-2-(4-pyridinyl)-2-propensäure.
- (*E*)-3-{5,6-Bis([1,1'-biphenyl]-4-ylmethoxy)-3-[4-(4-pyridinyloxy)-phenoxy]-benzo[*b*]thiophen-2-yl}-2-(4-pyridinyl)-2-propensäure.
- (*E*)-3-{5,6-Bis([1,1'-biphenyl]-4-ylmethoxy)-3-[4-(1*H*-imidazol-1-yl)-phenoxybenzo[*b*]thiophen-2-yl}-2-(4-pyridinyl)-2-propensäure.
- (*E*)-3-[5,6-Bis ([1,1'-biphenyl]-4-ylmethoxy)-3-phenoxy-benzo[*b*]thiophen-2-yl]-2-(4-pyridinyl)-2-propensäure,
- (*E*)-3-[5,6-Bis([1,1'-biphenyl]-4-ylmethoxy)-3-(3-fluorphenoxy)-benzo[*b*]thiophen-2-yl]-2-(4-pyridinyl)-2-propensäure.
- (*E*)-3-[5,6-Bis([1,1'-biphenyl]-4-ylmethoxy)-3-(3,4-difluorphenoxy)-benzo[*b*]-thiophen-2-yl]-2-(4-pyridinyl)-2-propensäure.
- (*E*)-3-{5,6-Bis([1,1'-biphenyl]-4-ylmethoxy)-3-[(6-chlor-3-pyridinyl)-oxy]benzo[*b*]thiophen-2-yl}-2-pyridinyl)-2-propensäure.

deren Isomere sowie deren Additionssalze mit einer pharmazeutisch annehmbaren Säure oder Base.

**19.** Verfahren zur Herstellung der Verbindungen der Formel (I) nach Anspruch 1, **dadurch gekennzeichnet, daß** man als Ausgangsprodukt eine Verbindung der Formel (II) verwendet:

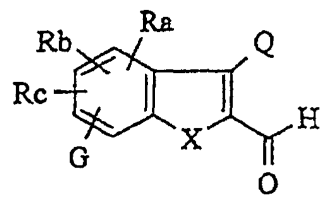

**(II)**

in der Ra, Rb, Rc und X die bezüglich der Formel (I) angegebenen Bedeutungen besitzen, G eine durch eine in klassischer Weise in der organischen Synthese verwendete Schutzgruppe geschützte Hydroxygruppe und Q ein Halogenatom oder eine Hydroxygruppe bedeuten, wobei vorzugsweise Q ein Halogenatom bedeutet, wenn X ein Schwefelatom eine Gruppe $NR_3$ darstellt, wenn $R_3$ die bezüglich der Formel (I) angegebenen Bedeutungen besitzt, und Q eine Hydroxygruppe darstellt, wenn X ein Sauerstoffatom bedeutet,
welche Verbindung der Formel (II) man unter basischen Bedingungen

- *entweder wenn Q ein Halogenatom darstellt*:

  ✧ mit einer Verbindung der Formel (III)

$$H - Y_1 - R_1 \qquad \textbf{(III)}$$

in der $R_1$ die bezüglich der Formel (I) angegebenen Bedeutungen besitzt und $Y_1$ ein Sauerstoffatom, ein Schwefelatom oder eine Gruppe $NR_3$, in der $R_3$ die bezüglich der Formel (I) angegebenen Bedeutungen besitzt, bedeutet, umsetzt, zur Bildung der Verbindungen der Formel (IV/a):

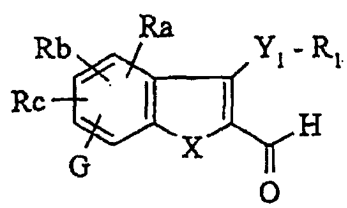

**(IV/a)**

in der Ra, Rb, Rc, G, $R_1$, X und $Y_1$ die oben angegebenen Bedeutungen besitzen,

  ✧ oder mit einer Verbindung der Formel (V):

$$(HO)_2B\text{-}R_1 \qquad \textbf{(V)}$$

in der R1 die bezüglich der Formel (I) angegebenen Bedeutungen besitzt, umsetzt, zur Bildung der Verbindungen der Formel (IV/b):

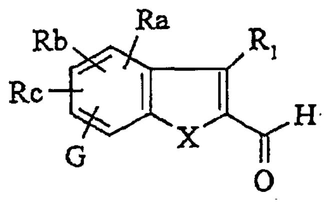

(IV/b)

in der Ra, Rb, Rc, G und $R_1$ die oben angegebenen Bedeutungen besitzen und $X_1$ eine Gruppe $NR_3$ darstellt, in der $R_3$ eine geradkettige oder verzweigte Heteroaryl-($C_1$-$C_6$)-alkylgruppe bedeutet,

- *oder wenn Q eine Hydroxygruppe darstellt*,
  mit einer Verbindung der Formel (VI):

$$Hal - R_1 \qquad \textbf{(VI)}$$

in der Hal ein Halogenatom darstellt und $R_1$ die oben angegebenen Bedeutungen besitzt, umsetzt, zur Bildung der Verbindungen der Formel (IV/c):

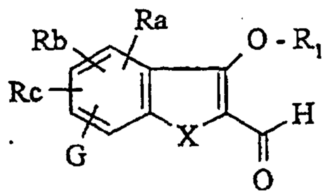

(IV/c)

in der Ra, Rb, Rc, G, X und $R_1$ die oben angegebenen Bedeutungen besitzen,
wobei die Gesamtheit der Verbindungen der Formeln (IV/a), (IV/b) und (IV/C) die Verbindungen der Formel (IV) bilden:

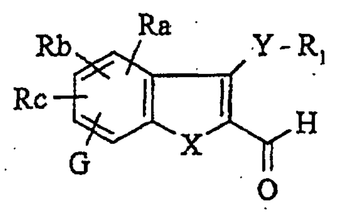

(IV)

in der Ra, Rb, Rc, G, $R_1$, X und Y die bezüglich der Formel (I) angegebenen Bedeutungen besitzen,
welche Verbindungen der Formel (IV):

♦ man in Gegenwart von Essigsäureanhydrid mit einer Verbindung der Formel (VII) kondensiert:

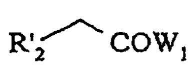

(VII)

in der $R'_2$ die für $R_2$ bezüglich der Formel (I) angegebenen Bedeutungen besitzt mit der Maßgabe, daß $R'_2$ kein Wasserstoffatom bedeutet, und $W_1$ eine Gruppe darstellt, ausgewählt aus geradkettigem oder verzweigtem ($C_1$-$C_6$)-Alkoxy, Aryloxy, geradkettigem oder verzweigtem Aryl-($C_1$-$C_6$)-alkoxy, Cycloalkyloxy, Heterocycloalkoxy, Heteroaryloxy und eine Aminogruppe (die ihrerseits gegebenenfalls unabhängig voneinander durch ein oder zwei gleichartige oder verschiedenartige Gruppen ausgewählt aus geradkettigem oder verzweigtem ($C_1$-$C_6$)-Alkyl, Aryl, geradkettigem oder verzweigtem Aryl-($C_1$-$C_6$)-alkyl und Cycloalkyl substituiert sind),
zur Bildung der Verbindungen der Formel (VIII):

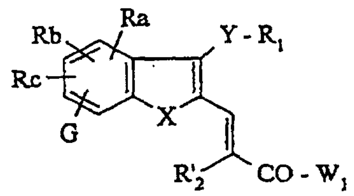

(VIII)

in der Ra, Rb, Rc, G, $R_1$, $R'_2$, X, Y und $W_1$ die oben angegebenen Bedeutungen besitzen, von welchen Verbindungen der Formel (VIII) man die Schutzgruppe der Hydroxygruppe bei klassischen Bedingungen der organischen Synthese abspaltet und sie dann in basischem Medium mit einer Verbindung der Formel (IX) umsetzt:

$$V_2 - U_2\ V_1 - U_1 - Hal \qquad \textbf{(IX)}$$

in der $U_1$, $V_1$, $U_2$ und $V_2$ die bezüglich der Formel (I) angegebenen Bedeutungen besitzen und Hal ein Halogenatom darstellt,
zur Bildung der Verbindungen der Formel (I/a), einem Sonderfall der Verbindungen der Formel (I):

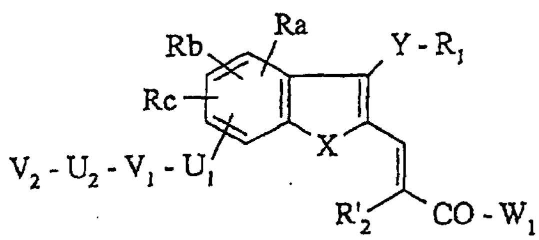

(I/a)

in der Ra, Rb, Rc, $U_1$, $V_1$, $U_2$, $V_2$, $R_1$, $R'_2$, X, Y und $W_1$ die oben angegebenen Bedeutungen besitzen, welche Verbindungen der Formel (I/a) man gewünschtenfalls:

- ✱ entweder den Bedingungen der katalytischen Hydrierung in Gegenwart von Palladium unterwirft zur Bildung der Verbindungen der Formel (I/b), im Sonderfall den Verbindungen der Formel (I):

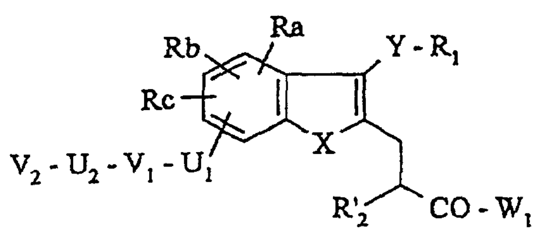

(I/b)

in der Ra, Rb, Rc, $U_1$, $V_1$, $U_2$, $V_2$, $R_1$, $R'_2$, X, Y und $W_1$ die oben angegebenen Bedeutungen besitzen,

- ✱ oder den Bedingungen der Hydrolyse in basischem Medium unterwirft zur Bildung der Verbindungen der Formel (I/c), einem Sonderfall der Verbindungen der Formel (I):

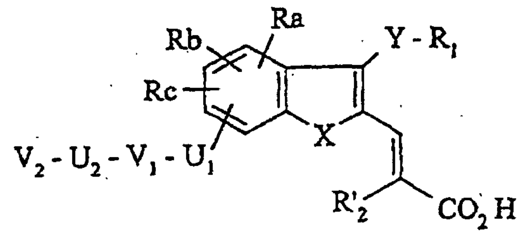

(I/c)

in der Ra, Rb, Rc, $U_1$, $V_1$, $U_2$, $V_2$, $R_1$, $R'_2$, X und Y die oben angegebenen Bedeutungen besitzen, bei welchem Verbindungen der Formel (I/c) man gewünschtenfalls die Doppelbindung durch katalytische Hydrierung reduziert zur Bildung der Verbindungen der Formel (I/d), einem Sonderfall der Verbindungen der Formel (I):

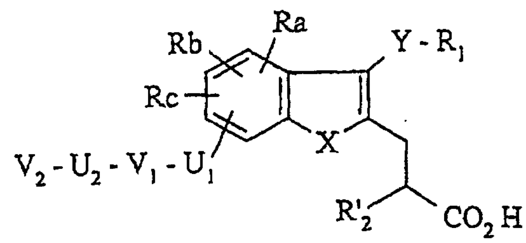

(I/d)

in der Ra, Rb, Rc, $U_1$, $V_1$, $U_2$, $V_2$, $R_1$, $R'_2$, X und Y die oben angegebenen Bedeutungen besitzen,

- oder der Einwirkung eines Phosphorylids der Formel (X) unterwirft:

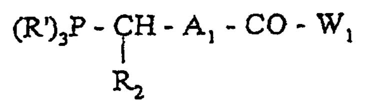

(X)

in der R1 eine geradkettige oder verzweigte ($C_1$-$C_6$)-Alkylgruppe oder eine Phenylgruppe bedeutet, $R_2$ die bezüglich der Formel (I) angegebenen Bedeutungen besitzt, $W_1$ die oben angegebenen Bedeutungen besitzt und $A_1$ eine Einfachbindung, eine (gegebenenfalls durch ein oder mehrere Gruppen ausgewählt aus geradkettigem oder verzweigtem ($C_1$-$C_6$)-Alkyl, Aryl, geradkettigem oder verzweigtem Aryl-($C_1$-$C_6$)-alkyl, Cycloalkyl, Heterocycloalkyl und Heteroaryl) substituierte Alkylengruppe, eine Arylengruppe, Cycloalkylengruppe, Heterocycloalkylengruppe oder Heteroarylengruppe darstellt,
zur Bildung der Verbindungen der Formel (XI):

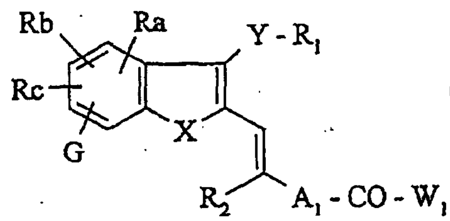

(XI)

in der Ra, Rb, Rc, G, $R_1$, $R_2$, X, Y, $A_1$ und $W_1$ die oben angegebenen Bedeutungen besitzen,
von welchen Verbindungen der Formel (X) man die Schutzgruppe der Hydroxygruppe unter Anwendung klassischer Bedingungen der organischen Synthese abspaltet und sie dann in basischem Medium mit einer Verbindung der Formel (IX) umsetzt:

$$V_2 - U_2 - V_1 - U_1 - Hal \qquad \textbf{(IX)}$$

in der $U_1$, $V_1$, $U_2$ und $V_2$ die bezüglich der Formel (I) angegebenen Bedeutungen besitzen und Hal ein Halogenatom darstellt, zur Bildung der Verbindungen der Formel (I/e), einem Sonderfall der Verbindungen der Formel (I):

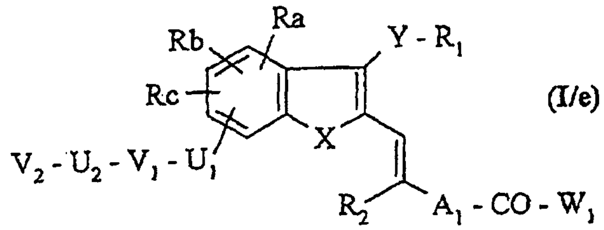

(I/e)

in der in der Ra, Rb, Rc, $U_1$, $V_1$, $U_2$, $V_2$, $R_1$, $R_2$, $A_1$, X, Y und $W_1$ die oben angegebenen Bedeutungen besitzen,
welche Verbindungen der Formel (I/e) man gewünschtenfalls:

✱ entweder den Bedingungen der Hydrolyse unter basischen Bedingungen unterwirft zur Bildung der Verbindungen der Formel (I/f), einem Sonderfall der Verbindungen der Formel (I):

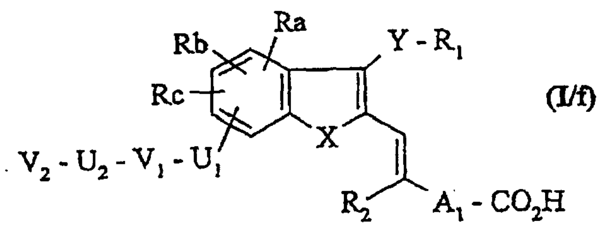

(I/f)

in der Ra, Rb, Rc, $U_1$, $V_1$, $U_2$, $V_2$, $R_1$, $R_2$, X, Y und $A_1$ die oben angegebenen Bedeutungen besitzen,

✱ oder den Bedingungen der katalytischen Hydrierung unterwirft zur Bildung der Verbindungen der Formel (I/g), einem Sonderfall der Verbindungen der Formel (I):

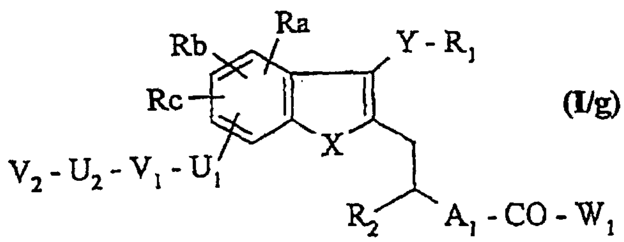

(I/g)

in der Ra, Rb, Rc, $U_1$, $V_1$, $U_2$, $V_2$, $R_1$, $R_2$, X, Y, $A_1$ und $W_1$ die oben angegebenen Bedeutungen besitzen,
welche Verbindungen der Formel (I/g) man bei den Bedingungen der basischen Hydrolyse behandeln kann zur Bildung der Verbindungen der Formel (I/h), einem Sonderfall der Verbindungen der Formel (I):

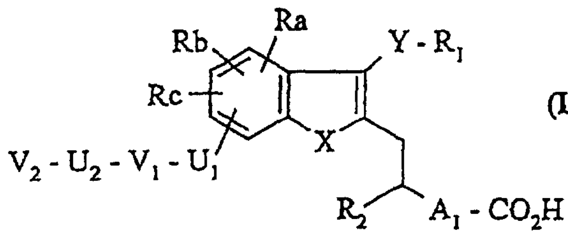

**(I/h)**

in der Ra, Rb, Rc, $U_1$, $V_1$, $U_2$, $V_2$, $R_1$, $R_2$, X, Y und $A_1$ die oben angegebenen Bedeutungen besitzen,

- oder von denen man die Schutzgruppe der Hydroxyfunktion unter Anwendung klassischer Bedingungen der organischen Synthese abspaltet und sie dann in basischem Medium mit einer Verbindung der Formel (IX) umsetzt:

$$V_2 - U_2 - V_1 - U_1 - Hal \qquad \textbf{(IX)}$$

in der $U_1$, $V_1$, $U_2$ und $V_2$ die bezüglich der Formel (I) angegebenen Bedeutungen besitzen und Hal ein Halogenatom darstellt, zur Bildung der Verbindungen der Formel (XII):

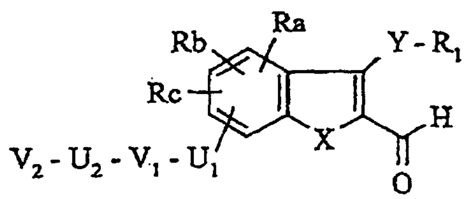

**(XII)**

in der in der Ra, Rb, Rc, $U_1$, $V_1$, $U_2$, $V_2$, $R_1$, X und Y die oben angegebenen Bedeutungen besitzen, von welchen Verbindungen der Formel (XII) man die Aldehydfunktion zu dem primären Alkohol reduziert zur Bildung der Verbindungen der Formel (XIII) :

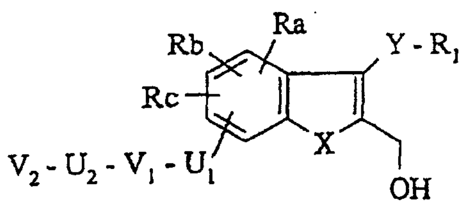

**(XIII)**

in der Ra, Rb, Rc, $U_1$, $V_1$, $U_2$, $V_2$, $R_1$ X und Y die bezüglich der Formel (I) angegebenen Bedeutungen besitzen,
bei welchen Verbindungen der Formel (XIII) man die endständige Hydroxygruppe unter Anwendung klassischer Bedingungen durch ein Halogen substituiert zur Bildung der Verbindungen der Formel (XIV):

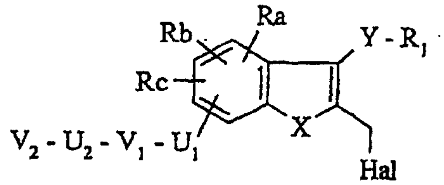

**(XIV)**

in der Ra, Rb, Rc, $U_1$, $V_1$, $U_2$, $V_2$, $R_1$, X und Y die oben angegebenen Bedeutungen besitzen, und Hal ein

Chlor- oder Bromatom bedeutet.
bei welchen Verbindungen der Formel (XIV):

✱ man das Halogenatom unter basischen Bedingungen mit einem Aminderivat der Formel (XV) substituiert:

$$R_2\text{-NH-}A_1\text{-CO-}W_1 \qquad \textbf{(XV)}$$

in der $R_2$ die bezüglich der Formel (I) angegebenen Bedeutungen besitzt und $A_1$ und $W_1$ die oben angegebenen Bedeutungen aufweisen,
zur Bildung der Verbindungen der Formel (I/i), einem Sonderfall der Verbindungen der Formel (I):

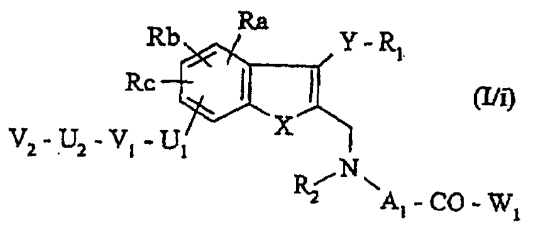

(I/i)

in der Ra, Rb, Rc, $U_1$, $V_1$, $U_2$, $V_2$, $R_1$, $R_2$, X, Y, $A_1$ und $W_1$ die oben angegebenen Bedeutungen besitzen,
bei welchen Verbindungen der Formel (I/i) man die endständige Carbonylgruppe unter basischen Bedingungen hydrolysiert zur Bildung der Verbindungen der Formel (I/j), einem Sonderfall der Verbindungen der Formel (I):

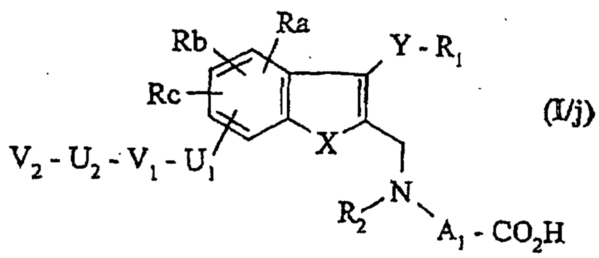

(I/j)

in der Ra, Rb, Rc, $U_1$, $V_1$, $U_2$, $V_2$, $R_1$, $R_2$, X, Y und $A_1$ die oben angegebenen Bedeutungen besitzen,

✱ oder man in einer ersten Stufe mit Natriumazid behandelt, worauf man das erhaltene Azid unter den Bedingungen der katalytischen Hydrierung zu einem primären Amin reduziert zur Bildung der Verbindungen der Formel (XVI):

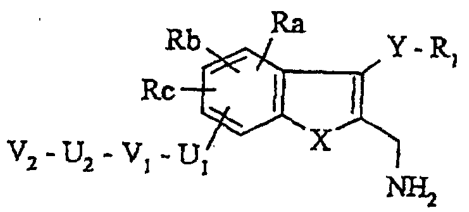

(XVI)

in der Ra, Rb, Rc, $U_1$, $V_1$, $U_2$, $V_2$, $R_1$, X und Y die bezüglich der Formel (I) angegebenen Bedeutungen besitzen,
welche Verbindungen der Formel (XVI) man unter basischen Bedingungen mit einem Chlorsulfonyl-Derivat der Formel (XVII) kondensiert:

$Cl - SO_2 - R_4 - CO - W_1$ **(XVII)**

in der R4 die bezüglich der Formel (I) angegebenen Bedeutungen besitzt und $W_1$ die oben angegebenen Bedeutungen aufweist, zur Bildung der Verbindungen der Formel (I/k), einem Sonderfall der Verbindungen der Formel (I):

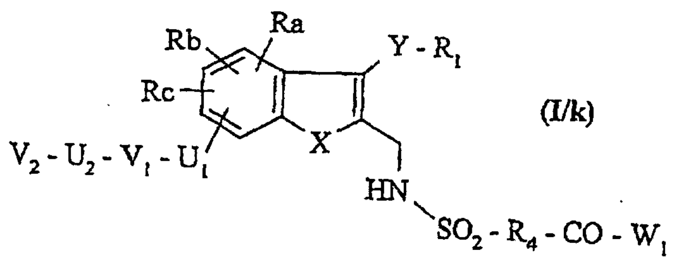

(I/k)

in der Ra, Rb, Rc, $U_1$, $V_1$, $U_2$, $V_2$, $R_1$, $R_4$, X, Y und $W_1$ die oben angegebenen Bedeutungen besitzen,

welche Verbindungen der Formel (I/k):

- man gewünschtenfalls den Bedingungen der Hydrolyse unter basischen Bedingungen unterwirft zur Bildung der Verbindungen der Formel (1/1), einem Sonderfall der Verbindungen der Formel (I):

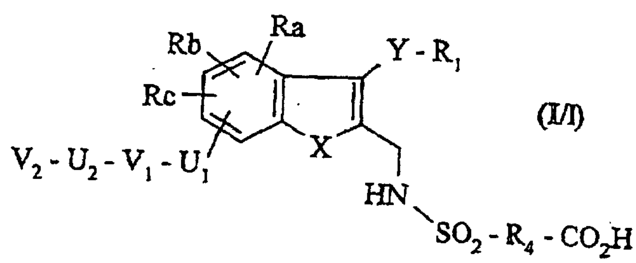

(I/l)

in der Ra, Rb, Rc, $U_1$, $V_1$, $U_2$, $V_2$, $R_1$, $R_4$, X und Y die oben angegebenen Bedeutungen besitzen,

- oder man in basischem Medium mit einer Verbindung der Formel (XVIII) kondensiert:

$Hal - R'_2$ **(XVIII)**

in der Hal ein Halogenatom, wie Iod bedeutet, und $R'_2$ die oben angegebenen Bedeutungen besitzt, zur Bildung der Verbindungen der Formel (I/m), einem Sonderfall der Verbindungen der Formel (I):

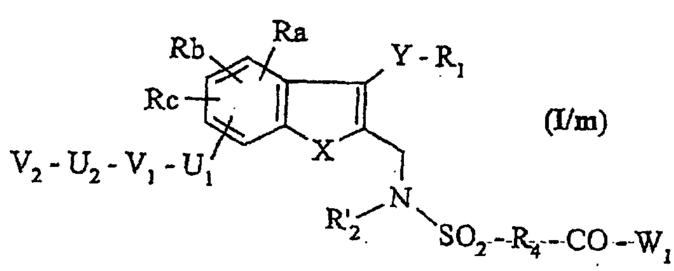

(I/m)

in der Ra, Rb, Rc, $U_1$, $V_1$, $U_2$, $V_2$, $R_1$, $R'_2$, $R_4$, X, Y und $W_1$ die oben angegebenen Bedeutungen besitzen, welche Verbindungen der Formel (I/m) man unter den Bedingungen der Hydrolyse in basischem Medium behandelt zur Bildung der Verbindungen der Formel (I/n), einem Sonderfall der Verbindungen der Formel (I):

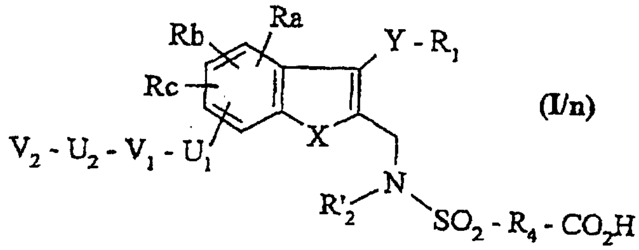

(I/n)

in der Ra, Rb, Rc, $U_1$, $V_1$, $U_2$, $V_2$, $R_1$, $R'_2$, $R_4$, X und Y die oben'angegebenen Bedeutungen besitzen,

wobei die Gesamtheit der Verbindungen der Formeln (I/c), (I/d), (I/f), (I/h), (I/j), (I/1) und (I/n) die Verbindungen der Formel (I') bilden:

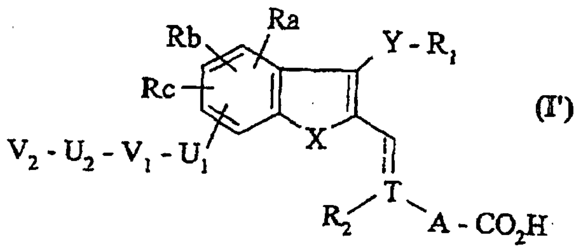

(I')

in der Ra, Rb, Rc, $U_1$, $V_1$, $U_2$, $V_2$, $R_1$, $R'_2$, X, Y, Z und A die bezüglich der Formel (I) angegebenen Bedeutungen besitzen,
welche Verbindungen der Formel (I') man mit einem O-substituierten Hydroxylamin umsetzt, so daß man nach der Abspaltung der Schutzgruppe der Hydroxylamin-Funktion die Verbindungen der Formel (I/o) erhält, einem Sonderfall der Verbindungen der Formel (I):

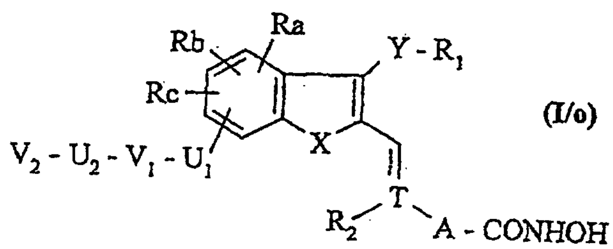

(I/o)

in der Ra, Rb, Rc, $U_1$, $V_1$, $U_2$, $V_2$, $R_1$, $R_2$, X, Y, Z und A die oben angegebenen Bedeutungen besitzen,
wonach man die Verbindungen (I/a) bis (I/o), welche die Gesamtheit der erfindungsgemäßen Verbindungen darstellen, gegebenenfalls mit Hilfe einer klassischen Reinigungsmethode reinigt, gewünschtenfalls mit Hilfe klassischer Trennungsmethoden in ihre verschiedenen Isomeren auftrennt und gegebenenfalls in ihre Additionssalze mit einer pharmazeutisch annehmbaren Säure oder Base umwandelt.

**20.** Pharmazeutische Zubereitungen enthaltend als Wirkstoff mindestens eine Verbindung der Formel (I) nach einem der Ansprüche 1 bis 18 allein oder in Kombination mit einem oder mehreren inerten, nicht-toxischen, pharmazeutisch annehmbaren Trägermaterialen oder Bindemitteln.

**21.** Pharmazeutische Zubereitungen nach Anspruch 20, enthaltend mindestens einen Wirkstoff nach einem der Ansprüche 1 bis 18, geeignet als Inhibitoren von PAI-1.

**22.** Pharmazeutische Zubereitungen nach Anspruch 20, enthaltend mindestens einen Wirkstoff nach einem der Ansprüche 1 bis 18, für die Behandlung der Thrombose, von Erkrankungen, die von Thrombose verursacht sind, und Erkrankungen, die zu einer Erhöhung von Thromboserisiken führen.

## Claims

1. Compounds of formula (1) :

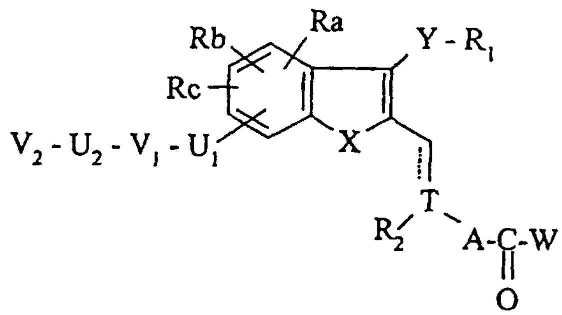

(I)

wherein:

X represents an oxygen atom, a sulphur atom, or an $NR_3$ group wherein $R_3$ represents a hydrogen atom, a linear or branched $(C_1\text{-}C_6)$alkyl group, a linear or branched $(C_1\text{-}C_6)$acyl group, an aryl group, an aryl-$(C_1\text{-}C_6)$alkyl group in which the alkyl moiety is linear or branched, or a heteroaryl-$(C_1\text{-}C_6)$alkyl group in which the alkyl moiety is linear or branched,

Y represents an oxygen atom, a sulphur atom or an $NR_3$ group, the $R_3$ group being as defined above, or may represent a single bond when X represents an $NR'_3$ group wherein $R'_3$ represents a heteroaryl-$(C_1\text{-}C_6)$alkyl group in which the alkyl moiety is linear or branched,

T represents a nitrogen atom when the bond that links it to the adjacent carbon atom is single (——), or a carbon atom or a CH group depending on whether the bond that links it to the adjacent carbon atom is single (—— ) or double (- - -——),

A represents a single bond or a group selected from $(C_1\text{-}C_6)$alkylene (optionally substituted by one or more linear or branched $(C_1\text{-}C_6)$alkyl groups, aryl groups, aryl-$(C_1\text{-}C_6)$alkyl groups in which the alkyl moiety is linear or branched, cycloalkyl groups, heterocycloalkyl groups or heteroaryl groups), arylene, cycloalkylene, heterocycloalkylene, heteroarylene and an $-SO_2\text{-}R_4$- group (the $SO_2$ moiety being linked to T) wherein $R_4$ represents a group selected from linear or branched $(C_1\text{-}C_6)$alkylene, arylene, aryl-$(C_1\text{-}C_6)$alkylene in which the alkylene moiety is linear or branched, cycloalkylene, heterocycloalkylene and heteroarylene,

W represents a group selected from hydroxy, linear or branched $(C_1\text{-}C_6)$alkoxy, aryloxy, aryl-$(C_1\text{-}C_6)$alkoxy in which the alkoxy moiety is linear or branched, cycloalkyloxy, heterocycloalkyloxy, heteroaryloxy, amino (which may itself be substituted by one or two identical or different groups each independently of the other selected from linear or branched $(C_1\text{-}C_6)$alkyl, aryl, aryl-$(C_1\text{-}C_6)$alkyl in which the alkyl moiety is linear or branched, and cycloalkyl) and hydroxyamino,

$U_1$ represents an oxygen atom, a sulphur atom or a linear or branched $(C_1\text{-}C_6)$alkylene chain wherein one or more of the carbon atoms may optionally be replaced by one or more hetero atoms selected from oxygen, nitrogen and sulphur, the said alkylene chain being optionally substituted by one or more identical or different groups selected from halogen atoms and hydroxy, linear or branched $(C_1\text{-}C_6)$alkyl and linear or branched $(C_1\text{-}C_6)$alkoxy groups,

$V_1$ represents an arylene, heteroarylene or heterocycloalkylene group,

$U_2$ represents a single bond, an oxygen, nitrogen or sulphur atom or a linear or branched $(C_1\text{-}C_6)$ alkylene chain wherein one or more carbon atoms may optionally be replaced by one or more identical or different groups selected from oxygen, sulphur and nitrogen atoms (a nitrogen atom being substituted by a group selected from hydrogen and linear or branched $(C_1\text{-}C_6)$alkyl) and

an $SO_2$ group,

$V_2$ represents an aryl, heteroaryl or heterocycloalkyl group,

Ra, Rb and Rc, which may be identical or different, each independently of the others represents a group selected from :

- a hydrogen or halogen atom,
- a hydroxy, cyano or nitro group,
- linear or branched ($C_1$-$C_6$)alkyl, linear or branched ($C_1$-$C_6$)alkoxy, linear or branched ($C_1$-$C_6$)acyl, carboxy, linear or branched ($C_1$-$C_6$)alkoxycarbonyl, or linear or branched ($C_1$-$C_6$)trihaloalkyl,
- amino (optionally substituted by one or two groups, which may be identical or different, each independently of the other linear or branched ($C_1$-$C_6$)alkyl, aryl or aryl-($C_1$-$C_6$)alkyl in which the alkyl moiety is linear or branched),
- aryloxy, aryl-($C_1$-$C_6$)alkoxy in which the alkoxy moiety is linear or branched, heteroaryloxy, or heteroaryl-($C_1$-$C_6$)alkoxy in which the alkoxy moiety is linear or branched,
- and a grouping of formula $-U_1-V_1-U_2-V_2$ wherein $U_1$, $U_2$, $V_1$ and $V_2$ are as defined hereinbefore,
- or two of them together form a methylenedioxy or ethylenedioxy group (each of those groups being optionally substituted by one or two linear or branched ($C_1$-$C_6$)alkyl groups, aryl groups or aryl-($C_1$-$C_6$)alkyl groups in which the alkyl moiety is linear or branched),

$R_1$ represents :

- an aryl group substituted by from one to five identical or different substituents each independently of the others selected from halogen, hydroxy, cyano, nitro, carboxy, linear or branched ($C_1$-$C_6$)alkyl, linear or branched ($C_1$-$C_6$)alkoxy, linear or branched ($C_1$-$C_6$)acyl, linear or branched ($C_1$-$C_6$)alkoxycarbonyl, linear or branched ($C_1$-$C_6$)trihaloalkyl (optionally substituted by a hydroxy group), linear or branched ($C_1$-$C_6$)trihaloalkoxy, amino (optionally substituted by one or two linear or branched ($C_1$-$C_6$)alkyl groups, one of which alkyl groups may be optionally substituted by a group selected from amino, linear or branched ($C_1$-$C_6$) alkylamino and di-($C_1$-$C_6$)alkylamino in which the alkyl moieties are each linear or branched), amino-($C_1$-$C_6$)alkoxy (in which the alkoxy moiety is linear or branched and the amino moiety is optionally substituted by one or two, identical or different, linear or branched ($C_1$-$C_6$)alkyl groups), linear or branched alkoxycarbonyl-($C_1$-$C_6$)alkyl, linear or branched ($C_1$-$C_6$)alkylcarbonylamino, aryl-($C_1$-$C_6$)alkyl in which the alkyl moiety is linear or branched, aryloxy, aryl-($C_1$-$C_6$)alkoxy in which the alkoxy moiety is linear or branched, arylamino, aryl-($C_1$-$C_6$)alkylamino in which the alkyl moiety is linear or branched, arylsulphanyl, aryl-($C_1$-$C_6$)alkylsulphanyl in which the alkyl moiety is linear or branched, heteroaryl, heteroaryl-($C_1$-$C_6$)alkyl in which the alkyl moiety is linear or branched, heteroaryloxy, heteroaryl-($C_1$-$C_6$)alkoxy in which the alkoxy moiety is linear or branched, heteroarylamino, heteroaryl-($C_1$-$C_6$)alkylamino in which the alkyl moiety is linear or branched, heteroarylsulphanyl and heteroaryl-($C_1$-$C_6$)alkylsulphanyl in which the alkyl moiety is linear or branched,
- a 1,3-dihydro-2*H*-indol-2-one, 3,4-dihydro-2(1*H*)-quinolinone or 1-hydroxy-2(1*H*)-pyridinone group,
- or an optionally substituted heteroaryl group,

$R_2$ represents a group selected from a hydrogen atom, a linear or branched ($C_1$-$C_6$)alkyl group, an aryl group, an aryl-($C_1$-$C_6$)alkyl group in which the alkyl moiety is linear or branched, a cycloalkyl group, a heterocycloalkyl group, a heterocycloalkyl-($C_1$-$C_6$)alkyl group in which the alkyl moiety is linear or branched, a heteroaryl group and a heteroaryl-($C_1$-$C_6$)alkyl group in which the alkyl moiety is linear or branched,

wherein :

- an aryl group is understood to be a phenyl, biphenyl, naphthyl, tetrahydronaphthyl or dihydronaphthyl group, each of those groups being optionally substituted by one or more identical or different groups selected from

halogen atoms, hydroxy groups, cyano groups, nitro groups, linear or branched ($C_1$-$C_6$)alkyl groups (optionally substituted by one or more groups selected from hydroxy, amino and mono- or di-($C_1$-$C_6$)alkylamino in which the alkyl moieties are each linear or branched), linear or branched ($C_1$-$C_6$)trihaloalkyl groups, linear or branched ($C_1$-$C_6$)alkoxy groups, aryloxy groups, linear or branched ($C_1$-$C_6$)acyl groups, carboxy groups, linear or branched ($C_1$-$C_6$)alkoxycarbonyl groups and amino groups (amino being optionally substituted by one or two identical or different linear or branched ($C_1$-$C_6$)alkyl groups),

- a cycloalkyl group is understood to be a mono- or bi-cyclic group containing from 3 to 8 carbon atoms,

- heterocycloalkyl is understood to be a mono- or bi-cyclic, saturated or unsaturated group, of non-aromatic character, having from 5 to 12 ring members containing one, two or three identical or different hetero atoms selected from oxygen, nitrogen and sulphur, it being understood that the heterocycloalkyl may be optionally substituted by one or more identical or different groups selected from halogen atoms, hydroxy groups, linear or branched ($C_1$-$C_6$)alkyl groups, linear or branched ($C_1$-$C_6$)trihaloalkyl groups, linear or branched ($C_1$-$C_6$) alkoxy groups, aryloxy groups, aryl-($C_1$-$C_6$)alkoxy groups in which the alkoxy moiety is linear or branched, amino groups (optionally substituted by one or two linear or branched ($C_1$-$C_6$)alkyl groups), linear or branched ($C_1$-$C_6$)acyl groups, linear or branched ($C_1$-$C_6$)alkoxycarbonyl groups, nitro groups and oxo groups,

- heteroaryl is understood to be a mono- or bi-cyclic heterocycloalkyl as defined hereinbefore, at least one of the rings of which has an aromatic character, it being possible for the hetero atom(s) to be located, in the case of a bicyclic system, on the ring having an aromatic character or on the partially unsaturated ring, it being understood that the heteroaryl group may be optionally substituted by one or more identical or different groups as defined for the substituents of heterocycloalkyl,

their isomers and addition salts thereof with a pharmaceutically acceptable acid or base.

**2.** Compounds of formula (I) according to claim 1, **characterised in that** X represents a sulphur atom or an $NR_3$ group wherein $R_3$ is as defined for formula (I), their isomers and addition salts thereof with a pharmaceutically acceptable acid or base.

**3.** Compounds of formula (I) according to claim 1, **characterised in that** Y represents an oxygen atom, their isomers and addition salts thereof with a pharmaceutically acceptable acid or base.

**4.** Compounds of formula (I) according to claim 1, **characterised in that** $R_1$ represents a group selected from phenyl optionally substituted by a group as defined for formula (I), optionally substituted quinolyl and optionally substituted pyridyl, their isomers and addition salts thereof with a pharmaceutically acceptable acid or base.

**5.** Compounds of formula (I) according to claim 1, **characterised in that** $R_2$ represents a group selected from aryl and heteroaryl, each of those groups being optionally substituted, their isomers and addition salts thereof with a pharmaceutically acceptable acid or base.

**6.** Compounds of formula (I) according to any one of claims 1 and 5, **characterised in that** $R_2$ represents a pyridyl group, their isomers and addition salts thereof with a pharmaceutically acceptable acid or base.

**7.** Compounds of formula (I) according to claim 1, **characterised in that** $U_1$ represents a linear ($C_1$-$C_4$)alkylene chain wherein one of the carbon atoms is replaced by an oxygen atom, $V_1$ represents an arylene group, $U_2$ represents a single bond and $V_2$ represents an aryl group optionally substituted by one of the groups as defined for formula (I), their isomers and addition salts thereof with a pharmaceutically acceptable acid or base.

**8.** Compounds of formula (I) according to any one of claims 1 and 7, **characterised in that** $-U_1-V_1-U_2-V_2$ represents a [1,1'-biphenyl]-4-ylmethoxy grouping, their isomers and addition salts thereof with a pharmaceutically acceptable acid or base.

**9.** Compounds of formula (I) according to claim 1, **characterised in that** at least one of the groups Ra, Rb or Rc represents a grouping of formula $-U_1-V_1-U_2-V_2$ as defined for formula (I), their isomers and addition salts thereof with a pharmaceutically acceptable acid or base.

**10.** Compounds of formula (I) according to any one of claims 1 to 3, **characterised in that** X represents a sulphur

atom and Y represents an oxygen atom, their isomers and addition salts thereof with a pharmaceutically acceptable acid or base.

**11.** Compounds of formula (I) according to claim 1, **characterised in that** X represents a sulphur atom, Y represents an oxygen atom, $R_1$ represents an optionally substituted phenyl group or an optionally substituted pyridyl group, and A represents a single bond when T represents a carbon atom or a CH group, their isomers and addition salts thereof with a pharmaceutically acceptable acid or base.

**12.** Compounds of formula (I) according to claim 1, **characterised in that** X represents a sulphur atom, Y represents an oxygen atom, $R_1$ represents a phenyl group optionally substituted by a group as defined for formula (I), and A represents an alkylene group (optionally substituted by a linear or branched ($C_1$-$C_6$)alkyl group, an aryl group or by an aryl-($C_1$-$C_6$)alkyl group in which the alkyl moiety is linear or branched) or an arylene group when T represents a nitrogen atom, their isomers and addition salts thereof with a pharmaceutically acceptable acid or base.

**13.** Compounds of formula (I) according to claim 1, **characterised in that** they represent compounds of formula (I bis) :

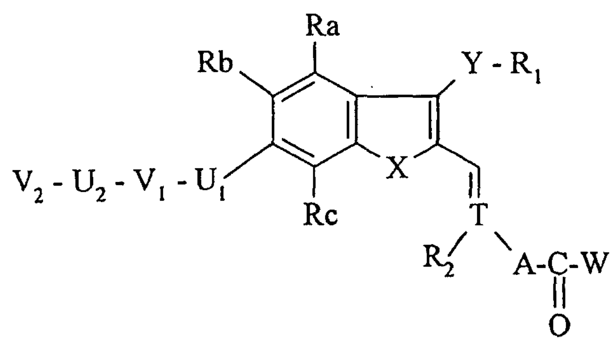

**(I bis)**

wherein :

X represents a sulphur atom,
Y represents an oxygen atom,
$R_1$ represents an optionally substituted phenyl group or a heteroaryl group selected from optionally substituted pyridyl and optionally substituted quinolyl,
A represents a single bond,
T represents a carbon atom,
Ra and Rc each represents a hydrogen atom,
$U_1$ represents a linear ($C_1$-$C_4$)alkyleneoxy chain,
$V_1$ represents an arylene group,
$U_2$ represents a single bond,
$V_2$ represents an aryl group,
Rb represents a $U_1$-$V_1$-$U_2$-$V_2$ grouping as defined hereinbefore,
$R_2$ represents a heteroaryl group,
W represents a group as defined for formula (I), their isomers and addition salts thereof with a pharmaceutically acceptable acid or base.

**14.** Compounds of formula (I) according to claim 13, **characterised in that** they represent compounds of formula (I bis) wherein Rb and -$U_1$-$V_1$-$U_2$-$V_2$ each represents a [1,1'-biphenyl]-4-ylmethoxy grouping, their isomers and addition salts thereof with a pharmaceutically acceptable acid or base.

**15.** Compounds of formula (I) according to claim 13, **characterised in that** they represent compounds of formula (I bis) wherein $R_2$ represents a pyridyl group, their isomers and addition salts thereof with a pharmaceutically acceptable acid or base.

**16.** Compounds of formula (I) according to claim 13, **characterised in that** they represent compounds of formula (I bis) wherein $R_1$ represents :

- a phenyl group optionally substituted by from one to three groups selected from halogen atoms, linear or branched ($C_1$-$C_6$)alkyl groups, heteroaryl groups, heteroaryl-($C_1$-$C_6$)alkoxy groups in which the alkoxy moiety is linear or branched, linear or branched ($C_1$-$C_6$)alkoxy, amino groups and linear or branched amino-($C_1$-$C_6$) alkoxy groups, it being possible for the amino moiety to be substituted by one or two identical or different linear or branched ($C_1$-$C_6$)alkyl groups,
- or a heteroaryl group selected from pyridyl and quinolyl optionally substituted by a halogen atom or a linear or branched ($C_1$-$C_6$)alkyl group,

their isomers and addition salts thereof with a pharmaceutically acceptable acid or base.

**17.** Compound of formula (I) according to claim 1 which is (E)-3-[5,6-bis([1,1'-biphenyl]-4-ylmethoxy)-3-(4-chlorophenoxy)benzo[b]thiophen-2-yl]-2-(4-pyridyl)-2-propenoic acid, its isomers and addition salts thereof with a pharmaceutically acceptable acid or base.

**18.** Compounds of formula (I) according to claim 1 which are :

- ethyl (*E*)-3-[5,6-bis-([1,1'-biphenyl]-4-ylmethoxy)-3-(4-chlorophenoxy)-benzo[*b*]thiophen-2-yl]-2-(4-pyridyl)-2-propenoate,
- (*E*)-3-[5,6-bis-([1,1'-biphenyl]-4-ylmethoxy)-3-(3-pyridyloxy)-benzo[*b*]thiophen-2-yl]-2-(4-pyridyl)-2-propenoic acid,
- 3-(*E*)-{5,6-bis-([1,1'-biphenyl]-4-ylmethoxy)-3-[6-(methyl)pyridyl-3-oxy]-benzo[*b*]-thiophen-2-yl}-2-(4-pyridyl)-2-propenoic acid,
- 3-(*E*)-[5,6-bis-([1,1'-biphenyl]-4-ylmethoxy)-3-(6-quinolyloxy)benzo[*b*]thiophen-2-yl]-2-(4-pyridyl)-2-propenoic acid,
- (*E*)-3-[5,6-bis-([1,1'-biphenyl]-2-ylmethoxy)-3-(4-chlorophenoxy)benzo[*b*]thiophen-2-yl]-2-(4-pyridyl)-2-propenoic acid,
- (*E*)-3-[5,6-bis([1,1'-biphenyl]-3-ylmethoxy)-3-(4-chlorophenoxy)-benzo[*b*]thiophen-2-yl]-2-(4-pyridyl)-2-propenoic acid,
- (*E*)-3-[5,6-bis([1,1'-biphenyl]-4-ylmethoxy)-3-(4-chloro-3-fluorophenoxy)-benzo[*b*]-thiophen-2-yl]-2-(4-pyridyl)-2-propenoic acid,
- (*E*)-3-[5,6-bis([1,1'-biphenyl]-4-ylmethoxy)-3-(4-chloro-3,5-dimethylphenoxy)-benzo[*b*]thiophen-2-yl]-2-(4-pyridyl)-2-propenoic acid,
- (*E*)-3-[5,6-bis([1,1'-biphenyl]-4-ylmethoxy)-3-(4-chloro-3-methylphenoxy)-benzo[*b*]-thiophen-2-yl]-2-(4-pyridyl)-2-propenoic acid,
- (*E*)-3-{5,6-bis([1,1'-biphenyl]-4-ylmethoxy)-3-[4-(4-pyridyloxy)phenoxy]-benzo[*b*]-thiophen-2-yl}-2-(4-pyridyl)-2-propenoic acid,
- (*E*)-3-{5,6-bis([1,1'-biphenyl]-4-ylmethoxy)-3-[4-(1*H*-imidazol-1-yl)phenoxy]-benzo[*b*]thiophen-2-yl}-2-(4-pyridyl)-2-propenoic acid,
- (*E*)-3-[5,6-bis([1,1'-biphenyl]-4-ylmethoxy)-3-phenoxy-benzo[*b*]thiophen-2-yl]-2-(4-pyridyl)-2-propenoic acid,
- (*E*)-3-[5,6-bis([1,1'-biphenyl]-4-ylmethoxy)-3-(3-fluorophenoxy)-benzo[*b*]thiophen-2-yl]-2-(4-pyridyl)-2-propenoic acid,
- (*E*)-3-[5,6-bis([1,1'-biphenyl]-4-ylmethoxy)-3-(3,4-difluorophenoxy)-benzo[*b*]-thiophen-2-yl]-2-(4-pyridyl)-2-propenoic acid,
- (*E*)-3-{5,6-bis([1,1'-biphenyl]-4-ylmethoxy)-3-[(6-chloro-3-pyridyl)oxy]-benzo[*b*]thiophen-2-yl}-2-(4-pyridyl)-2-propenoic acid,

their isomers and addition salts thereof with a pharmaceutically acceptable acid or base.

**19.** Process for the preparation of compounds of formula (I) according to claim 1, **characterised in that** there is used as starting material a compound of formula (II) :

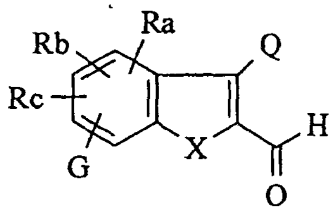

**(II)**

wherein Ra, Rb, Rc and X are as defined for formula (I), G represents a hydroxy group protected by a protecting group conventionally used in organic synthesis and Q represents a halogen atom or a hydroxy group and, preferably, Q represents a halogen atom when X represents a sulphur atom or an $NR_3$ group wherein $R_3$ is as defined for formula (I) and Q represents a hydroxy group when X represents an oxygen atom,
which compound of formula (II) is reacted, under basic conditions,

- *when Q represents a halogen atom*,

  ✧ with a compound of formula (III),

$$H - Y_1 - R_1 \qquad \textbf{(III)}$$

wherein $R_1$ is as defined for formula (I) and $Y_1$ represents an oxygen atom, a sulphur atom or an $NR_3$ group wherein $R_3$ is as defined for formula (I),
to yield the compounds of formula (IV/a) :

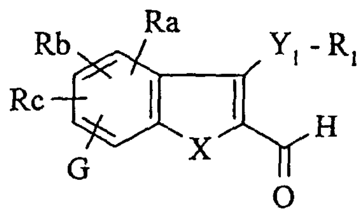

**(IV/a)**

wherein Ra, Rb, Rc, G, $R_1$, X and $Y_1$ are as defined hereinbefore,

  ✧ or with a compound of formula (V) :

$$(HO)_2B - R_1 \qquad \textbf{(V)}$$

wherein $R_1$ is as defined for formula (I),
to yield the compounds of formula (IV/b) :

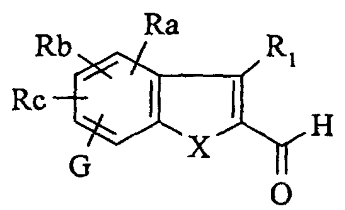

**(IV/b)**

wherein Ra, Rb, Rc, G and $R_1$ are as defined hereinbefore and $X_1$ represents an $NR_3$ group wherein $R_3$ represents a heteroaryl-($C_1$-$C_6$)alkyl group in which the alkyl moiety is linear or branched,

- *or, when Q represents a hydroxy group,* with a compound of formula (VI),

$$Hal - R_1 \qquad \textbf{(VI)}$$

wherein Hal represents a halogen atom and $R_1$ is as defined hereinbefore,
to yield the compounds of formula (IV/c) :

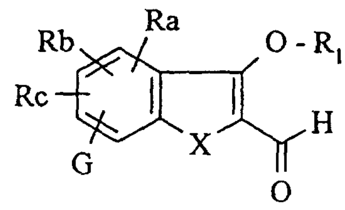

**(IV/c)**

wherein Ra, Rb, Rc, G, X and $R_1$ are as defined hereinbefore, the totality of the compounds of formulae (IV/a), (IV/b) and (IV/c) constituting the compounds of formula (IV) :

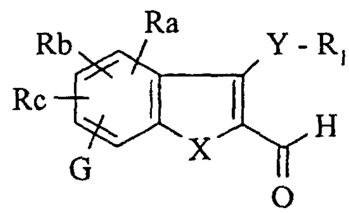

**(IV)**

wherein Ra, Rb, Rc, G, $R_1$, X and Y are as defined for formula (I), which compounds of formula (IV) :

- are condensed, in the presence of acetic anhydride, with a compound of formula (VII),

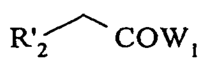

**(VII)**

wherein $R'_2$ has the same definition as $R_2$ for formula (1), with the exception that $R'_2$ cannot represent a hydrogen atom, and $W_1$ represents a group selected from linear or branched ($C_1$-$C_6$)alkoxy, aryloxy, aryl-($C_1$-$C_6$)alkoxy in which the alkoxy moiety is linear or branched, cycloalkyloxy, heterocycloalkoxy, heteroaryloxy and an amino group (which is itself optionally substituted by one or two identical or different groups each independently of the other selected from linear or branched ($C_1$-$C_6$)alkyl, aryl, aryl-($C_1$-$C_6$) alkyl in which the alkyl moiety is linear or branched, and cycloalkyl),
to yield the compounds of formula (VIII) :

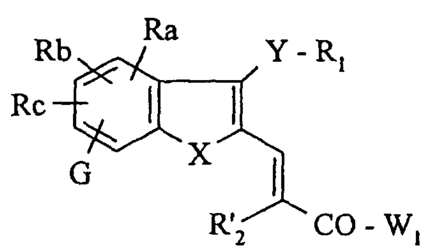

**(VIII)**

wherein Ra, Rb, Rc, G, $R_1$, $R'_2$, X, Y and $W_1$ are as defined hereinbefore,
the hydroxy function of which compounds of formula (VIII) is deprotected under conventional conditions of organic synthesis, and which are then reacted in a basic medium with a compound of formula (IX) :

$$V_2 - U_2 - V_1 - U_1 - Hal \qquad \textbf{(IX)}$$

wherein $U_1$, $V_1$, $U_2$ and $V_2$ are as defined for formula (I) and Hal represents a halogen atom, to yield the compounds of formula (I/a), a particular case of the compounds of formula (I) :

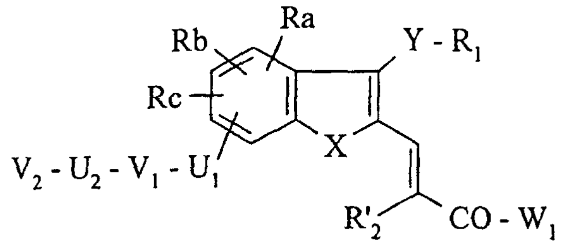

(I/a)

wherein Ra, Rb, Rc, $U_1$, $V_1$, $U_2$, $V_2$, $R_1$, $R'_2$, X, Y and $W_1$ are as defined hereinbefore, which compounds of formula (I/a) are, if desired, subjected :

✱ either to conditions of catalytic hydrogenation, in the presence of palladium, to yield the compounds of formula (I/b), a particular case of the compounds of formula (I) :

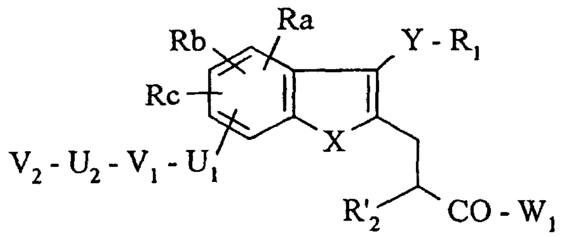

(I/b)

wherein Ra, Rb, Rc, $U_1$, $V_1$, $U_2$, $V_2$, $R_1$, $R'_2$, X, Y and $W_1$ are as defined hereinbefore,

✱ or to conditions of hydrolysis, in a basic medium, to yield the compounds of formula (I/c), a particular case of the compounds of formula (I) :

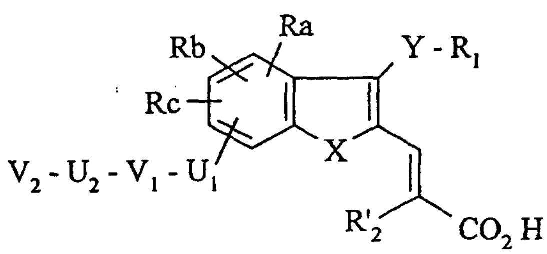

(I/c)

wherein Ra, Rb, Rc, $U_1$, $V_1$, $U_2$, $V_2$, $R_1$, $R'_2$, X and Y are as defined hereinbefore, the double bond of which compounds of formula (I/c) is, if desired, reduced by catalytic hydrogenation to yield the compounds of formula (I/d), a particular case of the compounds of formula (I) :

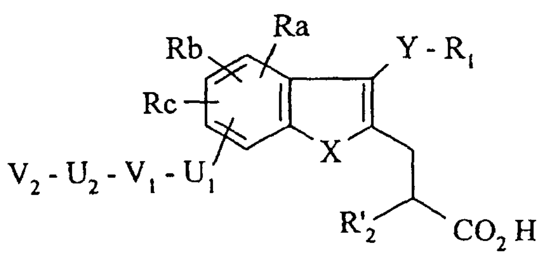

(I/d)

wherein Ra, Rb, Rc, $U_1$, $V_1$, $U_2$, $V_2$, $R_1$, $R'_2$, X and Y are as defined hereinbefore,

- or are subjected to the action of a phosphorus ylid of formula (X),

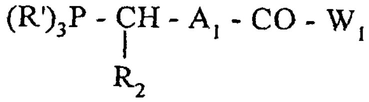

**(X)**

wherein R' represents a linear or branched ($C_1$-$C_6$)alkyl group or a phenyl group, $R_2$ is as defined for formula (I), $W_1$ is as defined hereinbefore and $A_1$ represents a single bond, an alkylene group (optionally substituted by one or more groups selected from linear or branched ($C_1$-$C_6$)alkyl, aryl, aryl-($C_1$-$C_6$)alkyl in which the alkyl moiety is linear or branched, cycloalkyl, heterocycloalkyl and heteroaryl), an arylene group, a cycloalkylene group, a heterocycloalkylene group or a heteroarylene group,
to yield the compounds of formula (XI) :

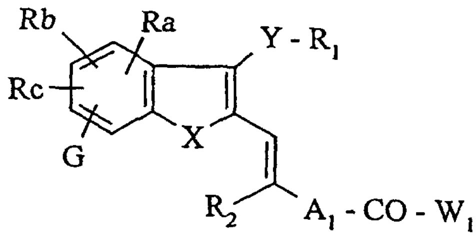

**(XI)**

wherein Ra, Rb, Rc, G, $R_1$, $R_2$, X, Y, $A_1$ and $W_1$ are as defined hereinbefore,
the hydroxy function of which compounds of formula (XI) is deprotected under conventional conditions of organic synthesis, and which are then reacted in a basic medium with a compound of formula (IX) :

$$V_2 - V_2 - V_1 - U_1 - Hal \qquad \textbf{(IX)}$$

wherein $U_1$, $V_1$, $U_2$ and $V_2$ are as defined for formula (I) and Hal represents a halogen atom, to yield the compounds of formula (I/e), a particular case of the compounds of formula (I) :

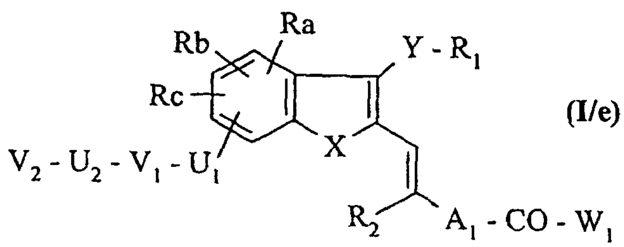

**(I/e)**

wherein Ra, Rb, Rc, $U_1$, $V_1$, $U_2$, $V_2$, $R_1$, $R_2$, $A_1$ X, Y and $W_1$ are as defined hereinbefore,
which compounds of formula (I/e) are, if desired, subjected :

✱ either to conditions of hydrolysis, under basic conditions, to yield the compounds of formula (1/f), a particular case of the compounds of formula (I) :

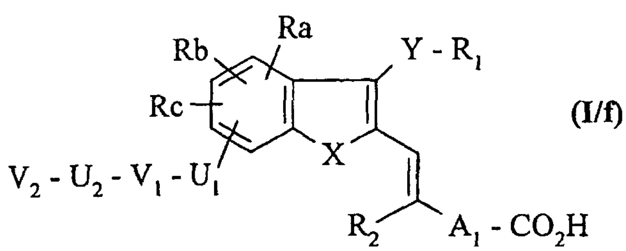

**(I/f)**

wherein Ra, Rb, Rc, $U_1$, $V_1$, $U_2$, $V_2$, $R_1$, $R_2$, X, Y and $A_1$ are as defined hereinbefore,

✱ or to conditions of catalytic hydrogenation to yield the compounds of formula (I/g), a particular

case of the compounds of formula (I) :

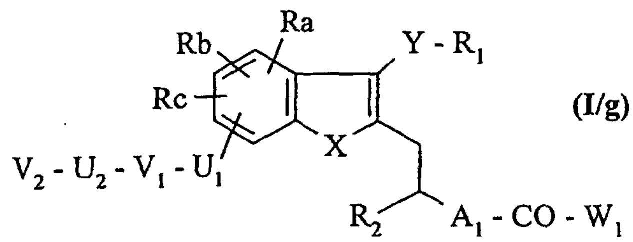

**(I/g)**

wherein Ra, Rb, Rc, $U_1$, $V_1$, $U_2$, $V_2$, $R_1$, $R_2$, X, Y, $A_1$ and $W_1$ are as defined hereinbefore, which compounds of formula (I/g) may be treated under conditions of basic hydrolysis to yield the compounds of formula (1/h), a particular case of the compounds of formula (I) ;

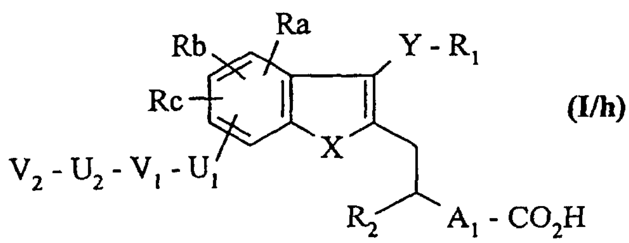

**(I/h)**

wherein Ra, Rb, Rc, $U_1$, $V_1$, $U_2$, $V_2$, $R_1$, $R_2$, X, Y and $A_1$ are as defined hereinbefore,

- or the hydroxy function of which compounds of formula (IV) is deprotected under conventional conditions of organic synthesis, and which are then reacted in a basic medium with a compound of formula (IX) :

$$V_2\text{-}U_2\text{-}V_1\text{-}U_1\text{-Hal} \qquad \textbf{(IX)}$$

wherein $U_1$, $V_1$, $U_2$ and $V_2$ are as defined for formula (I) and Hal represents a halogen atom, to yield the compounds of formula (XII) :

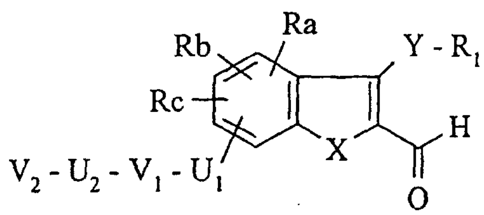

**(XII)**

wherein Ra, Rb, Rc, $U_1$, $V_1$, $U_2$, $V_2$, $R_1$, X and Y are as defined hereinbefore, the aldehyde function of which compounds of formula (XII) is reduced to the primary alcohol to yield the compounds of formula (XIII):

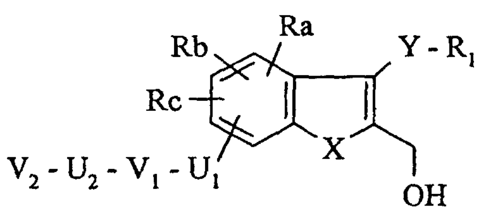

**(XIII)**

wherein Ra, Rb, Rc, $U_1$, $V_1$, $U_2$, $V_2$, $R_1$, X and Y are as defined for formula (I), the terminal hydroxy of which compounds of formula (XIII) is replaced by a halogen atom, under conventional conditions, to yield the compounds of formula (XIV) :

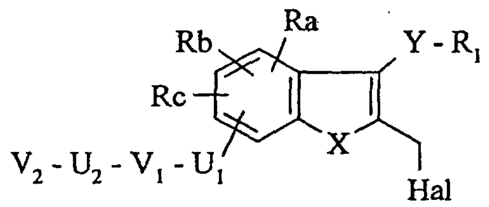

**(XIV)**

wherein Ra, Rb, Rc, $U_1$, $V_1$, $U_2$, $V_2$, $R_1$, X and Y are as defined hereinbefore and Hal represents a chlorine or bromine atom,
in which compounds of formula (XIV) :

✱ the halogen atom is replaced, under basic conditions, by an aminated compound of formula (XV) :

$$R_2 - NH - A_1 - CO - W_1 \qquad \textbf{(XV)}$$

wherein $R_2$ is as defined for formula (I) and $A_1$ and $W_1$ are as defined hereinbefore, to yield the compounds of formula (I/i), a particular case of the compounds of formula (I) :

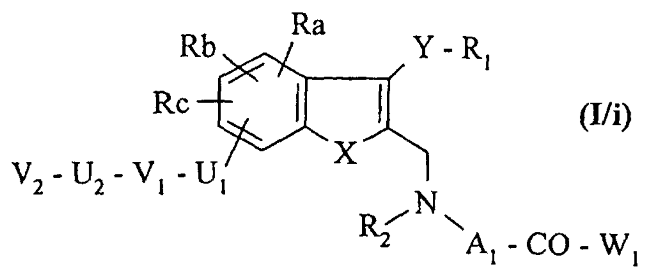

**(I/i)**

wherein Ra, Rb, Rc, $U_1$, $V_1$, $U_2$, $V_2$, $R_1$, $R_2$, X, Y, $A_1$ and $W_1$ are as defined hereinbefore, the terminal carbonyl group of which compounds of formula (I/i) is hydrolysed under basic conditions to yield the compounds of formula (I/j), a particular case of the compounds of formula (I) :

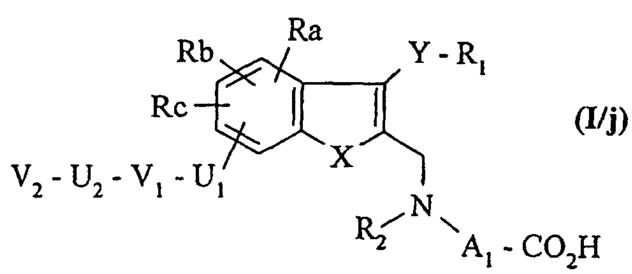

**(I/j)**

wherein Ra, Rb, Rc, $U_1$, $V_1$, $U_2$, $V_2$, $R_1$, $R_2$, X, Y and $A_1$ are as defined hereinbefore,

✱ or which compounds of formula (XIV) are initially treated with sodium azide, the resulting azide being reduced to the primary amine under conditions of catalytic hydrogenation to yield the compounds of formula (XVI) :

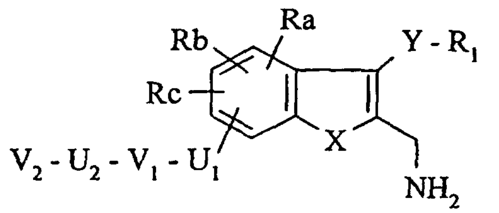

**(XVI)**

wherein Ra, Rb, Rc, $U_1$, $V_1$, $U_2$, $V_2$, $R_1$, X and Y are as defined for formula (I), which compounds of formula (XVI) are condensed, under basic conditions, with a chlorosulphonyl compound of formula (XVII) :

$$Cl - SO_2 - R_4 - CO - W_1 \qquad \textbf{(XVII)}$$

wherein $R_4$ is as defined for formula (I) and $W_1$ is as defined hereinbefore, to yield the compounds of formula (I/k), a particular case of the compounds of formula (I),

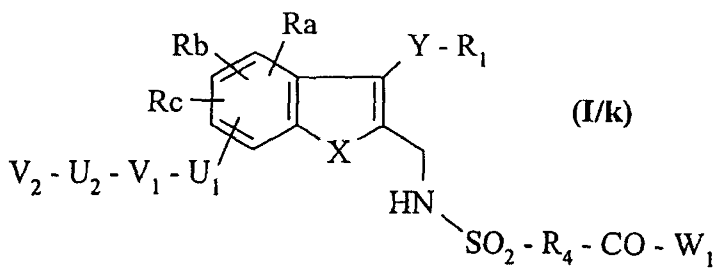

**(I/k)**

wherein Ra, Rb, Rc, $U_1$, $V_1$, $U_2$, $V_2$, $R_1$, $R_4$, X, Y and $W_1$ are as defined hereinbefore, which compounds of formula (I/k) :

- are subjected, if desired, to conditions of hydrolysis under basic conditions to yield the compounds of formula (I/1), a particular case of the compounds of formula (I) :

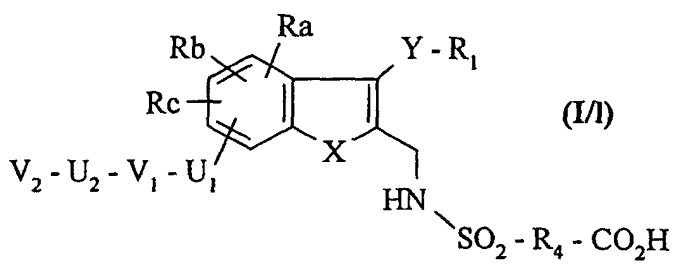

**(I/l)**

wherein Ra, Rb, Rc, $U_1$, $V_1$, $U_2$, $V_2$, $R_1$, $R_4$, X and Y are as defined hereinbefore,

- or condensed, in a basic medium, with a compound of formula (XVIII) :

$$Hal - R'_2 \qquad \textbf{(XVIII)}$$

wherein Hal represents a halogen atom, such as iodine, and $R'_2$ is as defined hereinbefore, to yield the compounds of formula (I/m), a particular case of the compounds of formula (I) :

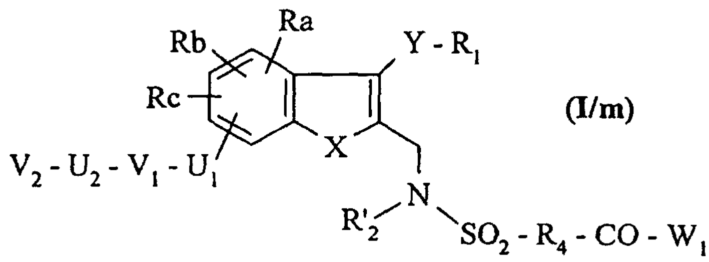

(I/m)

wherein Ra, Rb, Rc, $U_1$, $V_1$, $U_2$, $V_2$, $R_1$, $R'_2$, $R_4$, X, Y and $W_1$ are as defined hereinbefore, which compounds of formula (I/m) are treated under conditions of hydrolysis in a basic medium to yield the compounds of formula (I/n), a particular case of the compounds of formula (I) :

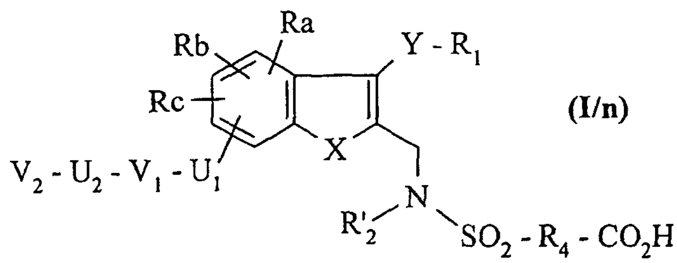

(I/n)

wherein Ra, Rb, Rc, $U_1$, $V_1$, $U_2$, $V_2$, $R_1$, $R'_2$, $R_4$, X and Y are as defined hereinbefore,

the totality of the compounds of formulae (I/c), (I/d), (I/f), (I/h), (I/j), (I/l) and (I/n) constituting the compounds of formula (I'):

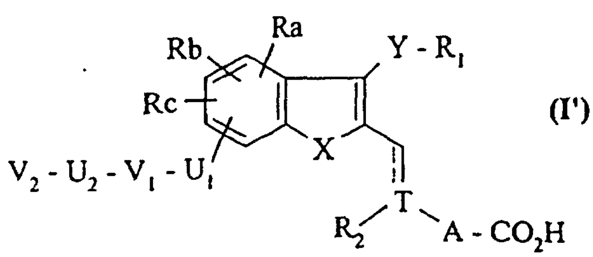

(I')

wherein Ra, Rb, Rc, $U_1$, $V_1$, $U_2$, $V_2$, $R_1$, $R_2$, X, Y, Z and A are as defined for formula (I), which compounds of formula (I') are reacted with an O-substituted hydroxylamine to yield, after deprotection of the hydroxylamine function, the compounds of formula (I/o), a particular case of the compounds of formula (I) :

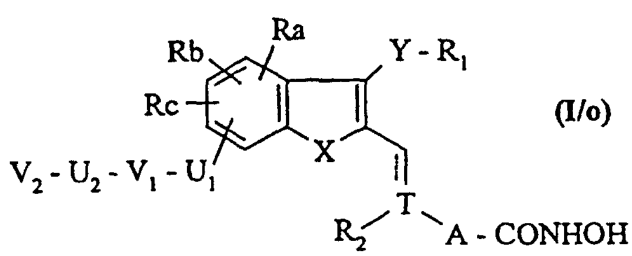

(I/o)

wherein Ra, Rb, Rc, $U_1$, $V_1$, $U_2$, $V_2$, $R_1$, $R_2$, X, Y, Z and A are as defined hereinbefore,
the compounds (I/a) to (I/o) constituting the totality of the compounds of the invention, which are purified, if necessary, according to a conventional purification technique, which may be separated, if desired, into their different isomers according to a conventional separation technique, and which are converted, where appropriate, into addition salts thereof with a pharmaceutically acceptable acid or base.

**20.** Pharmaceutical compositions comprising as active ingredient at least one compound of formula (I) according to any one of claims 1 to 18, alone or in combination with one or more inert, non-toxic, pharmaceutically acceptable excipients or carriers.

**21.** Pharmaceutical compositions according to claim 20 comprising at least one active ingredient according to any one of claims 1 to 18, for use as an inhibitor of PAI-1.

**22.** Pharmaceutical compositions according to claim 20 comprising at least one active ingredient according to any one of claims 1 to 18, for use in the treatment of thrombosis, pathologies the origin of which is thrombosis and pathologies causing an increase in thrombotic risks.